(19) Europäisches Patentamt
European Patent Office
Office européen des brevets

(11) **EP 3 612 493 B1**

(12) **EUROPÄISCHE PATENTSCHRIFT**

(45) Veröffentlichungstag und Bekanntmachung des Hinweises auf die Patenterteilung:
**10.07.2024 Patentblatt 2024/28**

(21) Anmeldenummer: **18718833.9**

(22) Anmeldetag: **20.04.2018**

(51) Internationale Patentklassifikation (IPC):
**B81B 3/00** *(2006.01)*    **H04R 1/24** *(2006.01)*

(52) Gemeinsame Patentklassifikation (CPC):
**B81B 3/0027; B81B 3/0024; H04R 1/24;**
B81B 2201/0257; B81B 2201/032; B81B 2201/036;
B81B 2203/051; B81B 2207/012; H04R 9/063;
H04R 9/08; H04R 15/00; H04R 17/00; H04R 19/02;
H04R 19/04; H04R 23/002;        (Forts.)

(86) Internationale Anmeldenummer:
**PCT/EP2018/060222**

(87) Internationale Veröffentlichungsnummer:
**WO 2018/193109 (25.10.2018 Gazette 2018/43)**

(54) **MEMS-WANDLER ZUM INTERAGIEREN MIT EINEM VOLUMENSTROM EINES FLUIDS UND VERFAHREN ZUM HERSTELLEN DESSELBEN**

MEMS CONVERTER FOR INTERACTION WITH A VOLUME FLOW RATE OF A FLUID, AND METHOD FOR PRODUCING SAME

TRANSDUCTEUR MEMS DESTINÉ À INTERAGIR AVEC UN DÉBIT VOLUMIQUE D'UN FLUIDE ET PROCÉDÉ DE FABRICATION DE CELUI-CI

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **21.04.2017 DE 102017206766**

(43) Veröffentlichungstag der Anmeldung:
**26.02.2020 Patentblatt 2020/09**

(73) Patentinhaber: **Fraunhofer-Gesellschaft zur Förderung
der angewandten Forschung e.V.
80686 München (DE)**

(72) Erfinder:
• **SCHENK, Harald
01109 Dresden (DE)**
• **CONRAD, Holger
01099 Dresden (DE)**

(74) Vertreter: **König, Andreas Rudolf et al
Schoppe, Zimmermann, Stöckeler
Zinkler, Schenk & Partner mbB
Patentanwälte
Radlkoferstraße 2
81373 München (DE)**

(56) Entgegenhaltungen:
DE-A1- 102010 029 936        DE-A1- 102012 210 052
DE-A1- 102015 210 919        US-A1- 2014 225 275
US-A1- 2015 069 537          US-A1- 2015 311 178
US-A1- 2016 204 050          US-A1- 2016 362 293

(52) Gemeinsame Patentklassifikation (CPC): (Forts.)
H04R 2201/003

**Beschreibung**

Die vorliegende Erfindung bezieht sich auf einen MEMS-Wandler zum Interagieren mit einem Volumenstrom eines Fluids, wie etwa einen MEMS-Lautsprecher, ein MEMS-Mikrophon oder eine MEMS-Pumpe und insbesondere MEMS-Wandler mit einer integrierten elektronischen Schaltung. Die vorliegende Erfindung bezieht sich ferner auf eine Vorrichtung mit einem derartigem MEMS-Wandler und auf ein Verfahren zum Herstellen eines MEMS-Wandlers. Ferner bezieht sich die vorliegende Erfindung auf MEMS-CMOS (komplementäre Metalloxid-Halbleiter) Lautsprechersysteme auf einem Chip.

Ein Fokus der MEMS-Technologie (MEMS = mikroelektromechanisches System) liegt neben der Miniaturisierbarkeit insbesondere im Potential der kostengünstigen Fertigbarkeit der Komponente bei mittleren und hohen Stückzahlen. Elektroakustische MEMS-Lautsprecher sind derzeit in unbedeutendem Maße kommerzialisiert. Mit wenigen Ausnahmen bestehen MEMS-Lautsprecher aus einer Membran, welche durch ein ausgewähltes physikalisches Wirkprinzip quasistatisch oder resonant ausgelenkt wird. Die Auslenkung hängt dabei linear oder nicht-linear von dem angelegten elektrischen Signal ab (Strom oder Spannung). Das Signal weist eine zeitliche Variation auf, welche in eine zeitliche Variation der Membranauslenkung übertragen wird. Die Hin- und Her-Bewegung der Membran überträgt sich in Form von Schall in das umgebende Fluid, für das im Folgenden vereinfachend aber nicht einschränkend Luft angenommen werden kann.

In manchen Fällen erfolgt die Aktuation der Membran nur in eine Richtung. Die Rückstellkraft wird dann durch die mechanische Federwirkung bei Membranauslenkung zur Verfügung gestellt. In anderen Fällen erfolgt die Aktuation in beide Richtungen, so dass die Membran eine sehr geringe Steifigkeit aufweisen kann.

Zur Aktuation der Membran ist der Einsatz elektrostatischer, piezoelektrischer, elektromagnetischer, elektrodynamischer und magnetostriktiver Wirkprinzipien beschrieben. Ein Überblick zu MEMS-Schallwandlern, die auf diesen Prinzipien basieren, findet sich beispielsweise in [1].

Elektrostatisch betriebene Wandler basieren auf der Kraft, die sich zwischen zwei mit unterschiedlichem elektrischem Potential belegten flächenhaften Elektroden ergeben. Im einfachsten Fall entspricht die Anordnung einem Plattenkondensator, wobei eine der beiden Platten beweglich aufgehängt ist. In der praktischen Umsetzung wird die bewegliche Elektrode als Membran ausgeführt, um einen akustischen Kurzschluss zu vermeiden. Bei einem Anlegen einer Spannung verwölbt sich die Membran in Richtung der Gegenelektrode. Bei einem speziellen Ausführungsbeispiel wird die Membran im sogenannten Berührungsmodus (engl.: touch-mode) betrieben. Hierbei berührt die Membran die untere Elektrode, auf die zur Vermeidung eines Kurschlusses eine dünne Isolatorschicht aufgebracht ist, wie es beispielsweise in [2] beschrieben ist. Die Auflagefläche wird dabei durch die Größe der anliegenden elektrischen Spannung bestimmt und variiert damit zeitlich gemäß dem zeitlichen Verlauf dieser Spannung. Die so erzeugbare Oszillation dient der Schallerzeugung. Prinzipiell kann beim klassischen elektrostatischen Aufbau die Membran nur in Richtung der Elektrode angezogen werden. Die Rückstellkraft kann durch die Steifigkeit der Membran zumindest teilweise bestimmt und muss genügend hoch sein, um auch die höheren Frequenzen im Hörschallbereich übertragen zu können.

Anderseits kann bei gegebener elektrischer Spannung die Auslenkung der Membran mit zunehmender Steifigkeit sinken. Zur Vermeidung dieses Problems wurde ein Ansatz mit einer sehr weichen Membran entwickelt, welche dann durch eine obere und untere Elektrode angesteuert und somit in beide Richtungen ausgelenkt werden kann, wie es in [3] beschrieben ist. Dieser Lautsprecher verwendet insgesamt zwei solcher Membranen, die im Inneren einer Kavität aufgehängt sind, welche wie bei einer Mikropumpe über einen Einlass und einen Auslass verfügt und ansonsten geschlossen ist.

Piezoelektrisch betriebene Wandler nutzen den inversen piezoelektrischen Effekt. Eine anliegende elektrische Spannung führt zu einer mechanischen Spannung in einem Festkörper. In MEMS-Technologie werden typischerweise Materialien wie PZT (Blei-Zirkonat-Titanat), AIN (Aluminiumnitrid) oder ZnO (Zinkoxid) eingesetzt. Diese Materialien werden üblicherweise als Funktionsschicht auf eine Membran aufgebracht und so strukturiert, dass die Membran abhängig von der elektrischen Spannung, die an der Funktionsschicht anliegt, ausgelenkt bzw. zum Schwingen angeregt werden kann. Nachteilig bei piezoelektrischen Funktionsschichten wirkt sich die Tatsache aus, dass der Betrieb nicht hysteresefrei erfolgen kann. Außerdem ist die Integration der keramischen Funktionsschichten komplex und aufgrund der nicht vorhandenen CMOS-Kompatibilität (CMOS = complementary metal oxide semiconductor, komplementärer Metalloxidhalbleiter) bei PZT und ZnO nur unter strenger Kontaminationskontrolle oder gleich in einem separaten Reinraumbereich möglich.

Elektromagnetisch betriebene Wandler beruhen auf der Kraftwirkung, die ein weichmagnetisches Material in einem ortsveränderlichen Magnetfeld (Gradient) erfährt. Für die Umsetzung des Prinzips wird neben dem weichmagnetischen Material ein Permanentmagnet und eine Spule benötigt, mittels derer der Ortsgradient des Magnetfeldes via Stromfluss zeitlich gesteuert werden kann. Das weichmagnetische Material wird beispielsweise in die Membran integriert. Alle anderen Komponenten werden in der Assemblierung zur Verfügung gestellt, wie es beispielsweise in [4] beschrieben ist. Der Aufbau ist voluminös, komplex und erscheint nicht sinnvoll für hohe Stückzahlen skalierbar zu sein.

Elektrodynamisch betriebene Wandler machen von der Lorentz-Kraft Gebrauch. Dieses bei makroskopischen Lautsprechern sehr weitverbreitete Verfahren ist auch bei einigen MEMS-Lautsprechern eingesetzt worden. Das Magnetfeld wird durch einen Permanentmagneten erzeugt. Im Magnetfeld wird eine stromdurchflossene Spule platziert. Üblicherweise wird die Spule durch Abscheidung und Strukturierung einer Metallschicht in die Membran integriert und der Permanentmagnet in der Assemblierung als externe Komponente zugefügt. Die Komplexität und die Einschränkungen bezüglich der Integration aller Komponenten in MEMS-Technologie stellen einen ähnlich großen Nachteil dar, wie bei den elektromagnetisch betriebenen Wandlern.

Magnetostriktiv betriebene Wandler beruhen auf einer Kontraktion oder Expansion einer Funktionsschicht bei anliegendem Magnetfeld. Bspw. ist Vanadium Permadur positiv magnetostriktiv, d. h., weist eine Expansion bei anliegendem Magnetfeld auf. Diese Kontraktion kann bei geeignetem Aufbau zur Erzeugung einer Membranschwingung genutzt werden. In [1] wurde als magnetostriktive Funktionsschicht Vanadium Permendur ($Fe_{49}Co_{49}V_2$) eingesetzt, das über eine Chrom-Haftschicht auf $SiO_2$ (Siliziumdioxid) abgeschieden wurde. Das externe Magnetfeld wird über eine Mikroflachspule zur Verfügung gestellt, welche durch galvanisch abgeschiedenes Kupfer realisiert wurde. Bezüglich Komplexität und Einschränkungen bei der Integration sind ähnliche Nachteile anzuführen, wie bei den beiden vorgenannten Wirkprinzipien.

Die vorangehend beschriebenen klassischen und am weitesten verbreiteten Varianten, die als gemeinsames Merkmal die Verwendung einer Membran haben, welche zu einer Schwingung angeregt werden kann, wird nachfolgend durch bestimmte Abwandlungen, die aufgrund spezieller Nachteile des klassischen Membranprinzips untersucht wurden, ergänzt.

Flexible Membranen können auch höhere Moden im Hörschallbereich aufweisen und somit zu parasitären Schwingungen führen, was die akustische Qualität senkt (Klirrfaktor), vgl. [1]. Zur Vermeidung bzw. Verringerung dieses Effektes werden daher Platten eingesetzt, welche eine deutlich höhere Steifigkeit aufweisen. Eine solche Platte wird über eine sehr weiche Aufhängung, die auch den akustischen Kurzschluss vermeiden soll, mit dem Chip verbunden, siehe hierzu [5].

Eine weitere Abwandlung stellt eine segmentierte Membran dar, die bei den oben beschriebenen magnetostriktiven Wandlern eingesetzt wurde. Dies entspricht einer speziellen topographischen Lösung für das Problem, dass sich die Funktionsschicht in zwei Richtungen kontrahiert oder expandiert. Konkret besteht der Aufbau aus mehreren auslenkbaren Biegebalken. Nach [1] kann die Anordnung für Abstände der Balken kleiner oder gleich 3 μm als akustisch geschlossen angesehen werden. Durch entsprechende Dimensionierung der einzelnen Balken bezüglich einer Resonanzfrequenz und der Abstände zwischen den Balken kann eine vergleichsweise hohe akustische Bandbreite erreicht werden und der Verlauf des Schallpegels als Funktion der Schwingungsfrequenz angepasst bzw. optimiert werden.

Neumann et al. verfolgen in [6] den Ansatz, statt einer einzelnen, großen Membran eine Vielzahl kleiner Teilmembranen zu verwenden. Jede Teilmembran hat eine so hohe Resonanzfrequenz, dass im Hörschallbereich eine quasistatische Auslenkung erfolgen kann. Damit wird insbesondere ein digitaler Betrieb des Lautsprechers ermöglicht.

Zusammenfassend lässt sich schlussfolgern, dass bezüglich Integration bekannte elektrostatisch betriebene Membranlautsprecher verhältnismäßig geringe Auslenkungen aufweisen, wenn moderate Antriebsspannungen vorausgesetzt werden. Als Referenz kann beispielsweise der elektrostatische Membranlautsprecher von Kim et al. gemäß [3] dienen. Jede der beiden Membranen hat eine Fläche von 2 x 2 mm². In einem Abstand von jeweils 7,5 μm ist die obere bzw. untere Elektrode angebracht. Je nach Geometrie der Membran und der Zunahme der Membransteifigkeit mit wachsender Auslenkung wird die Auslenkung aufgrund des sogenannten Pull-In-Effekts auf typischerweise 1/3 bis 1/2 des Elektrodenabstands beschränkt. Wird der höhere Wert von 1/2 angenommen, ergibt sich für die Auslenkung 7,5 μm / 2, und zwar einmal in die eine und einmal in die andere Richtung. Das verdrängte Volumen lässt sich abschätzen, indem davon ausgegangen wird, dass es dem Volumen einer ausgelenkten steifen Platte mit einer Auslenkung der halben Maximalauslenkung der Membran entspricht. Dafür ergibt sich beispielsweise:

$$\Delta V \approx (2 \times 2 \text{ mm}^2) \times 50 \text{ \%} * (2 \times 7.5 \text{ μm}) / 2 = 15 \times 10^{-3} \text{ mm}^3 \qquad \text{(Glg. 1)}$$

bzw.

$$\Delta V / \text{ aktive Fläche} = \Delta V / A = \Delta V / 4 \text{ mm}^2 = 3.75 \times 10^{-3} \text{ mm} \qquad \text{(Glg. 2)}$$

Ein generelles Problem bei der Herstellung von miniaturisierten Membran-Lautsprechern ist es, einen flachen Verlauf des Schalldrucks als Funktion der Frequenz zu erreichen. Der erreichbare Schalldruck ist proportional zur Strahlungsimpedanz und der Geschwindigkeit der Membran. Im Makroskopischen ist der Membrandurchmesser vergleichbar mit der akustischen Wellenlänge. Hier gilt, dass die Strahlungsimpedanz proportional zur Frequenz ist, vgl. [6]. Qualitativ hochwertige Lautsprecher werden häufig so entworfen, dass die Resonanz $f_0$ unterhalb des Hörschallbereichs liegt (für Mehrwegelautsprecher liegt die jeweilige Resonanzfrequenz unterhalb der unteren Kantenfrequenz des entsprechenden elektrischen Filters). Für f >> $f_0$ ist die Geschwindigkeit der Membran damit proportional zu 1/f. Insgesamt ergibt sich dann für die Frequenzabhängigkeit des Schalldrucks p der Ausdruck p $\propto$ 1. Es ergibt sich also in dieser (vereinfachten) Betrachtung ein vollständig flacher Verlauf der Schalldruckkurve.

Sobald der Durchmesser der Schallquelle / der Membran viel kleiner ist als die zu erzeugende Schallwellenlänge, kann für die Strahlungsimpedanz eine quadratische Abhängigkeit von der Frequenz angenommen werden, wie es in [7] beschrieben ist. Dies ist für MEMS-Lautsprecher mit Membranen in der Größenordnung von Millimetern gegeben. Wird wie oben f >> $f_0$ angenommen, so ergibt sich für den Verlauf der Schalldruckkurve die Abhängigkeit p $\propto$ f. Niedrige Frequenzen werden im Verhältnis zu den hohen Frequenzen mit zu geringem Schalldruck wiedergegeben. Bei quasistatischem Betrieb ist die Membrangeschwindigkeit proportional zu f. Es ergibt sich dann für den Schalldruckverlauf die für niedrige Frequenzen noch ungünstigere Abhängigkeit p $\propto$ $f^3$. DE 10 2015 210919 A1 offenbart einen MEMS-Wandler zum Interagieren mit einem Volumenstrom eines Fluids. Der MEMS-Wandler umfasst ein Substrat, das einen Stapel von Schichten und eine Kavität in dem Schichtstapel aufweist, sowie einen elektromechanischen Wandler, der mit dem Substrat in der Kavität verbunden ist. Dieser elektromechanische Wandler besitzt ein in einer Bewegungsebene der Substratebenen verformbares Element.

Miniaturisierte multifunktionale Systeme für die Mensch-Computer- bzw. Mensch-Maschine- sind auf verschiedenen Integrationsstufen erhältlich.

Stufe 1: Leiterplatten-Hybridintegration

Sensoren, Aktoren und elektronische Schaltkreise, die auf verschiedenen Substraten hergestellt wurden, werden auf einem gemeinsamen Verdrahtungsträger (hier: Leiterplatte) zusammengefasst.

Stufe 2: System-In-Package

Mindestens zwei Chips werden in einem speziellen Gehäuse zu einem System verbunden. Teilweise werden dafür auch Chip-auf-Chip-Bondungen eingesetzt. Oft werden auch Silizium-basierte Verdrahtungsträger verwendet (so genannte "Interposer-Technologien").

Stufe 3: Waferlevel-Hybridintegration und monolithische Integration

Sensoren, Aktoren und elektronische Schaltkreise, die teilweise auf unterschiedlichen Wafern hergestellt wurden, werden auf Waferebene miteinander verbunden. Die einzelnen Chips des gebondeten Waferstapels werden aus dem Verbund durch entsprechende Vereinzelungsprozesse herausgelöst. Bei der monolithischen Integration sind Sensoren, Aktoren und elektronische Schaltkreise auf einem Wafer realisiert. In beiden Fällen ist die elektrische Verbindung der Elemente durch integrierte Leitbahnen oder Chip-Durchkontaktierungen ("Through-Silicon-Vias", kurz: TSV) realisiert.

Ein Beispiel eines weit fortgeschrittenen hybrid integrierten Sensor-Aktor-Systems (Stufe I) sind die sogenannten Hearables. In Form eines In-Ear-Kopfhörers werden dabei heute bereits folgende Komponenten untergebracht: Lautsprecher (feinmechanisch hergestellt), Akku, Speicherchip, CPU, Rot- und Infrarot-Sensoren, Temperatursensoren, Optischer Touch-Sensor, Mikrofone, 3-Achsen-Beschleunigungs-Sensor, 3-Achsen-Magnetfeld-Sensor und 3-Achsen-Lage-Sensor. Ein solcher "System-in-a-Package"-Ansatz wird heute für Hearables von Firmen wie z. B. Bragi ("The Dash"), Samsung, Motorola und Sony eingesetzt.

Ein Beispiel eines weit fortgeschrittenen System-in-Package Sensor-Systems (Stufe 2) sind sogenannte 9-Achsen-Sensoren. Hier sind z. B. 3-Achsen-Beschleunigungs-Sensor und 3-Achsen-Lage-Sensor auf einem, ein-3-Achsen-Magnetfeld-Sensor auf einem zweiten Silizium-Chip und eine elektronische Schaltung auf einem dritten Chip realisiert und in einem gemeinsamen Gehäuse untergebracht. Ein Anbieter eines solchen Systems ist z. B. die Firma InvenSense (MPU-9150).

Beispiele für monolithisch integrierte Sensor-Systeme sind ein- oder mehrachsige Gyroskope bzw. Beschleunigungssensoren, Drucksensoren und Magnetfeldsensoren, bei denen jeweils das Sensorelement und elektronische Funktionen in einem einzigen Chip realisiert sind.

Die monolithische Integration bietet hinsichtlich der Baugröße sehr große Vorteile. Während im Falle der Leiterplatten-Hybridintegration für jedes Bauelement eine separates Gehäuse benötigt wird und beim System-in-Package ein verhältnismäßig großes Gehäuse vor allem bei Kombination mehrerer Einzelchips, entfällt dieser zusätzliche Platzbedarf bei der monolithischer Integration. Darüber hinaus sinken durch den Wegfall der komplexen, hybriden Aufbautechniken die Herstellungskosten gerade bei hohen Stückzahlen signifikant.

Im hier adressierten Fall miniaturisierter schallerzeugender (Mikrolautsprecher) oder schallaufnehmender (Mikrophon) MEMS-Komponenten ist die monolithische Integration mit Schaltkreisen oder anderen aktorischen oder sensorischen Elementen heute sehr stark eingeschränkt. Der Grund liegt zum einen in der Funktionsweise der MEMS-Mikrophone bzw. -Lautsprecher. Es wird in beiden Fällen eine verhältnismäßig große Membran benötigt, die in der Chipfläche gebildet wird. Die Chipfläche auf der Oberseite und Unterseite wird größtenteils für die bei der Bewegung der Membran entstehende Luftströmung benötigt. Es steht damit keine signifikante Chipfläche zur Verfügung, um weitere Funktionalitäten zu integrieren. Einer prinzipiell möglichen Vergrößerung der Chipoberfläche steht vor allem eine mit Chipfläche überproportional sinkende Ausbeute entgegen. Zum anderen sind die Herstellprozesse häufig nicht direkt kompatibel mit den Herstellprozessen der CMOS Schaltung, was die Komplexität des Gesamtprozesses erhöht. Aus diesen beiden Gründen werden daher heute bei den akustischen Komponenten weitere Funktionalitäten auf einem oder mehreren separaten Chips realisiert und das gesamte System über Hybridintegration/System-In-Package zusammengeführt.

Gleichzeitig besteht ein Bedarf, einen möglichst geringen Bauraum von MEMS-Wandlern zu erhalten, um einen Einsatz in portablen Vorrichtungen, etwa in Kopfhörern und insbesondere so genannte In-Ear-Kopförer (Im-Ohr-Kopfhörer) zu ermöglichen.

Wünschenswert wäre demnach ein Konzept für verbesserte MEMS-Wandler, die einen hohen Wirkungsgrad bei gleichzeitig geringem Bauraumbedarf aufweisen.

Die Aufgabe der vorliegenden Erfindung besteht deshalb darin, einen MEMS-Wandler und ein Verfahren zum Herstellen desselben zu schaffen, die einen Volumenstrom eines Fluids mit einem hohen Wirkungsgrad beeinflussen können und/oder von dem Volumenstrom mit einem hohen Wirkungsgrad beeinflusst werden können und dies mit einem geringen Bauraumbedarf ermöglichen.

Diese Aufgabe wird durch den Gegenstand der unabhängigen Patentansprüche gelöst.

Der Kerngedanke der vorliegenden Erfindung besteht darin, erkannt zu haben, dass obige Aufgabe dadurch gelöst werden kann, dass ein Volumenstrom eines Fluids besonders effizient mittels eines Elements beeinflussbar ist, das in einer Bewegungsebene (in-plane) verformbar ist bzw. das der Volumenstrom ein derartiges Element besonders effizient auslenken kann. Dies ermöglicht große Flächen des verformbaren Elements, die mit dem Volumenstrom in eine Wechselwirkung treten können, bei gleichzeitiger geringen Abmessungen einer Chipoberfläche, so dass insgesamt eine effiziente MEMS-Wandlervorrichtung mit einem hohen Wirkungsgrad erhalten wird. Ferner wurde erkannt, dass eine Integration einer elektronischen Schaltung zum Betrieb des MEMS in eine Schicht des Schichtstapels ansonsten ungenutzte Halbleiterflächen für die elektronische Schaltung nutzbar macht, was zu einem geringen Bauraumbedarf der Vorrichtung führt.

Gemäß der Erfindung umfasst ein MEMS-Wandler zum Interagieren mit einem Volumenstrom eines Fluids ein Substrat, das einen Schichtstapel mit einer Mehrzahl von Schichten aufweist. Das Substrat weist eine Kavität in dem Schichtstapel auf. Die Schichten bilden eine Mehrzahl von Substratebenen. Der MEMS-Wandler umfasst einen elektromechanischen Wandler, der mit dem Substrat in der Kavität verbunden ist und weist ein sich in zumindest einer Bewegungsebene der Mehrzahl von Substratebenen verformbares Element auf. Eine Verformung des verformbaren Elements in der Bewegungsebene und der Volumenstrom des Fluids hängen kausal zusammen. Der MEMS-Wandler umfasst ferner eine elektronische Schaltung, die in einer Schicht des Schichtstapels angeordnet ist. Die elektronische Schaltung ist mit dem elektromechanischen Wandler verbunden, und ausgebildet, um ein elektrisches Ansteuersignal in eine Auslenkung des verformbaren Elements zu konvertieren. Die elektronische Schaltung umfasst einen schaltenden Verstärker, der aus-gebildet ist, um ein digitales pulsweitenmoduliertes Ansteuersignal für das verformbare Element bereitzustellen.

Gemäß einem Ausführungsbeispiel umfasst eine Vorrichtung einen beschriebenen MEMS-Wandler, der als MEMS-Lautsprecher gebildet ist, wobei die Vorrichtung als mobile Musikwiedergabevorrichtung oder als Kopfhörer ausgebildet ist.

Gemäß einem weiteren Ausführungsbeispiel umfasst ein Gesundheitsassistenzsystem eine Sensoreinrichtung zum Erfassen einer Vitalfunktion eines Körpers und zum Ausgeben eines Sensorsignals basierend auf der erfassten Vitalfunktion. Das Gesundheitsassistenzsystem umfasst eine Verarbeitungseinrichtung zum Verarbeiten des Sensorsignals und zu Bereitstellen eines Ausgabesignals basierend auf der Verarbeitung und umfasst einen Kopfhörer umfassend einen beschriebenen MEMS-Wandler. Der MEMS-Wandler ist als Lautsprecher ausgeführt und umfasst eine Drahtlos-Kommunikationsschnittstelle zum Empfangen des Ausgabesignals und ist ausgebildet, um aufbauend hierauf ein akustisches Signal wiederzugeben.

Gemäß der Erfindung umfasst ein Verfahren zum Bereitstellen eines MEMS-Wandler zum Interagieren mit einem Volumenstrom eines Fluids ein Bereitstellen eines Substrats, das einen Schichtstapel mit einer Mehrzahl von Schichten, die eine Mehrzahl von Substratebenen bilden, und das eine Kavität in dem Schichtstapel aufweist. Das Verfahren umfasst ein Erzeugen eines elektromechanischen Wandlers, in dem Substrat, so dass dieser mit dem Substrat in der Kavität verbunden ist und ein sich in zumindest einer Bewegungsebene der Mehrzahl von Substratebenen verformbares Element aufweist, wobei eine Verformung des verformbaren Elements in der Bewegungsebene und der Volumenstrom des Fluids kausal zusammenhängen. Das Verfahren umfasst ferner ein Anordnen einer elektronischen Schaltung in einer Schicht des Schichtstapels, so dass die elektronische Schaltung mit dem elektromechanischen Wandler verbunden ist, und ausgebildet ist, um eine Konvertierung zwischen einer Verformung des verformbaren Elements und einem elektrischen Signal bereitzustellen. Die elektronische Schaltung wird so eingerichtet, um ein elektrisches Ansteuersignal in eine Auslenkung des verformbaren Elements zu konvertieren. Die elektronische Schaltung umfasst einen schaltenden Verstärker, der aus-gebildet ist, um ein digitales pulsweitenmoduliertes Ansteuersignal für das verformbare Element bereitzustellen

Weitere vorteilhafte Ausführungsformen sind der Gegenstand der abhängigen Patentansprüche.

Bevorzugte Ausführungsbeispiele der vorliegenden Erfindung werden nachfolgend Bezug nehmend auf die beiliegenden Zeichnungen erläutert. Diese Zeichnungen zeigen vor allem Beispiele für die Mikromechanik erfindungsgemäßer MEMS-Wandler. Zu einem erfindungsgemäßen MEMS-Wandler gehört aber stets auch eine elektronische Schaltung mit den in den unabhängigen Ansprüchen definierten Merkmalen. Es zeigen:

Fig. 1     eine schematische perspektivische Ansicht eines MEMS-Wandlers gemäß einem Ausführungsbeispiel;

Fig. 2a     eine schematische perspektivische Ansicht eines MEMS-Wandlers, der eine Vielzahl elektromechanischer Wandler umfasst, gemäß einem Ausführungsbeispiel;

Fig. 2b     eine schematische Aufsicht des MEMS-Wandlers aus Fig. 2a gemäß einem Ausführungsbeispiel.

Fig. 2c     eine schematische perspektivische Ansicht des MEMS-Wandlers aus Fig. 2a, bei dem die elektromechanischen Wandler einen verformten Zustand eines verformbaren Elements aufweisen, gemäß einem Ausführungsbeispiel;

Fig. 3     eine schematische perspektivische Ansicht eines verformbaren Elements, das als Bimorph ausgeführt ist, gemäß einem Ausführungsbeispiel;

Fig. 4a     eine schematische perspektivische Ansicht eines verformbaren Elements, das drei Bimorphstrukturen aufweist, gemäß einem Ausführungsbeispiel;

Fig. 4b     eine schematische perspektivische Ansicht des verformbaren Elements gem. Fig. 4a in einem ausgelenkten Zustand gemäß einem Ausführungsbeispiel;

Fig. 4c     eine schematische Aufsicht auf eine Anordnung zweier verformbarer Elemente, die benachbart zueinander angeordnet sind gemäß einem Ausführungsbeispiel;

Fig. 5     eine schematische Aufsicht auf einen MEMS-Wandler, bei dem die elektromechanischen Wandler verglichen mit dem MEMS-Wandler aus Fig. 2a eine veränderte Konfiguration aufweisen, gemäß einem Ausführungsbeispiel;

Fig. 6a     eine schematische Aufsicht auf einen elektromechanischen Wandler, bei dem gerade ausgebildete Federelemente zwischen Plattenelementen und verformbaren Elementen angeordnet sind, gemäß einem Ausführungsbeispiel;

Fig. 6b     eine schematische Aufsicht auf einen elektromechanischen Wandler, bei dem Federelemente von auslenkbaren Enden der verformbaren Elemente mit einem Winkel von weniger als 90° angeordnet sind, gemäß einem Ausführungsbeispiel;

Fig. 6c     eine schematische Aufsicht auf einen elektromechanischen Wandler, bei dem die Federelemente mit einem Winkel von mehr als 90° angeordnet sind, gemäß einem Ausführungsbeispiel;

Fig. 6d     eine schematische Aufsicht auf einen elektromechanischen Wandler, bei dem das Substrats benachbart zu einem verformbaren Element ein Federelement aufweist, gemäß einem Ausführungsbeispiel;

Fig. 6e     eine schematische Aufsicht auf einen elektromechanischen Wandler, bei dem Plattenelemente Aussparungen aufweisen, gemäß einem Ausführungsbeispiel;

Fig. 7a     eine schematische Aufsicht auf ein verformbares Element, das mit dem Plattenelement verbunden ist, gemäß einem Ausführungsbeispiel;

Fig. 7b     eine schematische Aufsicht auf eine Konfiguration, bei der das verformbare Element zwischen dem Substrat fest eingespannt ist und ausgebildet ist, gemäß einem Ausführungsbeispiel;

Fig. 7c     eine schematische Aufsicht auf eine Konfiguration des elektromechanischen Wandlers, bei dem die verformbaren Elemente in einem Mittenbereich Aussparungen aufweisen, gemäß einem Ausführungsbeispiel;

Fig. 7d     eine schematische Aufsicht auf eine Konfiguration, des elektromechanischen Wandlers bei der ein erstes verformbares Element und ein zweites verformbares Element parallel zu einander angeordnet sind;

Fig. 8a     eine schematische perspektivische Ansicht eines MEMS-Wandlers, bei dem die verformbaren Elemente alternierend mit dem Substrat bzw. mit einem Ankerelement verbunden sind, gemäß einem Ausführungsbeispiel;

Fig. 8b     eine schematische Aufsicht des MEMS-Wandlers aus Fig. 8a gemäß einem Ausführungsbeispiel;

Fig. 8c     eine schematische perspektivische Ansicht des MEMS-Wandlers aus Fig. 8a in einem ausgelenkten Zustand gemäß einem Ausführungsbeispiel;

Fig. 8d     eine schematische Aufsicht auf den MEMS-Wandler aus Fig. 8b in dem ausgelenkten Zustand gemäß einem Ausführungsbeispiel;

Fig. 9     eine schematische perspektivische Ansicht eines Stapels, der drei MEMS-Wandler aufweist, gemäß einem Ausführungsbeispiel;

Fig. 10     eine schematische perspektivische Aufsicht auf einen Ausschnitt eines MEMS-Wandlers, bei dem zwischen Seiten des Substrats verformbare Elemente angeordnet sind, gemäß einem Ausführungsbeispiel;

Fig. 11a     eine schematische Aufsicht auf einen Ausschnitt eines MEMS-Wandlers, bei dem die elektromechanischen Wandler bezogen auf eine laterale Richtung des Substrats schräg angeordnet ist, gemäß einem Ausführungsbeispiel;

Fig. 11b     eine schematische Aufsicht auf einen Ausschnitt eines MEMS-Wandlers, der als Pumpe einsetzbar ist; gemäß einem Ausführungsbeispiel;

Fig. 12a     eine schematische Aufsicht auf einen Ausschnitt eines MEMS-Wandlers, der bspw. als MEMS-Pumpe einsetzbar ist, in einem ersten Zustand;

Fig. 12b     den MEMS-Wandler aus Fig. 12a in einem zweiten Zustand;

Fig. 13     eine schematische Ansicht zweier verformbaren Elemente, die entlang einer lateralen Erstreckungsrichtung miteinander verbunden sind, gemäß einem Ausführungsbeispiel;

Fig. 14     eine schematische Ansicht eines Stapels umfassend zwei MEMS-Wandler, die miteinander verbunden sind und eine gemeinsame Schicht aufweisen, gemäß einem Ausführungsbeispiel;

Fig. 15     eine schematische Seitenschnittansicht eines verformbaren Elements, das zwei Schichten aufweist, die über Verbindungselemente miteinander beabstandet und verbunden sind, gemäß einem Ausführungsbeispiel;

Fig. 16     eine schematische Aufsicht auf ein verformbares Element, das benachbart zu einer Elektrode angeordnet ist, gemäß einem Ausführungsbeispiel;

Fig. 17a     eine schematische perspektivische Ansicht eines MEMS-Wandlers gemäß einem weiteren Ausführungsbeispiel von einer ersten Seite;

Fig. 17b     eine schematische Ansicht des MEMS-Wandlers aus Fig. 17a von einer zweiten Seite;

Fig. 17c     eine schematische perspektivische Ansicht des MEMS-Wandlers gemäß der Ansicht in Fig. 17a, bei dem gemäß einem weiteren Ausführungsbeispiel Öffnungen so ausgestaltet sind, dass Gitterstege angeordnet sind;

Fig. 18a     eine schematische perspektivische Ansicht eines MEMS-Wandlers gemäß einem Ausführungsbeispiel, bei dem benachbart zu einer elektronischen Schaltung ein Funktionselement angeordnet ist;

Fig. 18b     eine schematische perspektivische Ansicht eines MEMS-Wandlers, der gegenüber dem MEMS-Wandler aus Fig. 18a dahin gehend modifiziert ist, dass gemäß einem Ausführungsbeispiel eine Öffnung in einer Deckelschicht angeordnet ist;

Fig. 19a     eine schematische Ansicht einer Schicht, zur Anpassung von Abstandsrastern gemäß einem Ausführungsbeispiel;

Fig. 19b     eine schematische Ansicht einer Verwendung einer Adaptionsschicht gemäß einem Ausführungsbeispiel,

Fig. 20     ein schematisches Blockschaltbild eines MEMS-Systems gemäß einem Ausführungsbeispiel;

Fig. 21a     eine Vorrichtungen gemäß einem Ausführungsbeispiel;

Fig. 21b     ein schematisches Blockdiagramm eines weiteren Systems gemäß einem Ausführungsbeispiel, das als Universalübersetzer und/oder als Navigationsassistenzsystem ausgebildet sein kann;

Fig. 22     eine schematische Darstellung eines Gesundheitsassistenzsystems gemäß einem Ausführungsbeispiel;

Fig. 23     eine schematische Aufsicht auf einen MEMS-Wandler gemäß einem Ausführungsbeispiel, der eine Vielzahl von elektromechanischen Wandlern mit einseitig eingespannten Balkenelementen aufweist; und

Fig. 24     eine schematische Aufsicht auf einen MEMS-Wandler gemäß einem Ausführungsbeispiel, der eine Vielzahl von elektromechanischen Wandlern mit zweiseitig eingespannten Balkenelementen aufweist.

Bevor nachfolgend Ausführungsbeispiele der vorliegenden Erfindung im Detail anhand der Zeichnungen näher erläutert werden, wird darauf hingewiesen, dass identische, funktionsgleiche oder gleichwirkende Elemente, Objekte und/oder Strukturen in den unterschiedlichen Figuren mit den gleichen Bezugszeichen versehen sind, so dass die in unterschiedlichen Ausführungsbeispielen dargestellte Beschreibung dieser Elemente untereinander austauschbar ist bzw. aufeinander angewendet werden kann.

Nachfolgend wird Bezug genommen auf MEMS-Wandler (MEMS = mikroelektromechanisches System). Ein MEMS-Wandler kann eine oder mehrere elektroaktive Komponenten aufweisen, die basierend auf einer angelegten elektrischen Größe (Strom, Spannung, Ladung oder dergleichen) eine Veränderung in einer mechanischen Komponente bewirken. Diese Veränderung kann sich beispielsweise auf eine Verformung, auf eine Erwärmung oder eine Verspannung der mechanischen Komponente beziehen. Alternativ oder zusätzlich kann eine mechanische Beeinflussung der Komponente, etwa eine Verformung, eine Erwärmung oder eine Verspannung, zu einem elektrischen Signal oder einer elektrischen Information (Spannung, Strom, Ladung oder dergleichen) führen, die an elektrischen Anschlüssen der Komponente erfasst werden kann. Manche Materialien oder Komponenten weisen eine Reziprozität auf, das bedeutet, die Effekte sind wechselseitig vertauschbar. Beispielsweise können Piezomaterialien den inversen piezoelektrischen Effekt (Verformung basierend auf einem angelegten elektrischen Signal) und den piezoelektrischen Effekt (Bereitstellen einer elektrischen Ladung basierend auf einer Verformung) aufweisen.

Manche der nachfolgend beschriebenen Ausführungsbeispiele beziehen sich auf vollintegrierte und hochminiaturisierte Systeme für Mensch-Computer- bzw. Mensch-Maschine-Schnittstellen-Anwendungen und -Interaktion inklusive Anwendungen im Bereich "persönlicher Assistent (personal assistent)". Dabei steht die akustische Schnittstelle im Mittelpunkt. Die Ausführungsbeispiele beziehen sich auf die Integration von MEMS und CMOS.

Manche der nachfolgend beschriebenen Ausführungsbeispiele beziehen sich darauf, dass ein verformbares Element eines elektromechanischen Wandlers ausgebildet ist, um mit einem Volumenstrom eines Fluids zu interagieren. Eine Interaktion kann beispielsweise eine Verformung des verformbaren Elements, bewirkt durch eine elektrisches Ansteuersignal, umfassen, das zu einer Bewegung, Verschiebung, Kompression oder Dekompression des Fluids führt. Alternativ oder zusätzlich kann der Volumenstrom des Fluids das verformbare Element verformen, so dass basierend auf der Wechselwirkung zwischen dem Volumenstrom und dem verformbaren Element eine Anwesenheit, eine Charakteristik (Druck, Strömungsgeschwindigkeit oder dergleichen) oder eine andere Information bezüglich des Fluids (etwa eine Temperatur) erhalten werden kann. Das bedeutet, dass eine Verformung des verformbaren Elements entlang der lateralen Bewegungsrichtung und der Volumenstrom des Fluids kausal zusammenhängen. MEMS können beispielsweise in Siliziumtechnologie hergestellt werden. Der elektromechanische Wandler kann das verformbare Element und weitere Elemente, etwa Elektroden und/oder elektrische Anschlüsse umfassen. Das verformbare Element kann ausgebildet sein, um sich (makroskopisch) entlang einer lateralen Bewegungsrichtung zu verformen, d. h., ein Element oder Bereich kann entlang der lateralen Bewegungsrichtung beweglich sein. Bei dem Element oder Bereich kann es sich bspw. um ein Balkenende oder um einen Mittenbereich einer Balkenstruktur handeln. Mikroskopisch betrachtet, kann bei einer Verformung des verformbaren Elements entlang der lateralen Bewegungsrichtung eine Verformung des verformbaren Elementes senkrecht zu der lateralen Bewegungsrichtung auftreten. Nachfolgend beschriebene Ausführungsbeispiele beziehen sich auf die makroskopische Betrachtungsweise.

Ausführungsbeispiele können miniaturisierte, in Silizium gefertigte Lautsprecher, Mikrophone und/oder Pumpen zur Verfügung stellen, die bezogen auf ihre jeweilige Baugröße einen möglichst hohen Schallpegel, eine möglichst hohe Empfindlichkeit und/oder eine möglichst hohe Flussrate des Fluids erzeugen können. Andere Ausführungsbeispiele können einen MEMS-Wandler, ein MEMS-Ventil und/oder ein MEMS-Dosiersystem schaffen.

Ausführungsbeispiele der vorliegenden Erfindung können genutzt werden, um Luftschall zu erzeugen, insbesondere im Hörschallbereich. Ausführungsbeispiele beziehen sich somit auf Lautsprecher, insbesondere miniaturisierte Lautsprecher, etwa für Hörgeräte, Kopfhörer darunter In-Ear-Kopfhörer (Im-Ohr-Kopfhörer), Headsets, Mobiltelefone oder dergleichen. Der wechselseitige kausale Zusammenhang zwischen dem Volumenstrom und der Verformung des verformbaren Elements ermöglicht auch eine Anwendung in Lautsprechern. Ausführungsbeispiele beziehen sich somit auf elektroakustische Wandler.

Ausführungsbeispiele ermöglichen es, ein möglichst stark miniaturisiertes, multifunktionales System zur Verfügung zu stellen, das in der Mensch-Computer- bzw. Mensch-Maschine-Interaktion eingesetzt werden kann und in Volumenproduktion kostengünstig herstellbar ist. Die Kernfunktionalität, die durch weitere Funktionen ergänzt werden soll, liegt dabei im Bereich der Schallerzeugung bzw. der Schallaufnahme.

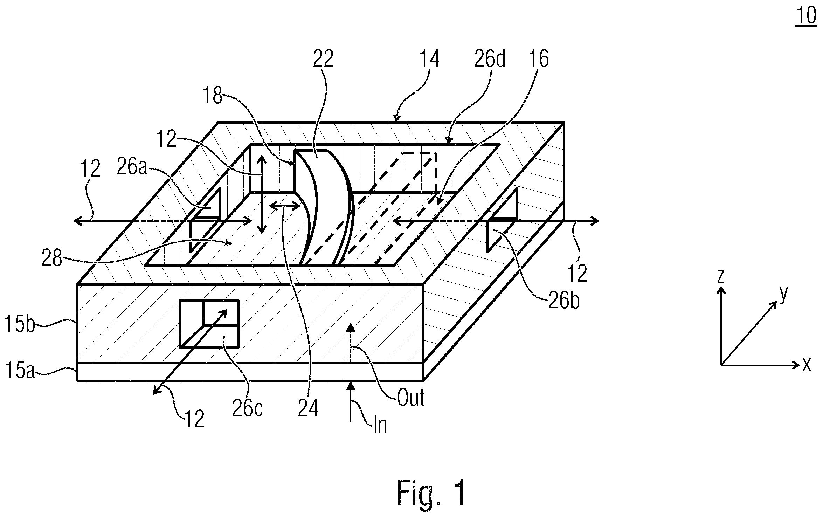

Fig. 1 zeigt eine schematische perspektivische Ansicht von Teilen eines MEMS-Wandlers 10 gemäß einem Ausführungsbeispiel. Der MEMS-Wandler 10 ist ausgebildet, um mit einem Volumenstrom 12 eines Fluids zu interagieren. Bei dem Fluid kann es sich um ein Gas (etwa Luft) und/oder eine Flüssigkeit handeln. Beispielsweise kann es sich bei der Flüssigkeit um eine medizinische Lösung, ein Medikament, eine Chemikalie für einen technischen Prozess oder dergleichen handeln.

Der MEMS-Wandler 10 weist ein Substrat 14 auf. Das Substrat 14 kann ein beliebiges Material aufweisen. Beispielsweise kann das Substrat 14 ein Holzmaterial, ein Metallmaterial und/oder ein Halbleitermaterial, etwa ein Siliziummaterial umfassen. Das Substrat 14 umfasst eine Kavität 16. Die Kavität 16 kann beispielsweise als Aussparung oder als zumindest teilweise umschlossenes Volumen des Substrats 14 verstanden werden. In der Kavität 16 kann zumindest bereichsweise das Fluid des Volumenstroms 12 angeordnet sein. Das Substrat 14 umfasst zumindest eine Materialschicht, etwa eine einstückige Schicht die die Kavität 16 aufweist, wie es bspw. durch Fräsen oder Ätzen aus einem Material heraus erhalten werden kann, und/oder wenn bei einem Erzeugen des Substrats 14 die Kavität 16 ausgelassen wird, so dass das Substrat 14 um die Kavität 16 herum erzeugt wird. Das Substrat 14 kann auch mehrere Schichten 15a und 15b aufweisen, was bspw. bei einer Halbleiterfertigung vorteilhaft sein kann. Unterschiedliche Strukturen, etwa die Anwesenheit und die Abwesenheit der Kavität 16, können in unterschiedlichen Schichten des Substrats 14 implementiert sein. Alternativ kann das Substrat auch eine höhere Anzahl von Schichten aufweisen, wie es nachfolgend erläutert wird.

Der MEMS-Wandler 10 umfasst einen elektromechanischen Wandler 18. Der elektromechanische Wandler 18 ist mit dem Substrat 14 verbunden. Der elektromechanische Wandler 18 umfasst ein verformbares Element 22, das entlang einer lateralen Bewegungsrichtung 24 verformbar ist. Beispielsweise kann ein Anlegen eines elektrischen Signals an den elektromechanischen Wandler 18 zu der Verformung des verformbaren Elements 22 entlang der lateralen Bewegungsrichtung 24 führen. Alternativ oder zusätzlich kann der Volumenstrom 12, wenn er auf das verformbare Element 22 trifft, dazu führen, dass das verformbare Element 22 die Verformung ausführt, so dass ein elektrisches Signal von dem elektromechanischen Wandler 18, das auf dem Volumenstrom 12 basiert, erhalten werden kann. D. h., dass die Verformung des verformbaren Elements 22 und der Volumenstrom 12 kausal zusammenhängen. Beispielsweise kann der elektromechanische Wandler 18 zumindest eine, etwa zwei, piezoelektrische Schicht umfassen oder daraus bestehen. Beide Schichten können durch elektrische Spannung verformt werden. Der elektromechanische Wandler kann weitere Elemente umfassen, bspw. Elektroden.

Der MEMS-Wandler 10 umfasst eine nicht dargestellte elektronische Schaltung, die in zumindest einer der Schichten 15a oder 15b des Schichtstapels angeordnet ist. Die nicht dargestellte elektronische Schaltung ist mit dem elektromechanischen Wandler 18 verbunden, und ausgebildet ist, um eine Konvertierung zwischen einer Verformung des verformbaren Elements 22 und einem elektrischen Signal bereitzustellen. Das bedeutet, die nicht dargestellte elektronische Schaltung kann je nach Implementierung des MEMS-Wandlers zur sensorischen und/oder aktuatorischen Ansteuerung des MEMS-Wandlers 10 genutzt werden. Eine Anordnung der elektronischen Schaltung in einer Deckelschicht des Substrats 14, etwa der Schicht 15a, kann ermöglichen, eine große Fläche der Deckelschicht für die elektronische Fläche zu nutzen, was eine entsprechende Einsparung von Chipfläche und/oder Bauraum bei anderen Bauteilen ermöglicht, etwa Platinen, auf die der MEMS-Wandler 10 montiert wird. Eine Anordnung zumindest von Teilen der elektronischen Schaltung in einer Deckelfläche des Schichtstapels kann ferner den Vorteil ermöglichen, dass eine einfache Kontaktierung des MEMS-Wandlers mit anderen elektronischen Bauteilen ermöglicht ist.

Der Begriff Konvertierung ist hierbei als Umsetzung von einer Eingangsgröße in eine Ausgangsgröße zu verstehen. Die elektronische Schaltung ist ausgebildet, um ein elektrisches Ansteuersignal in eine Auslenkung eines oder mehrerer verformbaren Elemente zu konvertieren, d. h., eine aktuatorische Ansteuerung vorzunehmen, und optional um eine Verformung eines oder mehrerer verformbaren Elemente in ein elektrisches Ausgangssignal zu konvertieren, d. h., eine sensorische Auswertung oder Ansteuerung vorzunehmen. Für die Konvertierung kann die elektronische Schaltung zumindest eines aus einem Digital-Analog-Wandler zum Konvertieren einer digitalen Version des Ansteuersignals in eine analoge Version des Ansteuersignals und/oder einem Analog-Digital-Wandler zum Umsetzen einer analogen Version des elektrischen Ausgangssignals in eine digitale Version des elektronischen Ausgangssignals umfassen. Vereinfacht ausgedrückt kann die elektronische Schaltung 17 das Ansteuersignal In in einer analogen oder digitalen Form erhalten und in ein für den MEMS-Wandler 10 geeignetes analoges oder digitales Signal Out umsetzen. Erfindungsgemäß ist die elektronische Schaltung ausgebildet, um das elektrische Ansteuersignal In in eine Auslenkung des verformbaren Elements zumindest eines elektromechanischen Wandlers zu konvertieren. Hierfür umfasst die elektronische Schaltung einen schaltenden Verstärker (so genannter Klasse-D Verstärker). Dieser ist ausgebildet, um das Signal Out als digitales pulsweitenmoduliertes Ansteuersignal für das verformbare Element bereitzustellen.

Es sei hier angemerkt, dass die Verwendung des Begriffs Deckelfläche auf den beschriebenen Schichtstapel bezogen ist, die bspw. äußere Schichten des Stapels beschreiben. Dies soll jedoch nicht so verstanden werden, dass keine weiteren Schichten angeordnet werden können, denn es ist möglich an dem beschriebenen Stapel zusätzliche Schichten vorzusehen, die die äußeren Schichten teilweise oder vollständig bedecken. Bspw. können isolierende Schichten, etwa ein Halbleiteroxid oder Lacke vorgesehen sein, aber auch weitere elektrisch funktionelle Schichten.

Das Substrat 14 kann eine oder mehrere Öffnungen 26a-d umfassen, durch die der Volumenstrom 12 von einer Umgebung des MEMS-Wandlers 10 in die Kavität 16 und/oder aus der Kavität 16 in eine Umgebung des MEMS-Wandlers 10 gelangen kann. Eine Bewegung, die das verformbare Element 22 bei der Verformung ausführt, kann bezogen auf das Substrat 14 als in der Ebene (in plane) verstanden werden. Der Volumenstrom 12 kann zumindest teilweise senkrecht zu der Bewegungsrichtung 24 aus der Kavität 16 austreten oder in diese gelangen, wie es beispielsweise für den Volumenstrom 12 durch die Öffnung 26c oder 26d dargestellt ist. Vereinfacht ausgedrückt, kann eine Bewegung des verformbaren Elements 22 in-plane zu einem Volumenstrom 12 out-of-plane führen und andersherum. Das bedeutet, dass die laterale Bewegungsrichtung und/oder die Verkrümmung des verformbaren Elements in-plane bezogen auf das Substrat erfolgen kann.

Die Öffnungen 26c und 26d sind senkrecht zu der lateralen Bewegungsrichtung 24 in dem Substrat 14 angeordnet. Die Verformung des verformbaren Elements 22 entlang der lateralen Bewegungsrichtung 24 kann zu einer Bewegung zumindest eines Bereichs des verformbaren Elements 22 hin zu der Öffnung 26a erfolgen, so dass eine Teilkavität 28 basierend auf der Verformung verkleinert wird. Ein Druck des Fluids, das sich in der Teilkavität 28 befindet, kann basierend darauf erhöht werden. Vereinfacht ausgedrückt, kann das Fluid komprimiert werden. Dies kann ein Ausströmen des Fluids aus der Teilkavität 28 bzw. der Kavität 16 ermöglichen. Durch die Öffnungen 26d und 26c kann der Volumenstrom 12 senkrecht zu der lateralen Bewegungsrichtung 24 erhalten werden.

Eine Grundfläche des MEMS-Wandlers 10 kann beispielsweise in einer x/y-Ebene angeordnet sein. Eine große Abmessung des MEMS-Wandlers 10 entlang einer z-Richtung, die senkrecht zu der x-Richtung und/oder der y-Richtung im Raum angeordnet ist bzw. eine hohe Abmessung des verformbaren Elements 22 entlang der z-Richtung kann zu einer Erhöhung des Volumenstroms 12 führen, während die Grundfläche des MEMS-Wandlers 10 unverändert bleibt. Eine Vergrößerung der Teilkavität 28 kann zu einem Unterdruck des Fluids in der Teilkavität 28 führen, so dass der Volumenstrom basierend auf der Verformung des verformbaren Elements 22 senkrecht zu der lateralen Bewegungsrichtung 24 in die Kavität 28 bzw. 16 strömt.

Das verformbare Element kann eine axiale Ausdehnung, beispielsweise entlang der y-Richtung, aufweisen, die einen Wert in einem Bereich von zumindest 1 $\mu$m und höchstens 100 mm, bevorzugt von zumindest 100 $\mu$m und höchstens 10 mm und besonders bevorzugt in einem Bereich von zumindest 500 $\mu$m und höchstens 5 mm aufweist. Das verformbare Element 22 kann eine Ausdehnung entlang der lateralen Bewegungsrichtung 24 aufweisen, die einen Wert in einem Bereich von zumindest 0,1 $\mu$m und höchstens 1000 $\mu$m, bevorzugt von zumindest 1 $\mu$m und höchstens 100 $\mu$m und besonders bevorzugt in einem Bereich von zumindest 5 $\mu$m und höchstens 30 $\mu$m aufweist. Das verformbare Element kann eine Ausdehnung entlang einer lateralen Richtung, die senkrecht zu der lateralen Bewegungsrichtung angeordnet ist, beispielsweise entlang der z-Richtung, aufweisen, die einen Wert in einem Bereich von zumindest 0,1 $\mu$m und höchstens 1000 $\mu$m, bevorzugt von zumindest 1 $\mu$m und höchstens 300 $\mu$m und besonders bevorzugt in einem Bereich von zumindest 10 $\mu$m und höchstens 100 $\mu$m aufweist.

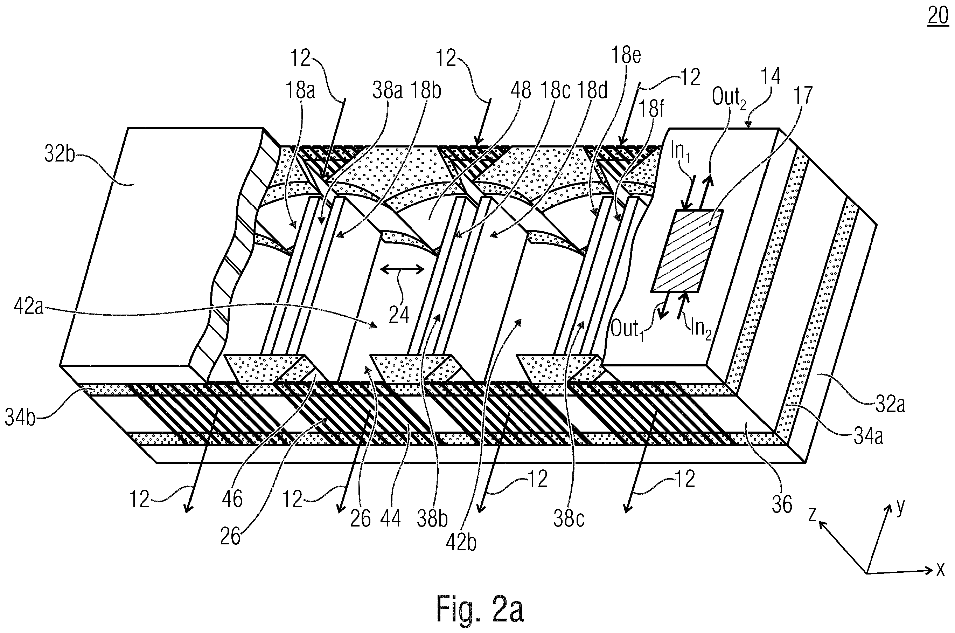

Fig. 2a zeigt eine schematische perspektivische Ansicht eines MEMS-Wandlers 20, der eine Vielzahl elektromechanischer Wandler 18a-f umfasst. Die elektromechanischen Wandler 18a-f sind mit dem Substrat 14 verbunden und können jeweils ein entlang der lateralen Bewegungsrichtung 24 verformbares Element aufweisen, wie es im Zusammenhang mit der Fig. 1 beschrieben ist.

Das Substrat 14 umfasst beispielsweise eine erste Schicht 32a, eine erste Abstandsschicht 34a, eine Zwischenschicht 36, eine zweite Abstandsschicht 34b und eine zweite Schicht 32b, die in der genannten Reihenfolge aufeinander angeordnet sind. Gemäß weiteren Ausführungsbeispielen kann zwischen zwei der als aufeinanderfolgend angeordneten Schichten eine oder mehrere weitere Schichten angeordnet sein. Gemäß weiteren Ausführungsbeispielen ist zumindest eine der Schichten 32a, 32b, 34a, 34b und/oder 36 mehrschichtig aufgebaut. Eine elektronische Schaltung 17, etwa die im Zusammenhang mit der Fig. 1 beschriebene elektronische Schaltung kann teilweise oder vollständig in der Schicht 32b angeordnet sein. Alternativ oder zusätzlich kann die elektronische Schaltung 17 zumindest teilweise in einer oder mehrerer der Schichten 32a, 34a, 36 und/oder 34b angeordnet sein.

Die elektronische Schaltung ist ausgebildet, ein elektrisches Ansteuersignal $In_1$ in eine Auslenkung des verformbaren Elements zu konvertieren, indem dem elektromechanischen Wandler ein Signal Out, basierend auf dem Signal $In_1$ bereitgestellt wird. Zusätzlich kann basierend auf einer Verformung des verformbaren Elements ein Signal $In_2$ erhalten werden, das von der elektronischen Schaltung in ein elektrisches Ausgangssignal $Out_2$ konvertiert werden kann. Die Konvertierung kann hierbei so erfolgen, dass die elektronische Schaltung 17 zumindest eines aus einem Digital-Analog-Wandler (ADC) und einem Digital-Analog-Wandler (DAC) oder auch einem AC/AC-Umsetzer oder DC/DC-Umsetzer aufweist. Basierend hierauf kann die elektronische Schaltung 17 ausgebildet sein, um ein Eingangssignal $In_1$ in einer analogen Version zu erhalten und in eine digitale Version zu überführen, um das Signal Out, zu erhalten. Alternativ können beide Signale $In_1$ und Out, digital sein. Zusätzlich kann die elektronische Schaltung 17 eine Konvertierung des Signals $In_2$, das von dem elektromechanischen Wandler erhalten wird, etwa in einer analogen Form, in das Signal $Out_2$ bereitstellen. Das Signal $Out_2$ kann analog oder digital sein. Die elektronische Schaltung 17 kann somit einen Analog-Digital-Wandler zum Umsetzen einer analogen Version des elektrischen Ausgangssignals $In_1$ in eine digitale Version des elektronischen Ausgangssignals $(Out_1)$ umfassen.

Die elektromechanischen Wandler 18a-f sind ausgebildet und/oder durch die elektronische Schaltung 17 ansteuerbar, so dass sich diese basierend auf dem Volumenstrom 12 und/oder basierend auf einer Ansteuerung teilweise aufeinander zu- und teilweise voneinander wegbewegen.

Beispielsweise sind die elektromechanischen Wandler 18a und 18b ausgebildet, um sich voneinander wegzubewegen, während die elektromechanischen Wandler 18b und 18c sich aufeinander zubewegen. Zwischen den elektromechanischen Wandlern 18a und 18b, 18c und 18d und 18e und 18f sind Teilkavitäten 38a-c angeordnet, wobei sich die Teilkavitäten 38a-c basierend auf der Verformung der elektromechanischen Wandler 18a-f vergrößern können. Zwischen den elektromechanischen Wandlern 18b und 18c bzw. 18d und 18e sind Teilkavitäten 42a und 42b angeordnet, die sich basierend auf der Bewegung oder Verformung gleichzeitig verkleinern können. In einem darauffolgenden Zeitintervall kann die Verformung oder Bewegung der elektromechanischen Wandler bzw. der verformbaren Elemente umkehrbar sein, so dass sich die Volumina der Teilkavitäten 38a, 38b und 38c verkleinern, während sich Volumina der Teilkavitäten 42a und 42b vergrö-ßern.

Die elektronische Schaltung 17 kann entlang einer Richtung senkrecht zu der Bewegungsebene angeordnet sein, in welcher sich die elektromechanischen Wandler bewegen. Wird ein Ort der elektronischen Schaltung in die Bewegungsebene projiziert, so kann dieser einem Ort entsprechen, an dem sich das verformbare Element während der Verformung zumindest zeitweise befindet. Das bedeutet, dass das verformbare Element sich bspw. über oder unter der elektronischen Schaltung 17 befinden kann.

In anderen Worten, auf dem unteren Deckel (erste Schicht 32a), der den Chip auf einer Seite (beispielsweise jedoch ohne Einschränkung eine Unterseite) teilweise oder vollständig abschließt, kann eine strukturierte Schicht, die Abstandsschicht 34a, angeordnet sein, welche beispielsweise als Abstandshalter zwischen dem unteren Deckel und der auf der strukturierten Schicht 34a angeordneten Zwischenschicht 36 nutzbar ist. Auf die strukturierte Schicht 36 kann wiederum eine strukturierte Abstandsschicht 34b angeordnet sein, die in ihrer Funktion als Abstandshalter ganz oder teilweise der Abstandsschicht 34a entspricht und eine identische oder ähnliche Form aufweisen kann. Der MEMS-Wandler 20 bzw. dessen Kavität kann durch den oberen Deckel, die zweite Schicht 32b entlang der z-Richtung teilweise oder vollständig abgeschlossen werden. Fig. 2a zeigt die Schicht 32b als teilweise aufgebrochene Darstellung, um im Bereich der Kavität angeordnete Elemente darstellbar zu machen. In einer x/y-Ebene der Zwischenschicht 36 können paarweise elektromechanische Wandler 18b und 18c bzw. 18d und 18e angeordnet sein, wobei sich eine derartige Anordnung entlang einer Raumrichtung, beispielsweise entlang der x-Richtung mehrfach wiederholen kann. Die elektronische Schaltung 17 kann ganz oder teilweise in zumindest einem der Deckel 32a und/oder 32b angeordnet sein. Es ist ferner möglich, dass sich die elektronische Schaltung 17 auch über mehrere Schichten erstreckt und bspw. teilweise in der Schicht 32a und der benachbarten Schicht 34a oder in der Schicht 32b und der benachbarten Schicht 34b angeordnet ist.

Eine Anordnung der elektronischen Schaltung ganz oder teilweise in zumindest einem der Deckel 32a und/oder 32b ermöglicht eine platzsparende und somit flächeneffiziente Anordnung der elektronischen Schaltung. Dies ist insbesondere vorteilhaft in Kombination mit einem lateral, d. h., in-plane beweglichen Element möglich. Im Gegensatz zu senkrecht hierzu und out-of-plane beweglichen Elementen, wie es bspw. bei Lautsprechermembranen der Fall ist, kann auf ein Ausdünnen der entsprechenden Schicht (etwa zur Membranausbildung) verzichtet werden und/oder eine Bedeckung des beweglichen Elements mit dem Deckel angeordnet werden, ohne die Funktionalität zu beeinträchtigen. Bei out-of-plane Bewegungen wäre die ausgedünnte Membranschicht, die das bewegliche Element zumindest teilweise bildet, entweder wenig geeignet für eine Anordnung einer elektronischen Schaltung und/oder eine zusätzliche bedeckende Schicht würde die Performance der Vorrichtung beeinträchtigen. In Ausführungsbeispielen ist der Deckel des Stapels, der auch von weiteren Schichten bedeckt sein kann, kein Teil des beweglichen Elements.

Das Substrat kann eine Vielzahl von Öffnungen 26 aufweisen, die mit einer Vielzahl von Teilkavitäten 38a-c bzw. 42a-b verbunden sind, wobei bspw. jeweils eine Öffnung 26 mit einer Teilkavität 38a-c oder 42a-b verbunden sein kann. Ein Volumen jeder Teilkavität 38a-c oder 42a-b kann von einem Auslenkungszustand zumindest eines entlang der lateralen Bewegungsrichtung 24 verformbaren Elements 22 beeinflusst sein. Benachbarte Teilvolumina können komplementär während eines ersten oder zweiten Zeitintervalls vergrößerbar bzw. verkleinerbar sein. Vereinfacht ausgedrückt, kann ein Teilvolumen einer Teilkavität 38a-c oder 42a-b verkleinert werden, während ein benachbartes Teilvolumen einer Teilkavität 42a-b bzw. 38a-c vergrößert wird.

In einem Bereich einer oder mehrerer Öffnungen 26 können Stabstrukturen 44 angeordnet sein. Die Stabstrukturen 44 können so angeordnet sein, dass ein Passieren des Volumenstroms 12 in eine oder zwei Richtungen ermöglicht ist, wohingegen ein Eindringen oder Heraustreten von Partikeln in die Kavität oder aus der Kavität reduziert oder verhindert ist. Eine Form der Schichten 32a, 32b, 34a, 34b und/oder 36 kann beispielsweise während eines Herstellungsprozesses durch selektives Entfernen und/oder selektives Anordnen oder Aufwachsen von Schichten beeinflusst sein. Beispielsweise können die Stabstrukturen 44 basierend auf einem selektiven Ätzprozess aus den Schichten 34a, 36 und/oder 34b herausgebildet werden. Ferner kann während des Herstellungsprozesses eine Form der Kavitäten 38a-c und 42a-b beeinflusst werden. Beispielsweise können Wandungen einer oder mehrerer Schichten 32a, 32b, 34a, 34b und/oder 36 an eine Bewegung der verformbaren Elemente der elektromechanischen Wandler 18a-f angepasst sein, beispielsweise um einen zumindest näherungsweise konstanten und/oder geringen Abstand zwischen den verformbaren Elementen und dem Substrat 14 zu ermöglichen.

Benachbart oder an den Stabstrukturen oder Stabelementen kann eine Abdeckung 43 angeordnet sein. Die Abdeckung 43 kann benachbart zur Kavität 16 und/oder mittels der Stabelementen 44 hiervon getrennt angeordnet sein. Die Abdeckung kann bspw. ein Fließmaterial (Mesh-Material), ein Schaumstoffmaterial und/oder ein Papiermaterial umfassen. Die Abdeckung kann ein Eindringen von Partikeln in die Kavität 16 oder ein Austreten aus der Kavität 16 mit geringerem Durchmesser als einem Abstand zwischen Stabstrukturen ermöglichen. Alternativ kann die Abdeckung 43 auch benachbart oder an einer Öffnung 26 angeordnet sein, die die Stabelemente 44 nicht aufweist.

Bewegt sich ein freies Ende der bewegbaren Elemente, beispielsweise in einer gebogenen Bahn und/oder einer Kreisbahn, so kann das Substrat 14 in einem Bereich, in welchem sich das bewegliche Ende bewegt, eine parallele oder ähnliche Form aufweisen.

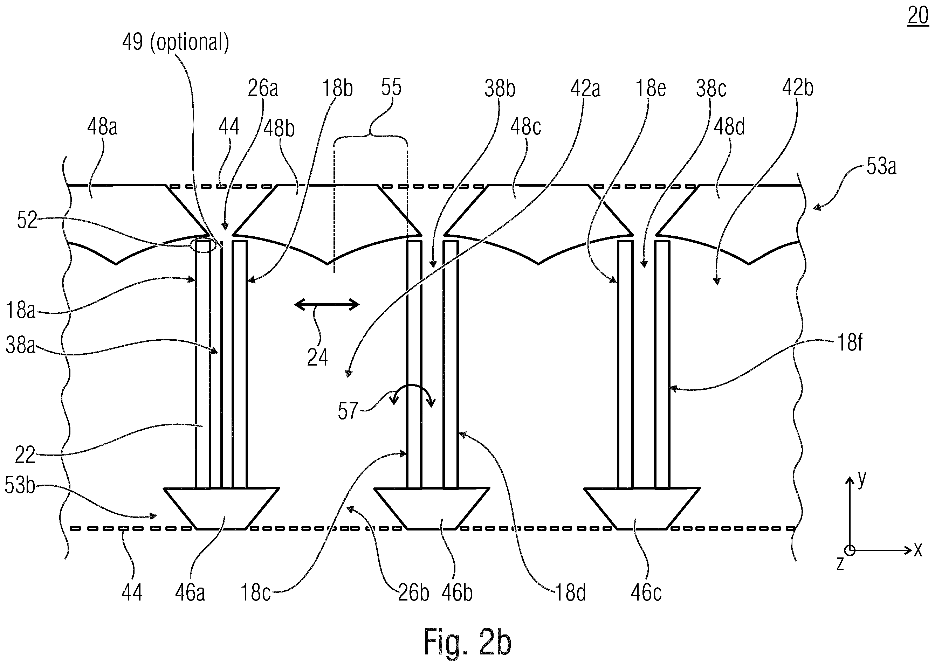

Fig. 2b zeigt eine schematische Aufsicht des MEMS-Wandlers 20 aus Fig. 2a. Die elektromechanischen Wandler 18a-f können beispielsweise mit dem Substrat 14 an Elementen 46a-c kraft- oder formschlüssig verbunden sein. Beispielsweise können ein oder mehr verformbare Elemente der elektromechanischen Wandler 18a-f einstückig mit den Elementen 46a-c gebildet sein. Die Elemente 46a-c können in einer Ebene der Schicht 36 angeordnet sein oder Teile der Schicht 36 sein. Eine Ausdehnung der verformbaren Elemente 22 der elektromechanischen Wandler 18a-f kann beispielsweise kleiner oder gleich sein als eine Ausdehnung der Schichten 34a, 36 und 34b entlang der z-Richtung. Das bedeutet, dass die verformbaren Elemente 22 der elektromechanischen Wandler 18a-f kontaktfrei zu der Schicht 32a und/oder 32b angeordnet und bewegbar sein können. Alternativ kann zumindest ein verformbares Element auch kontaktbehaftet verformt werden. Bspw. kann zwischen dem zumindest einen verformbaren Element und einer benachbarten Schicht, etwa die Schicht 32a und/oder 32b eine reibungsarme, d. h., einen geringen Reibungskoeffizienten aufweisende, Schicht angeordnet sein. Die reibungsarme Schicht kann eine fluidische Trennung zwischen Teilkavitäten ermöglichen, wie es bspw. für die Wandstruktur 49 beschrieben ist. Ein Reibungskoeffizient kann bspw. um 10 %, 20 % oder 50 % geringer sein, als ein Reibungskoeffizient der Schicht 32a und/oder 32b oder der Schicht 34a und/oder 34b. Eine Reibungskraft zwischen dem verformbaren Element 22 und angrenzenden Schichten kann geringer sein, als eine Kraft, die für eine Verformung des verformbaren Elementes 22 benötigt wird. Basierend auf einer reduzierten Reibungskraft kann bspw. eine von einem Aktor bereitzustellende Kraft geringer sein, so dass der Aktor leistungsärmer ausgeführt werden kann. Alternativ oder zusätzlich kann eine Empfindlichkeit des verformbaren Elementes 22 auf den Volumenstrom 12 erhöht werden.

Die elektromechanischen Wandler 18b und 18c bilden beispielsweise Seitenwände der Teilkavität 42a (Kammer). Die bewegbaren Elemente 22 der elektromechanischen Wandler 18a-f können an den Elementen 46a-c formschlüssig befestigt sein. Zwischen einem auslenkbaren oder beweglichen Ende 52 der verformbaren Elemente 22 kann ein Abstand zu dem Substrat 14 bzw. zu Elementen 48a-d des Substrats 14 angeordnet sein. Das Ende 52 des verformbaren Elements 52 kann somit frei beweglich angeordnet sein. Ein oder mehrere verformbare Elemente 22 können aufgrund von Dimensionsverhältnissen, etwa einer Ausdehnung entlang der x-Richtung in einem Verhältnis zu einer Ausdehnung entlang der y-Richtung, vereinfacht ein Verhältnis Balkenbreite zu Balkenhöhe, besonders weit entlang der lateralen Richtung 24 auslenkbar sein. Sind die elektromechanischen Wandler 18a-f beispielsweise als Aktoren ausgebildet, können diese Aktoren bei Anlegen eines entsprechenden Signals auslenkbar sein, d. h. verkrümmt werden, so dass sich beispielsweise das Ende 52 des verformbaren Elements 22 auf einer gebogenen Bahn bewegt. Entsprechend dem Verlauf dieser Bahn kann zumindest eines der Elemente 48a-d so ausgebildet sein, dass ein Abstand zwischen und dem Ende 52 auch bei Auslenkung des verformbaren Elements 22 in etwa konstant und/oder klein bleibt.

Der MEMS-Wandler 20 kann zumindest eine Wandstruktur 49 aufweisen. Eine Bewegung der Aktoren, elektromechanischen Wandler 18a-e oder verformbaren Elemente kann bspw. für eine Kammer 42a-b zur Folge haben kann, dass aufgrund von durch die Bewegung ausgelösten Fluidströmungen zur Befüllung der Kammer 38a-c eine fluidmechanische Kopplung zu den benachbarten Kammern auftreten kann. Basierend auf der fluidmechanischen Kopplung kann ein Fluidstrom 57 zwischen den Teilkavitäten 42a und 38b auftreten. Um diese direkte Kopplung bzw. den Fluidstrom 57 zu reduzieren oder zu vermeiden, können ein oder mehrere Trennwände (Wandstrukturen 49), die ggf. unbeweglich ausgestaltet sein können, zur Trennung benachbarter Kammerpaare 38 und 42 angeordnet sein. Die Wandstrukturen können einfach realisiert werden, beispielsweise an den entsprechenden Stellen als ein Element, das durchgängig aus den Schichten 34a, 36 und 34b gebildet ist. Beispielsweise können während eines selektiven Ätzverfahrens derartige Strukturen angeordnet bleiben. Die Wandstruktur 49 kann außerdem die mechanische Stabilität des MEMS-Wandlers 20 erhöhen und kann einen Bondvorgang zwischen den einzelnen Schichten erleichtern. Die zumindest eine Wandstruktur 49 kann Öffnungen aufweisen oder vollständig durchgängig gestaltet sein, was es ermöglicht, durch das Herein-/Herausströmen des Fluids aus den Kammern 38a-c und 42a-b entstehende Dämpfung gezielt zu modifizieren, insbesondere zur Einstellung der Breite der Resonanzkurve bzw. allgemein zur Einstellung von dynamischen Eigenschaften der Aktor-Kammersysteme.

Wird die Fig. 2b zusammen mit der Fig. 1 betrachtet, so kann ein Volumen der Kavität 16 und/oder der Vielzahl von Teilkavitäten 38a-c und 42a-b durch die Schichten 32a und 32b und Seitenbereichen 53a und 53b des Substrats 14 beeinflusst oder bestimmt sein. Die Seitenbereiche 53a und 53b können zwischen den Schichten 32a und 32b angeordnet sein. Die verformbaren Elemente der elektromechanischen Wandler 18a-c können ausgebildet sein, um zumindest in einem Abschnitt 55 der lateralen Bewegungsrichtung 24 eine Bewegung parallel zu der ersten Schicht 32a und/oder 32b auszuführen. Das bedeutet, dass sich das verformbare Element zwischen den Schichten 32a und 32b verformen oder bewegen kann.

Eine Resonanzfrequenz einer Kavität oder Teilkavität kann von einer Geometrie des Volumens, von einer Frequenz einer Ansteuerung der elektromechanischen Wandler und/oder von einer mechanischen Resonanzfrequenz des oder der verformbaren Elemente beeinflusst sein. Von einander zumindest teilweise fluidisch getrennte (Teil-)Kavitäten, etwa mittels einer Wandstruktur 49, einer Anordnung einer reibungsarmen Schicht, oder basierend auf einer Anordnung in verschiedenen MEMS-Wandlern können verschiedene Resonanzfrequenzen aufweisen und/oder mit verschiedenen Frequenzen angesteuert werden, etwa mittels einer Steuervorrichtung. Basierend auf verschiedenen Ansteuerfrequenzen und/oder verschiedenen Resonanzfrequenzen kann ein Mehr-Wege-Lautsprecher erhalten werden. Resonanzfrequenzen von Kavitäten werden bspw. im Bereich der Hohlraumresonatoren oder Helmholtz-Resonatoren genutzt.

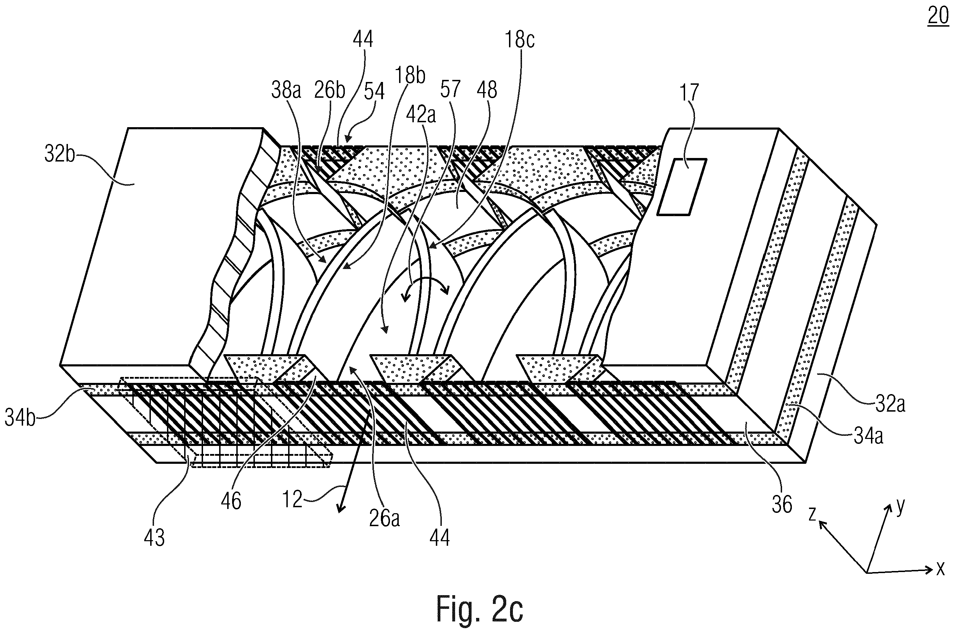

Fig. 2c zeigt eine schematische perspektivische Ansicht des MEMS-Wandlers 20, bei dem die elektromechanischen Wandler 18a-f einen verformten Zustand des verformbaren Elements aufweisen. Beispielsweise sind die verformbaren Elemente bis zu einer maximalen Auslenkung ausgelenkt. Verglichen mit der Darstellung der Fig. 2a ist ein Volumen der Teilkavität 42a basierend auf der Verformung (Verbiegung) der verformbaren Elemente (Balken) verringert. Ist beispielsweise eine Dicke (Abmessung entlang der z-Richtung oder Dickenrichtung) der Schichten 34a und 34b (Abstandshalter) gering, kann bei einer Bewegung der elektromechanischen Wandler 18a-f eine Umströmung der elektromechanischen Wandler 18a-f bzw. der verformbaren Elemente vernachlässigbar sein. Dies kann ebenfalls für einen Abstand zwischen dem elektromechanischen Wandler 18a-f und dem Substrat, beispielsweise dem Element 48, gelten. Basierend auf der Verformung des verformbaren Elements kann ein Volumen des Fluids, beispielsweise ein Luftvolumen, das der Volumendifferenz der Teilkavitäten 42a in den Fig. 2a und 2c entsprechen kann, an eine Umgebung des MEMS-Wandlers 20 abgegeben werden, etwa in Form des Fluidstroms (Volumenstroms) 12.

Eine Abmessung der Abstandsschicht 34a oder 34b entlang der z-Richtung, entlang der die erste und zweite Abstandsschicht 34a und 34b an der Zwischenschicht 36 angeordnet sind, kann einen Wert in einem Bereich von zumindest 1 nm und höchstens 1 mm, bevorzugt in einem Bereich von zumindest 20 nm und höchstens 100 $\mu$m oder besonders bevorzugt in einem Bereich von zumindest 50 nm und höchstens 1 $\mu$m aufweisen. Ist beispielsweise die Abmessung der Abstandsschichten 34a und 34b gering gegenüber einer Abmessung der elektromechanischen Wandler 18a-f entlang der z-Richtung, so kann ein Ausmaß des Fluidstroms 57, der den elektromechanischen Wandler 18a-f von einer ersten Seite zu einer zweiten Seite (beispielsweise von einer positiven x-Richtung zu einer negativen x-Richtung oder andersherum) umströmt, während das verformbare Element sich verformt, kleiner sein als ein Ausmaß des Volumenstroms 12 in der Kavität.

Die Umströmung bzw. der Fluidstrom 57 kann beispielsweise basieren auf einer zumindest teilweisen Entfernung der Abstandsschichten 34a und/oder 34b in einem Bereich, in dem sich der elektromechanische Wandler 18a-f bewegt, resultieren. Vereinfacht ausgedrückt kann basierend auf dem Abstand zwischen dem elektromechanischen Wandler und benachbarten Schichten ein Fluidstrom um bewegliche Elemente herum resultieren (fluidische Verluste). Diese können verglichen mit dem Fluidstrom 12 gering sein. Beispielsweise können sie kleiner sein als das Ausmaß des Volumenstroms dividiert durch den Wert 10, dividiert durch den Wert 15 oder dividiert durch den Wert 20.

Die elektromechanischen Wandler können sich paarweise auf einander zu und von einander wegbewegen. So können sich bspw. die elektromechanischen Wandler 18a und 18b gegenüber dem Zustand in Fig. 2b paarweise von einander weg bewegen und in einem nachfolgenden Zeitintervall paarweise auf einander zu bewegen. Gleichzeitig können sich bspw. die elektromechanischen Wandler 18b und 18c paarweise auf einander zu oder von einander weg bewegen. Eine derartige jeweils paarweise komplementäre Bewegung von elektromechanischen Wandlern, die auch dann möglich ist, wenn die Wandler nicht benachbart zu einander angeordnet sind, kann eine zumindest teilweise aber auch vollständige Kompensation von Inertialkräften ergeben, so dass ein geringes Maß an Vibrationen oder keine Vibrationen in dem MEMS-Wandler erhalten wird bzw. vom MEMS-Wandler an die Umgebung übertragen wird.

In anderen Worten kann es ein besonderes Merkmal des bisher beschriebene Kammeransatzes darstellen, dass sich die Aktoren paarweise stets gegenläufig aufeinander zu bzw. weg bewegen. Es entstehen also (bei entsprechend sorgfältiger Auslegung der beiden jede Kammerwand begrenzenden aktiven Biegeaktoren) keine Vibrationen, die z. B. beim Einsatz als Hörgerät oder In-Ear-Kopfhörer (Im-Ohr-Kopfhörer) störend sein würden.

Der Fluidstrom 12 kann beispielsweise die Öffnung 26a und/oder 26b passieren. Die Öffnungen 26a und 26b können gleich ausgebildet sein oder an eine Geometrie der benachbarten Teilkavität 38a bzw. 42a angepasst sein. Die Öffnung 26a kann bspw. entlang einer axialen Richtung (etwa die y-Richtung) einen veränderlichen Querschnitt aufweisen, etwa eine Abmessung entlang der x-Richtung. Die Abmessung der Öffnung 26b entlang der x-Richtung kann in eine Richtung hin zu einem Inneren des MEMS-Wandlers 20, d. h. hin zu der Kavität oder Teilkavität 42a abnehmen. Alternativ oder zusätzlich kann die Öffnung 26 entlang einer weiteren Richtung, etwa einer z-Richtung (Dickenrichtung) senkrecht zu der axialen Richtung y eine veränderliche Abmessung oder einen veränderlichen Querschnitt aufweisen. Der veränderliche Querschnitt kann von einer Außenseite des MEMS-Wandlers 20 in eine Richtung hin zu der Kavität 16 abnehmen. Ein sich verjüngender Querschnitt oder eine sich verringernde Abmessung der Öffnung 26 von der Außenseite des MEMS-Wandlers 20 in Richtung der Kavität 16 entlang einer oder mehrerer Richtungen x und/oder z kann als trichterförmige Öffnung bezeichnet werden.

Die ggf. trichterförmige Öffnung 26b kann als Vorrichtung zur Impedanzanpassung nutzbar sein. Eine Impedanzanpassung kann beispielsweise bei einer Verwendung des MEMS-Wandlers 20 als Lautsprecher vorteilhaft sein. Eine Ausgestaltung oder Geometrie der Öffnung 26b kann analog zu makroskopischen Lautsprechern mit Abmessungen von mehreren Zentimetern ausgeführt sein. Eine Form der Öffnung 26b kann ermöglichen, dass die eigentliche Schallabstrahlung durch die äußere Fläche des Trichters definiert ist. Die Öffnung 26b kann beispielsweise durchgängig in den strukturierten Schichten 34a, 36 und 34b gebildet sein. Ein Stabgitter 54, das zumindest ein Stabelement 44 umfasst, kann Öffnungen bzw. Zwischenräume zwischen Stabelementen 44 und/oder zwischen Stabelementen 44 und dem benachbarten Substrat aufweisen. Die Zwischenräume können so gebildet sein, dass das Fluid durch sie hindurch strömen kann.

Das Stabgitter 54 kann einen Schutz gegen ein Eindringen von Partikeln in die Kavität des MEMS-Wandlers 20 darstellen. Eine Breite der Öffnungen des Stabgitters 54, d. h. ein Abstand zwischen Stabelementen 44, kann so ausgeführt sein, dass der Fluidstrom 12 strömungstechnisch in einem gewünschten Maß beeinflusst oder unbeeinflusst ist. Beispielsweise oder idealerweise kann der Abstand zwischen den Stabelementen 44 kleiner sein als kleinste Spaltabstände im MEMS-Wandler 20, so dass das Stabgitter eine hohe Anzahl von oder gar alle relevanten Partikel filtern kann. Ein Spaltabstand kann bspw. einen Abstand eines verformbaren Elementes 18a-c zu einer Schicht 32a oder 32b beschreiben. Der Abstand zwischen den Stabelementen 44 kann beispielsweise geringer sein als 5 $\mu$m, als 1 $\mu$m, als 0,1 $\mu$m oder 0,05 $\mu$m.

Abmessungen der Stabelemente 44 entlang der Raumrichtungen können so implementiert sein, dass die Stabelemente 44 keine Resonanzen im Hörschallbereich, d. h. in einem Frequenzbereich von zumindest 16 Hz und höchstens 22 KHz aufweisen. Obwohl die Stabelemente 44 so dargestellt sind, dass sie an einer Außenseite des MEMS-Wandlers 20 angeordnet sind, etwa in einem Bereich, an dem die Öffnung 26a oder 26b eine maximale Abmessung entlang der x-Richtung aufweist, können ein oder mehrere Stabelemente auch an einer anderen Stelle der Öffnung 26a oder 26b angeordnet sein, etwa in einem verjüngten Bereich der Öffnung 26a bzw. 26b.

Durch die Verformung der verformbaren Elemente kann das Volumen einer Teilkavität 42a verringert werden. Während eines gleichen Zeitintervalls kann sich ein Volumen der Kammer (Teilkavität) 38a erhöhen. Die Teilkavität 38a kann in gleicher oder ähnlicher Weise wie die Teilkavität 42a über eine trichterförmige Öffnung 26b und/oder ein Stabgitter 54 umfassend ein oder mehrere Stabelemente 44 mit der Umgebung des MEMS-Wandlers 20 verbunden sein. Elektromechanische Wandler 18a-f können ausgebildet sein, um mit einer von einander verschiedenen Frequenz angesteuert zu werden oder eine von einander verschiedene Resonanzfrequenz aufweisen. Ein Volumen einer jeden Teilkavität kann sich mit einer von einander verschiedenen Frequenz oder mit zumindest teilweise gleichen Frequenzen ändern.

Die Öffnung 26a und die Öffnung 26b können an oder in einander entgegengesetzt im Raum angeordneten Seiten des MEMS-Wandlers 20 angeordnet sein. Bspw. kann auf jeweils einer Seite, die die Öffnung 26a oder 26b aufweist, mittels der Teilkavitäten 42a bzw. 38a oder einer Vielzahl derartiger Teilkavitäten der Fluidstrom ausgestoßen 12 oder angesaugt werden kann. Das bedeutet, dass der Fluidströmung 12 in entgegengesetzte Richtungen erzeugbar ist. Beispielsweise kann in einem ersten Zeitintervall der Volumenstrom 12 in eine negative y-Richtung aus der Öffnung 26a ausgestoßen und in die Teilkavität 38a eingesaugt werden. In einem zweiten Zeitintervall können sich diese Richtungen umkehren. Ein Strömungskurzschluss entlang des MEMS-Wandlers 20 kann so verhindert oder ausgeschlossen werden.

Die verformbaren Elemente (Balken) der elektromechanischen Wandler 18a-f können ausgebildet sein, um sich gemäß einem von außen zugeführten Signal zu verkrümmen. Eine Frequenz, mit der die Verkrümmung erfolgt, kann eine Frequenz, mit der der Volumenstrom 12 generiert wird und/oder oszilliert und mithin eine Schallfrequenz beeinflussen oder bestimmen. Eine über das zugeführte Signal bestimmte Amplitude der Schwingung kann bei einer oder mehreren (Resonanz-Frequenzen) eine Amplitude des Volumenstroms 12 beeinflussen oder bestimmen und mithin Auswirkungen auf den Schallpegel haben.

Ebenfalls kann mindestens eine Kammer (Kavität oder Teilkavität) als sensorisches Element fungieren und eine andere Kammer als aktorisches Element fungieren. Das bedeutet dass der MEMS-Wandler zumindest ein sensorisches und zumindest ein aktorisches verformbares Element umfassen kann. Die Bewegung der Balken wird detektiert und ausgewertet. So können bspw. die elektromechanischen Wandler 18a und 18b als Aktoren angesteuert werden, während die elektromechanischen Wandler 18c und/oder 18d als Sensoren zur Detektion innerhalb des Fluids genutzt werden können. Zur Detektion können elektrostatische (kapazitive), piezoelektrische oder piezoresistive Sensorelemente integriert sein. Ein solches Element kann als Mikrofon bzw. Drucksensor eingesetzt werden. Ein solches integriertes Mikrofon bzw. ein solcher Drucksensor kann auch für die Kontrolle und Steuerung der Eigenschaften der Lautsprecherkammern (Aktoren) bzw. Ultraschallsenderkammer bzw. Pumpenkammer eingesetzt werden. Dazu ist eine entsprechende Elektronik als Steuerkreis/Kontrollkreis einzusetzen.

Nachfolgend werden weitere Ausführungsbeispiele für die elektromechanischen Wandler bzw. Aktoren erläutert. Obwohl der MEMS-Wandler 20 so beschrieben wurde, dass ein unausgelenkter bzw. nicht-aktuierter Zustand unausgelenkte verformbare Elemente aufweist, können die Zustände auch wechselseitig vertauschbar sein. Das bedeutet, dass in einem ersten, unaktuierten Zustand, die verformbaren Elemente verformt oder gekrümmt sein können und sich basierend auf einem Ansteuersignal in einen weniger gekrümmten, stärker gekrümmten oder geraden Zustand verformen können.

Obwohl obige Ausführungen erläutern, dass ein elektrisches Signal an den MEMS-Wandler 20 herangeführt wird, etwa von einer Steuervorrichtung, kann auch der Volumenstrom 12 zu einer Verformung der verformbaren Elemente führen, wobei die Verformung mittels eines elektrischen Signals an dem MEMS-Wandler 20 erhalten werden kann, d. h. der MEMS-Wandler 20 ist auch als Sensor konfigurierbar.

Nachfolgend wird Bezug genommen auf vorteilhafte Weiterbildungen des verformbaren Elements. Einer oder mehrere elektromechanische Wandler können verformbare Elemente gemäß den nachfolgend beschriebenen Weiterbildungen aufweisen.

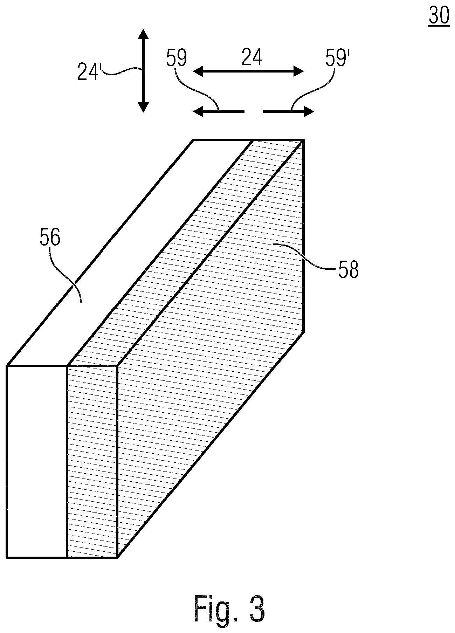

Fig. 3 zeigt eine schematische perspektivische Ansicht eines verformbaren Elements 30, das als Bimorph ausgeführt ist. Das verformbare Element 30 weist eine erste Schicht 56 und eine zweite Schicht 58 auf, die zumindest stellenweise, vorteilhafterweise ganzflächig, fest miteinander verbunden sind. Die erste Schicht 56 und die zweite Schicht 58 sind ausgebildet, um sich basierend auf einem mechanischen, physikalischen oder chemischen Einfluss unterschiedlich stark zu verformen, etwa auszudehnen oder zu kontrahieren. Beispielsweise können die Schichten 56 und 58 voneinander verschiedene thermische Ausdehnungskoeffizienten aufweisen. Alternativ oder zusätzlich kann die Schicht 56 oder die Schicht 58 ausgebildet sein, um sich basierend auf einem elektrischen Signal, das an die entsprechende Schicht geführt wird, auszudehnen oder zu kontrahieren. Beispielsweise kann diese Schicht Piezomaterialien aufweisen.

Voneinander verschiedene Kontraktionen oder Ausdehnungen der Schichten 56 und 58 können zu einer Verformung des verformbaren Elements 30 entlang einer Aktuierungsrichtung 59 oder 59' führen. Die Aktuierungsrichtung kann parallel zu der lateralen Bewegungsrichtung 24 angeordnet sein. Die Aktuierungsrichtung kann eine Richtung sein, entlang der das verformbare Element 30 durch Anlegen einer positiven elektrischen Spannung auslenkbar ist.

Alternativ oder zusätzlich kann auch eine Verformung entlang einer weiteren lateralen Bewegungsrichtung 24' nutzbar sein, die beispielsweise basierend auf einer Querkontraktion oder Querexpansion des verformbaren Elements 30 bzw. der Kontraktion oder Expansion einer der Schichten basiert. Das bedeutet, dass das verformbare Element 30 ausgebildet sein kann, um sich mit seiner Balkenstruktur entlang einer axialen Richtung (etwa die y-Richtung bzw. in-plane) der Balkenstruktur zu verkrümmen. Dies kann basierend auf einer Hin- und Her-Bewegung erfolgen, also entlang der lateralen Bewegungsrichtung 24 und entlang einer entgegengesetzten Richtung.

In anderen Worten kann der Bimorph einem Balken entsprechen, der aus zwei Schichten besteht. Die Schichten sind beispielsweise in einer Richtung (beispielsweise vertikal) zueinander angeordnet. Eine passive Schicht (beispielsweise die Schicht 56) kann mit einer aktiven Schicht (beispielsweise die Schicht 58) fest verbunden sein. Durch Anlegen eines geeigneten Signals kann in der aktiven Schicht 58 eine mechanische Spannung generiert werden, welche zur Kontraktion oder Expansion der Schicht 58 führt. Eine Richtung der Längenänderung der Schicht 58 kann so gewählt werden, dass sich der Bimorph lateral in die eine (Kontraktion) oder andere (Expansion) Richtung verbiegt.

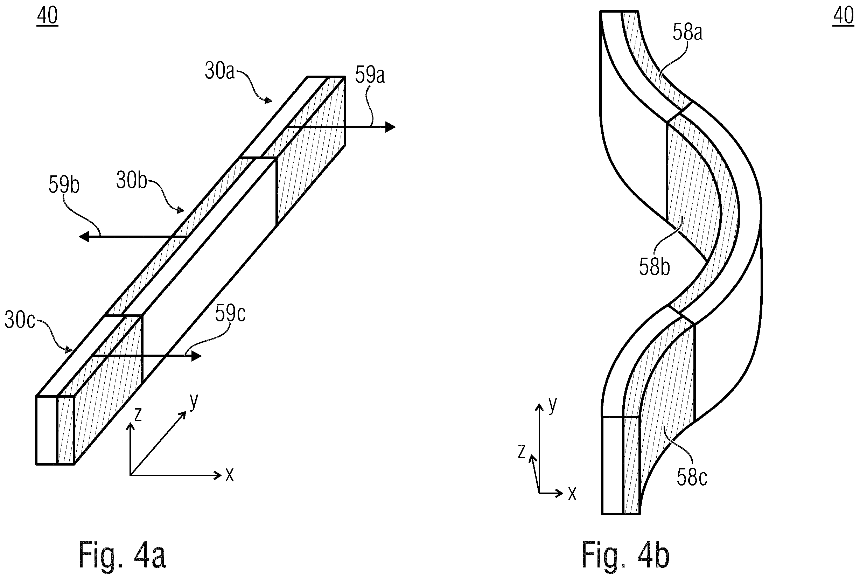

Fig. 4a zeigt eine schematische perspektivische Ansicht eines verformbaren Elements 40, das drei Bimorphstrukturen 30a-c aufweist, wie sie im Zusammenhang mit Fig. 3 beschrieben sind. Eine schematische Anordnung des verformbaren Elements 40 im Raum entlang der x-, y- und z-Richtung ist beispielhaft (jedoch nicht einschränkend) so dargestellt, wie das verformbare Element 40 beispielsweise in dem MEMS-Wandler 10 oder 20 angeordnet werden kann. Die verformbaren (Teil-)Elemente 30a-c können voneinander verschiedene Abmessungen aufweisen, beispielsweise entlang der x,- y- oder z-Richtung. Beispielsweise können die verformbaren Elemente 30a und 30c eine gleiche Ausdehnung entlang der y-Richtung aufweisen. Die Aktuierungsrichtungen 59a-c der verformbaren Elemente 30a-c kann bspw. alternierend oder eine wechselseitige Ausrichtung aufweisend angeordnet sein, bspw. in positiver/negativer/positiver x-Richtung. Vereinfacht kann dies so verstanden werden, dass die verformbaren Elemente 30a und 30c eine gleiche Länge aufweisen. Das verformbare Element 30b kann eine hiervon verschiedene Länge aufweisen. Beispielsweise kann eine Länge des verformbaren Elements 30b doppelt so lang sein wie die vergleichbare Länge des Elements 30a oder 30c. Zwischen den verformbaren Elementen 30a-c können gemäß weiterer Ausführungsbeispiele auch weitere Elemente angeordnet sein, beispielsweise Federelemente.

Eine Richtung, entlang der sich die verformbaren Elemente 30a-c beim Anlegen einer gleichen oder vergleichbaren Größe (etwa ein Vorzeichen einer elektrischen Spannung) auslenken, kann entlang der Länge des verformbaren Elements 40 alternierend sein. Dies ermöglicht einen alternierenden Krümmungsverlauf. Obwohl das verformbare Element 40 so dargestellt ist, dass es drei verformbare Elemente 30a-c umfasst, können zwei verformbare Elemente oder mehr als drei verformbare Elemente 30 angeordnet sein.

Fig. 4b zeigt eine schematische perspektivische Ansicht des verformbaren Elements 40 in einem ausgelenkten Zustand. Die Schichten 58a-c sind beispielsweise kontrahiert, so dass entlang eines axialen Verlaufs (y-Richtung) eine Mehrfachkrümmung resultiert.

In anderen Worten können drei in Fig. 3 dargestellte Balken in Richtung ihrer Ausdehnung aneinandergesetzt angeordnet sein. Dies kann so erfolgen, dass ein erster Balken und ein dritter Balken (30a und 30c) bei entsprechendem Signal eine Krümmung in eine erste Richtung und der zweite Balken (30b) eine Krümmung in die andere Richtung aufweist. So kann ein Aktor erhalten werden, der ausgehend von seiner gestreckten Form, wie sie in Fig. 4a dargestellt ist, ohne anliegendes Signal sich mit einem entsprechenden Signal S-artig verformt, wie es in Fig. 4b dargestellt ist. Die Konfiguration mit Signal und ohne Signal ist wechselseitig vertauschbar. So können die verformbaren Elemente 30 beispielsweise eine Vorauslenkung oder Vorspannung aufweisen, die basierend auf dem anliegenden Signal hin zu einer reduzierten Krümmung oder geraden Erstreckung des verformbaren Elements 30 und/oder 40 führt. Beispielsweise kann angenommen werden, dass die Krümmungen der einzelnen Balken 30a-c bis auf das Vorzeichen identisch sind und eine Länge des ersten und dritten Balkens 30a und 30c jeweils in etwa einem Viertel einer Gesamtlänge des verformbaren Elements entspricht und wobei eine Länge des mittleren Balkens 30b in etwa einer Hälfte der Länge des verformbaren Elements 40 entspricht.

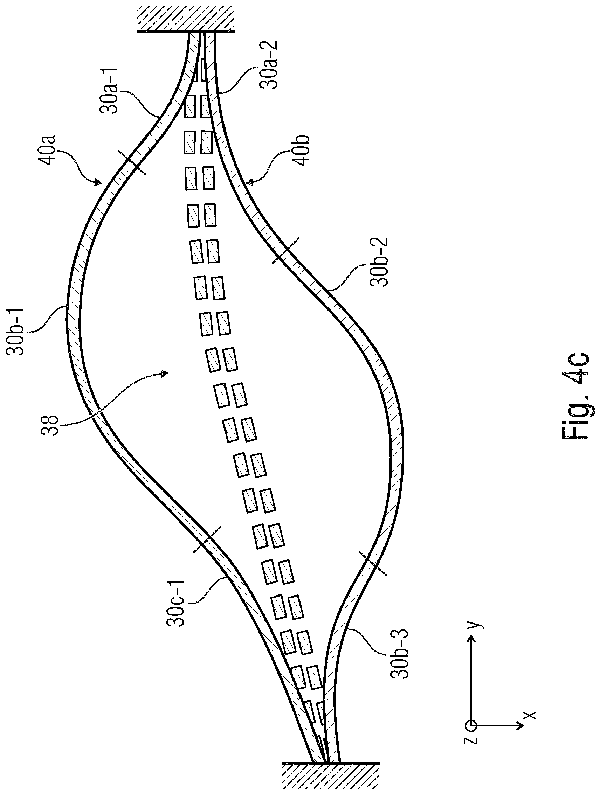

Fig. 4c zeigt eine schematische Aufsicht auf eine Anordnung zweier beidseitig eingespannter verformbarer Elemente 40a und 40b, die benachbart zueinander angeordnet sind, so dass zwischen den verformbaren Elementen die Teilkavität 38 angeordnet ist. Die durchgezogenen Linien zeigen bspw. einen aktuierten Zustand der verformbaren Elemente 40a und 40b während die gestrichelten Linien einen unaktuierten Zustand zeigen, wobei diese Beschreibung der verformbaren Elemente wechselseitig vertauschbar ist, da der unaktuierte Zustand durch die Herstellung beliebige Form annehmen kann.

Die verformbaren Elemente 40a und 40b können so gebildet sein, dass sie in dem unaktuierten Zustand eine Krümmung aufweisen. Ferner können die verformbaren Elemente 40a und 40b aus drei Segmenten 30a-1 bis 30c-1 bzw. 30a-2 bis 30c-2 gebildet sein, die eine wechselseitige Krümmung während der Aktuierung ausführen. Jedes Segment, etwa das mittlere Segment 30b-a oder 30b-2 kann auch aus zwei oder mehr Segmenten gebildet sein. Verglichen mit den Darstellungen der Fig. 4a und 4b können die Segmente 30a-1, 30b-1 und 30c-1 zueinander und mit jedem anderen Segment eine von einander verschiedene Länge aufweisen. Die Länge kann an eine gewünschte Form anpassbar sein, die bei einer Aktuierung erhalten werden soll. Die S-förmigen Aktoren besitzen den sehr großen Vorteil, dass mit Ihnen nicht nur ein großer planarer Füllfaktor erreicht werden kann, sondern sie dabei auch zweiseitig eingespannt werden können. Durch die zweiseitige Einspannung wird eine technologisch nie zu vermeidende Vorauslenkung der Balken aufgrund von Schichtspannungsgradienten sehr deutlich verringert. Damit können die Abstände zum unteren und oberen Deckel des Substrats sehr gering gehalten werden, was die Strömungs-/Druckverluste überproportional verringert und damit die Effizienz von Lautsprechern, Ultraschallwandlern, Mikrophonen und Pumpen nicht nur deutlich erhöht, sondern deren korrekte Funktionsweise unter Umständen erst ermöglicht. Gemäß weiterer Ausführungsbeispielen kann auch lediglich eines der verformbaren Elemente 40 angeordnet sein, etwa in dem MEMS-Wandler 10.

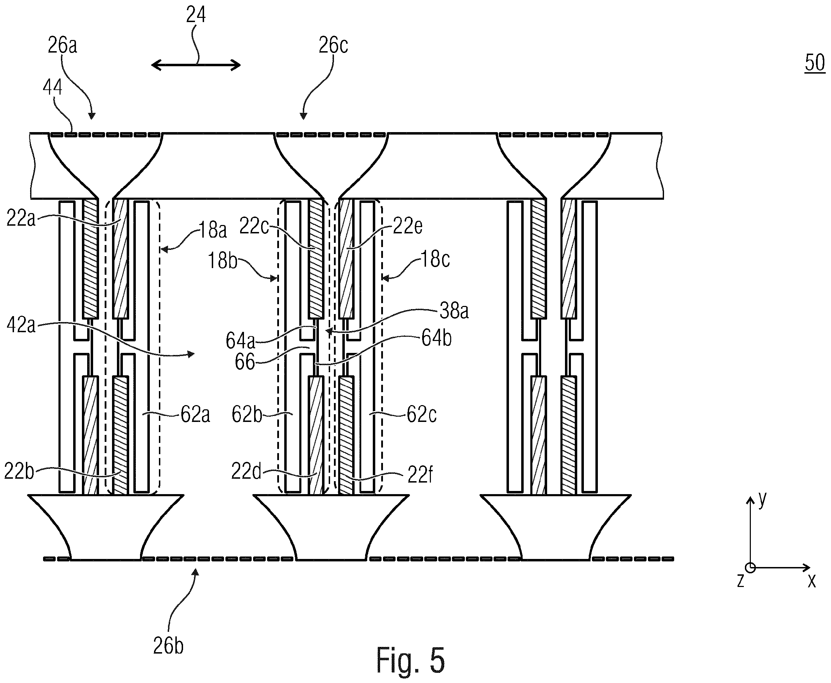

Fig. 5 zeigt eine schematische Aufsicht auf einen MEMS-Wandler 50, bei dem die elektromechanischen Wandler 18a-c verglichen mit dem MEMS-Wandler 20 eine veränderte Konfiguration aufweisen. Die elektromechanischen Wandler 18a-c umfassen jeweils ein erstes und ein zweites verformbares Element 22a und 22b, 22c und 22d bzw. 22e und 22f. Die verformbaren Elemente sind einander gegenüberliegend angeordnet. Auslenkbare Enden der Balkenelemente sind einander zugewandt angeordnet. Bereiche, an denen die verformbaren Elemente 22a-f mit dem Substrat verbunden sind, sind einander abgewandt angeordnet.

Die elektromechanischen Wandler 18a-c umfassen jeweils ein Plattenelement 62a-c, das mit den jeweiligen verformbaren Elementen 22a und 22b, 22c und 22d bzw. 22e und 22f verbunden ist. Das jeweilige Plattenelement 62a-c kann mit den auslenkbaren Enden der jeweiligen verformbaren Elemente 22a-f verbunden sein.

Die verformbaren Elemente 22a-f können ganz oder teilweise als verformbares Element 30 oder 40 ausgeführt sein oder eine andere Konfiguration aufweisen. Unterschiedliche Schraffierungen der verformbaren Elemente 22a und 22b, 22c und 22d bzw. 22e und 22f deuten an, dass die Verformung des jeweiligen verformbaren Elements voneinander verschieden ist. Die verformbaren Elemente eines elektromechanischen Wandlers 18a-c können so angeordnet sein, dass diese unabhängig von einer jeweiligen Ausführung des verformbaren Elements 22a-f eine Auslenkung der auslenkbaren Enden entlang einer gleichen Raumrichtung ausführen.

Beispielsweise kann von dem in der Fig. 5 dargestellten unausgelenkten Zustand eine Ansteuerung dazu führen, dass die auslenkbaren Enden der verformbaren Elemente 22a und 22b entlang einer positiven x-Richtung ausgeführt werden. Ferner kann eine Ansteuerung der verformbaren Elemente 22c und 22d bewirken, dass eine Auslenkung der jeweiligen auslenkbaren Enden entlang einer negativen x-Richtung ausgeführt wird. Dies ermöglicht, dass sich während dieser Ansteuerung die Plattenelemente 62a und 62b aufeinander zubewegen, so dass die Teilkavität 42a basierend auf der Bewegung der Plattenelemente verkleinert wird. Alternativ oder zusätzlich kann ein Unterdruck in der Kavität 42a dazu führen, dass sich die Plattenelemente 62a und 62b aufeinander zubewegen, so dass eine Verformung der verformbaren Elemente 22a-d erhalten wird. Alternativ oder zusätzlich ist ebenfalls vorstellbar, dass ein oder mehrere verformbare Elemente 22a-d elektrisch passiv ausgestaltet sind. Beispielsweise kann an einem oder mehreren Plattenelementen 62a-c ein elektrisches Potential anlegbar sein, so dass basierend auf einem elektrischen Potential der Plattenelemente 62a und 62b eine anziehende oder abstoßende Kraft zwischen den Plattenelementen 62a und 62b erhalten werden kann, die eine Bewegung der Plattenelemente 62a und 62b und mithin eine Verformung der verformbaren Elemente 22a-d bewirkt. Alternativ oder zusätzlich können gleichzeitig oder zeitversetzt die verformbaren Elemente 22c-f und/oder die Plattenelemente 62b und 62c angesteuert werden, um eine Verformung der verformbaren Elemente 22c-f und eine Veränderung des Volumens der Teilkavität 38a zu erhalten.

In anderen Worten zeigt Fig. 5 eine Variante der in den Fig. 2a-c dargestellten Konfiguration, bei der für die Verengung bzw. Erweiterung jeder Kammer (Kavitäten 42a und 38a) vier Biegebalken 22a-d bzw. 22c-f eingesetzt werden. Im Zusammenhang mit den Fig. 2a-c ist dies basierend auf jeweils zwei Biegebalken (verformbaren Elementen) beschrieben. Fig. 5 zeigt dabei einen nicht-aktuierten Zustand. Dabei sind der aktuierte und nichtaktuierte Zustand wechselseitig austauschbar. So kann generell jedes ansteuerbare verformbare Element bei nicht anliegendem Signal verformt sein und seine Verformung signalabhängig verändern, wozu auch das Erreichen eines gestreckten (nicht ausgelenkten) Zustands als Sonderfall gehört.

Vertikal (bspw. entlang der y-Richtung) gegenüberliegende Biegebalken, etwa die verformbaren Elemente 22a und 22b bzw. 22c und 22 d, können jeweils über einen biegbaren Steg umfassend die Elemente 64a und 64b miteinander verbunden sein. In einem Mittenbereich des so erhaltenen Steges kann eine verhältnismäßig steife Verlängerung, das Element 66, angeordnet sein. An diesem kann wiederum das Plattenelement 62b angeordnet sein, das steif oder möglichst steif ausgeführt ist. Bei einem Anlegen eines entsprechenden Signals können sich die Plattenelemente 62a-c parallel aufeinander zu- oder voneinander wegbewegen, um Volumina von Teilkavitäten zu verringern bzw. zu erhöhen. Die parallele Bewegung der Plattenelemente kann ermöglichen, dass das Volumen der Teilkavität 42a im Grenzfall null beträgt, das bedeutet, die Plattenelemente 62a und 62b berühren sich. Verglichen mit einer Konfiguration, wie sie im Zusammenhang mit den Figuren 2a-c beschrieben ist, kann eine derartige Anordnung einen Volumenstrom des Fluids bereitstellen, der deutlich höher ist als der Volumenstrom des MEMS-Wandlers 20. Bei Verringerung des Volumens der Teilkavität 42a kann das Volumen der Teilkavität 38b entsprechend oder zumindest basierend darauf vergrößert werden. Die Zufuhr des Fluids kann, wie es im Zusammenhang mit dem MEMS-Wandler 20 beschrieben ist, durch eine Öffnung 26a, 26b bzw. 26c erfolgen. Die Elemente 64a und 64b können auch als Federelemente bezeichnet werden.

Die verformbaren Elemente (Biegebalken) 22a und 22b können so entworfen sein, dass sie sich bei anliegendem Signal nach rechts (positive x-Richtung) verkrümmen. Die verformbaren Elemente 22c und 22d können so entworfen sein, dass sie sich bei anliegendem Signal nach links (negative x-Richtung) verkrümmen. Beide Balkenarten (Schraffuren der verformbaren Elemente) können so ausgelegt sein, dass sie sich beispielsweise bei einem ersten Signal wie im Zusammenhang mit den Fig. 3 oder 4 verkrümmen und bei einem zweiten Signal in die entgegengesetzte Richtung verkrümmen. In diesem Fall kann sowohl die Verengung als auch die Erweiterung der Kammer (Teilkavität) auf die Ursprungsgröße unabhängig von der mechanischen Rückstellkraft aufgrund der Verbiegung der Balken erreicht werden. Das erste und das zweite Signal können beispielsweise eine positive und eine negative elektrische Spannung sein. Wird beispielsweise die Fig. 3 betrachtet, können auch die Schichten 56 und 58 jeweils aktive Schichten sein bzw. an der Schicht 56 an einer der Schicht 58 abgewandten Seite eine weitere aktive Schicht angeordnet werden, wobei die beiden aktiven Schichten voneinander separat adressiert werden können, um eine Auslenkung in die eine oder die andere Richtung zu erhalten.

Ein Volumen zwischen zwei gegenüberliegenden verformbaren Elementen, etwa die verformbaren Elemente 22c und 22d und dem mit ihnen verbundenen Plattenelement 62b kann sich bei einer Bewegung oder Verformung der Biegebalken ebenfalls ändern. Das Plattenelement 62 kann beispielsweise starr ausgeführt sein, Um einen verbesserten Druckausgleich zu ermöglichen, können die verformbaren Elemente 22c und/oder 22d und/oder verbindende Elemente 64 bzw. 66, die das Plattenelement 62b mit den verformbaren Elementen 22c und 22d verbinden, lokal ausgedünnt oder abgedünnt werden, um einen lokalen Strömungskanal bereitzustellen. Dies kann beispielsweise durch eine zusätzliche Strukturierung oder Ätzung erfolgen. Die verbindenden Elemente 64a, 64b und 66 können in einer T-Anordnung angeordnet sein. Das verbindende Element 66 kann verglichen mit den Elementen 64a und 64b eine hohe Steifigkeit aufweisen. Während einer Verformung der verformbaren Elemente 22c und 22d können sich somit bevorzugt die Elemente 64a und 64b verformen, um eine geradlinige Bewegung des jeweiligen Plattenelements zu ermöglichen.

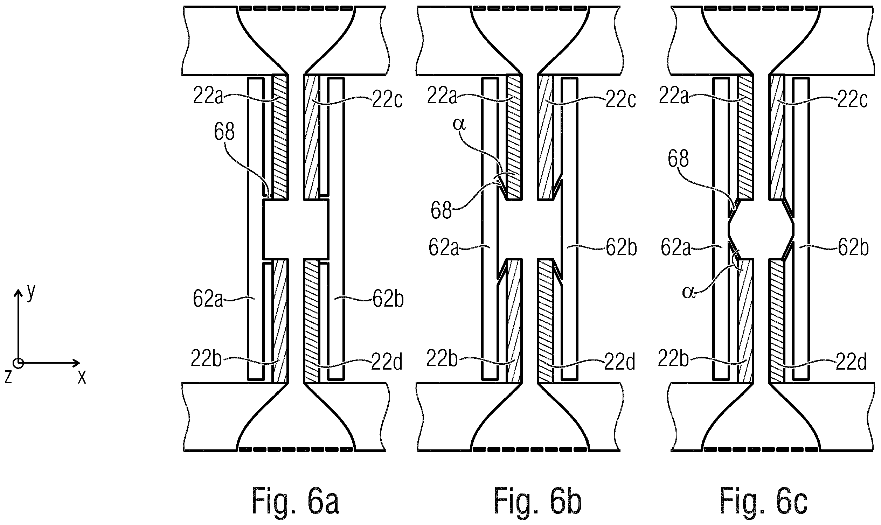

Nachfolgend werden anhand der Fig. 6a-e vorteilhafte Ausführungsbeispiele beschrieben, in denen das Plattenelement 62a bzw. 62b mit jeweils gegenüberliegenden verformbaren Elementen 22a und 22b bzw. 22c und 22d verbunden ist.

Obwohl sich nachfolgende Ausführungen auf eine Verbindung der Plattenelemente mit den verformbaren Elementen beziehen, die jeweils gleich ausgestaltet sind, können voneinander verschiedene elektromechanische Wandler und/oder Verbindungen von einzelnen verformbaren Elementen zu einem Plattenelement voneinander verschieden ausgeführt werden. Die nachfolgend beschriebenen Details beschreiben nicht abschließende vorteilhafte Weiterbildungen und können für sich allein oder in Kombination miteinander oder weiterer vorteilhaften Ausführungsformen implementierbar sein.

Fig. 6a zeigt eine schematische Aufsicht auf eine Konfiguration, bei der gerade ausgebildete Federelemente 68 zwischen dem Plattenelement 62a bzw. 62b und den verformbaren Elementen 22a und 22b bzw. 22c und 22d angeordnet sind. Die Federelemente 68 können aus einem Material der verformbaren Elemente 22a-d oder einem Material der Plattenelemente 62a oder 62b gebildet sein und/oder einstückig mit einem oder mehreren dieser Elemente gebildet sein. Beispielsweise können die Federelemente 68 einen rechten Winkel zu dem Plattenelementen 62a oder 62b aufweisen.

Fig. 6b zeigt eine alternative Konfiguration, bei der Federelemente 68' von auslenkbaren Enden der verformbaren Elemente mit einem Winkel $\alpha$ von weniger als 90°, beispielsweise 30 oder 40°, angeordnet sind. Dies ermöglicht einen verglichen mit der Konfiguration der Fig. 6a erhöhten Abstand der Kontaktpunkte an dem Plattenelement 62a, was zu einer verringerten Durchbiegung des Plattenelements 62a während der Bewegung führen kann.

Fig. 6c zeigt eine Konfiguration, bei der die Federelemente 62a mit einem Winkel $\alpha$ von mehr als 90° angeordnet sind. Dies kann beispielsweise zu verringerten Rückstellkräften der Federelemente 68 führen, wenn die Konfiguration, wie sie in Fig. 6a dargestellt ist, vergleichsweise herangezogen wird.

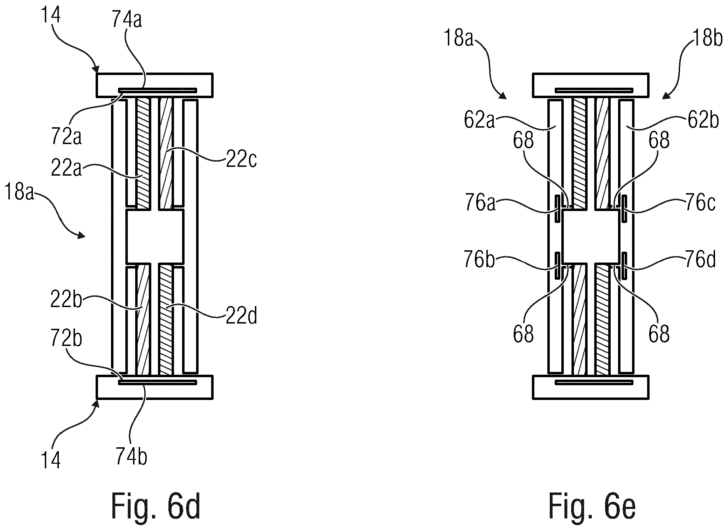

Fig. 6d zeigt eine Konfiguration, bei der die Konfiguration aus Fig. 6a dahin gehend modifiziert ist, dass in Bereichen des Substrats 14 benachbart zu denen der elektromechanische Wandler 18a angeordnet ist bzw. das jeweilige verformbare Element mit dem Substrat 14 verbunden ist, ein Federelement 72a oder 72b angeordnet ist.

Das Federelement 72a und/oder 72b kann beispielsweise durch eine Aussparung (Hohlraum) 74a bzw. 74b in dem Substrat 14 zumindest teilweise bestimmt sein. Das bedeutet, dass beispielsweise durch die Aussparungen 74a oder 74b eine Steifigkeit des Substrats 14 lokal reduzierbar ist, so dass die Federelemente 72a bzw. 72b gebildet werden. Obwohl die Aussparungen 74a und 74b so dargestellt sind, dass sie sich über benachbarte verformbare Elemente 22a und 22c bzw. 22b und 22d in dem Substrat 14 hinweg erstrecken, kann die Aussprung 74a oder 74b auch lediglich benachbart zu einem verformbaren Element oder benachbart zu mehreren verformbaren Elementen angeordnet sein. Alternativ kann das Substrat 14 auch mehrere Aussparungen oder Federelemente aufweisen.

In anderen Worten zeigt Fig. 6d eine Konfiguration, bei der eine weitere Struktur in Form einer Biegefeder (Federelemente 72a und 72b) an der die verformbaren Elemente (Balken) befestigt sind, zu einer weiteren Reduktion der Zugspannung führen kann. Solche Biegefederelemente können beispielsweise auch in die starre Platte integriert werden, wie es in der Konfiguration der Fig. 6e gezeigt ist und im Zusammenhang mit den Aussparungen 76a-d beschrieben ist. Diese Elemente können sich im Fall der Auslenkung der Balken S-förmig verformen und die Zugbelastung auf die starre Platte reduzieren.

Fig. 6e zeigt eine Konfiguration von elektromechanischen Wandlern 18a und 18b, bei der die Plattenelemente 62a und 62b verglichen mit der Konfiguration, wie sie im Zusammenhang mit der Fig. 6d beschrieben ist, Aussparungen 76a-d benachbart zu einem Bereich aufweisen, an dem die Plattenelemente 62a bzw. 62b über die Federelemente 68 mit den verformbaren Elementen verbunden sind. Ein Abstand zwischen den Aussparungen 76a-d und einer den verformbaren Elementen zugewandten Seite der Plattenelemente 62a bzw. 62b kann eine Steifigkeit des Plattenelements 62a bzw. 62b in diesem Bereich beeinflussen. Die Aussparungen 76a-d ermöglichen verringerte Rückstellkräfte, die auf die verformbaren Elemente 22a-d wirken.

In anderen Worten zeigen die Fig. 6a-e Varianten für eine Ausgestaltung der beweglichen Elemente bzw. der elektromechanischen Wandler. Diese unterscheiden sich von einer Ausführung, wie sie im Zusammenhang mit der Fig.

5 beschrieben ist, beispielsweise oder insbesondere dadurch, dass die in Fig. 5 dargestellten Elemente 64a oder 64b mit der Versteifung 66 hin zu den Federelementen 68 verschmolzen wurden. Die Konfiguration gemäß Fig. 6a kann eine höhere Steifigkeit gegenüber parasitären Verkippungen der Plattenelemente 62a bzw. 62b um eine Achse senkrecht zur Zeichenebene (x/y-Ebene) aufweisen. Ähnliches kann für die Konfigurationen gemäß den Fig. 6b und 6c gelten. Alle drei Konfigurationen ermöglichen außerdem größere Auslenkungen der Biegebalken im Vergleich zu der Konfiguration aus Fig. 5. Dort kann das Element 64a bzw. 64b (biegbarer Steg) bei einer Auslenkung der Balken unter Zugspannung stehen, welche mit wachsender Auslenkung in einen zunehmenden mechanischen Widerstand für die Balkenauslenkung der verformbaren Elemente resultieren kann. Bei den Varianten gemäß den Fig. 6a-c kann die mechanische Verbindung der beiden verformbaren Elemente deutlich weicher (weniger steif) ausgeführt sein, da die jeweils verbindenden Federelemente 68 mit einer Verbiegung reagieren können, die bei entsprechender Gestaltung dieser Elemente einen deutlich geringeren mechanischen Widerstand darstellen kann.

Die verbindenden Elemente/Federn 68 und/oder die im Zusammenhang mit der Fig. 5 beschriebenen Elemente/Federn 64a-b können auch eine gekrümmte oder mäanderförmige Form aufweisen. Dies ermöglicht eine erhöhte Flexibilität in einer bevorzugten Richtung. Die Konfigurationen, wie sie im Zusammenhang mit den Fig. 6d und 6e beschrieben sind, ermöglichen eine Verringerung der Zugbelastung, die zu einer effektiven Versteifung des verformbaren Elements führen würde. Die in den Fig. 6a-e beschriebenen Konfigurationen vernachlässigen Ein- bzw. Auslassöffnungen 26. Sind diese Öffnungen angeordnet, können Aussparungen bzw. Federelemente in dem Substrat in Bereichen, in denen die Öffnung angeordnet ist, entfallen. Alternativ oder zusätzlich kann eines, mehrere oder jedes der durch zumindest eine Aussparung erhaltenen Federelemente 72a, 72b und/oder in den Plattenelementen 62a oder 62b basierend auf zwei oder mehreren voneinander getrennten und unabhängigen Federelementen realisiert sein.

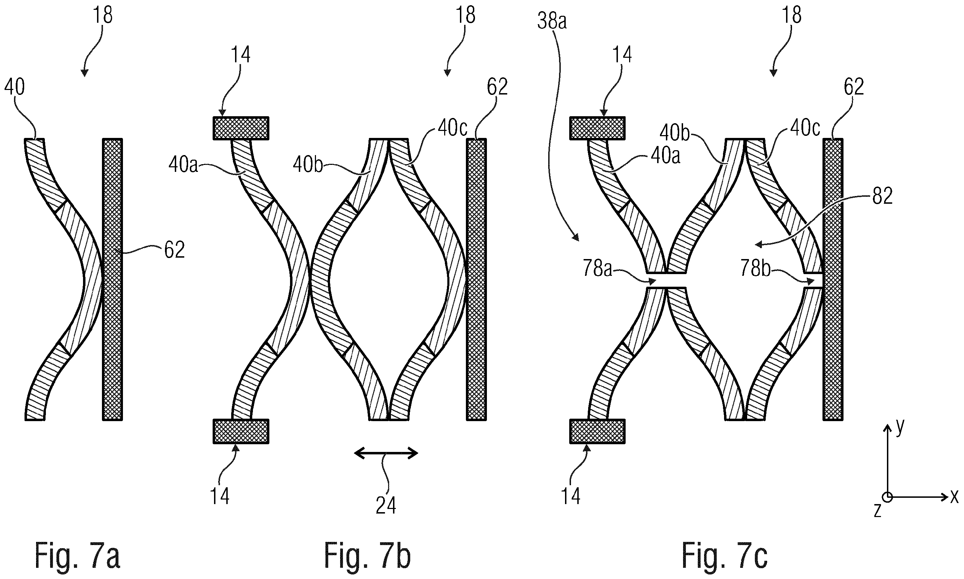

Nachfolgend beschriebene Fig. 7a-c beschreiben beispielhaft mögliche Anordnungen von verformbaren Elementen und Plattenelementen.

Fig. 7a zeigt das verformbare Element 40, das mit dem Plattenelement 62 verbunden ist. Das Plattenelement 62 kann beispielsweise unmittelbar an dem verformbaren Element 40 angeordnet sein.

Fig. 7b zeigt eine Konfiguration, bei der das verformbare Element 40a zwischen dem Substrat 14 fest eingespannt ist und ausgebildet ist, um sich entlang der lateralen Richtung 24 zu verformen. Zwischen dem verformbaren Element 40 und dem Plattenelement 62 sind zwei weitere verformbare Elemente 40b und 40c angeordnet, deren Enden miteinander verbunden sein können. Ausgehend von den Verbindungen können die verformbaren Elemente 40b und 40c so zueinander ausgerichtet sein, dass eine Wölbung des jeweiligen verformbaren Elements 40b oder 40c von dem anderen verformbaren Element weg weist. Die verformbaren Elemente 40a-c können beispielsweise gemeinsam angesteuert werden oder gemeinsam auf den Volumenstrom des Fluids reagieren, wobei beispielsweise eine gemeinsame Ansteuerung der verformbaren Elemente 40a-c zu einer Vergrößerung des Stellwegs, d. h. zu einer Vergrößerung des Weges, um das das Plattenelement 62 ausgelenkt wird, führen. Das bedeutet, dass zwischen dem verformbaren Element und dem Plattenelement zumindest ein weiteres verformbares Element angeordnet sein kann, das ausgebildet ist, um bei einer gemeinsamen Ansteuerung mit dem verformbaren Element einen Stellweg des verformbaren Elements zu erhöhen.

Fig. 7c zeigt eine Konfiguration des elektromechanischen Wandlers 18, bei dem die verformbaren Elemente 40a-c in einem Mittenbereich Aussparungen 70a oder 70b aufweisen, die eine fluidische Kopplung eines Volumens 82 zwischen den verformbaren Elementen 40b und 40c mit einer weiteren Teilkavität, beispielsweise der Teilkavität 38a ermöglichen. Die verformbaren Elemente 40a, 40b und/oder 40c können jeweils zweiteilig ausgeführt sein, um die Aussparungen 78a und 78b bereitzustellen. Alternativ oder zusätzlich können die Aussparungen 78a und 78b als Aussparungen ausgeführt sein, die entlang einer Dickenrichtung (z-Richtung) von weiterem Material der verformbaren Elemente 40a, 40b bzw. 40c umschlossen sind.

In anderen Worten zeigt Fig. 7a eine Konfiguration mit aktuierten S-förmigen Biegebalken nach Fig. 4, bei dem in der Mitte der starren Platte eine Verbindung zu dem Biegebalken angeordnet ist. Zur Erhöhung der Auslenkung können die Biegeaktoren mehrfach hintereinander (seriell) angeordnet werden. Die Fig. 7b und 7c zeigen schematisch eine Anordnung von drei seriell geschalteten S-Aktoren. Gemäß weiteren Ausführungsbeispielen können zwei S-Aktoren (verformbare Elemente 40) oder mehr als drei Aktoren seriell geschaltet werden. Die Schraffuren der verformbaren Elemente in den Fig. 7a-c sind beispielsweise in Übereinstimmung mit den Schraffuren, wie sie in Fig. 4 gewählt wurde, dargestellt. Voneinander verschiedene Schraffuren können eine voneinander verschiedene Krümmungsrichtung der jeweiligen Abschnitte bedeuten. Fig. 7c zeigt eine Konfiguration, die in der Mitte der S-förmigen Aktoren eine Öffnung aufweist (Aussparungen 78a und 78b), die eine verbesserte Belüftung des Zwischenraums (Kavität 82) ermöglicht.

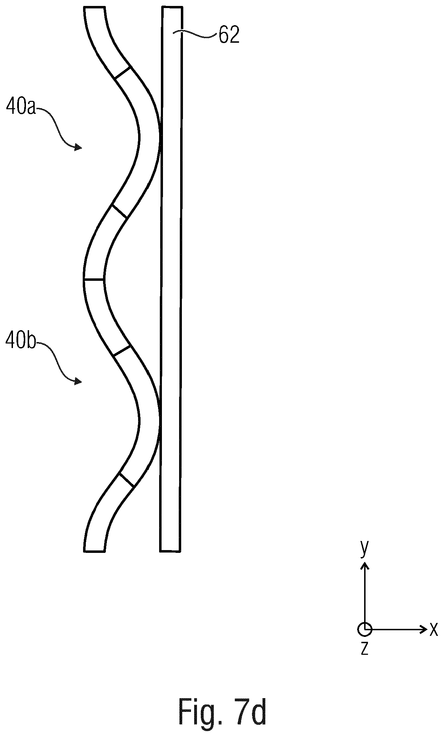

Fig. 7d zeigt eine Konfiguration des elektromechanischen Wandlers, bei der ein erstes verformbares Element 40a und ein zweites verformbares Element 40b entlang der y-Richtung parallel zu einander angeordnet sind. Dies ermöglicht eine Erhöhung der Kraftwirkung, mit der das Plattenelement 62 ausgelenkt wird. Enden der verformbaren Elemente können mit einander verbunden sein oder gemeinsam an dem Substrat angeordnet sein. Alternativ können auch zwei oder mehrere verformbare Elemente 40a und 40b entlang einer anderen Richtung parallel angeordnet sein, etwa entlang der z-Richtung (Dickenrichtung). Alternativ oder zusätzlich kann auch eine Serienschaltung und eine Parallelschaltung von verformbaren Elementen kombiniert werden.

Bewegliche Elemente können bei einer hohen oder zu hohen Auslenkung auf ein anderes bewegliches Element oder ein festes Element treffen. Dies kann zu Sticking führen. Die beweglichen Elemente oder die festen Elemente können bevorzugt mit Abstandselementen (Pollern) versehen werden, welche ermöglichen, die Kontaktfläche signifikant zu verringern und damit Sticking zu reduzieren oder zu vermeiden. Anstelle sogenannter Poller können auch kleine als Federelemente ausgebildete Strukturen angeordnet werden. Neben der Vermeidung von Sticking kann so der Impuls beim Auftreffen zweier Elemente umgekehrt werden, womit Energieverluste reduziert oder vermieden werden können bzw. das dynamische Verhalten der Aktoren verbessert werden kann.

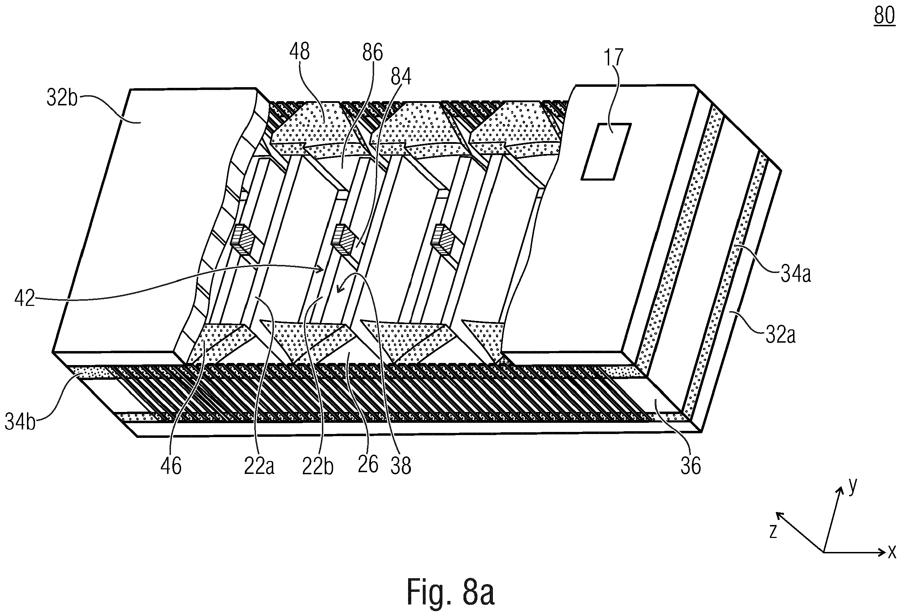

Fig. 8a zeigt eine schematische perspektivische Ansicht eines MEMS-Wandlers 80, bei dem die verformbaren Elemente alternierend mit dem Substrat bzw. der Zwischenschicht 36 bzw. mit einem Ankerelement 84, das mit dem Substrat verbunden ist, verbunden sind. Beispielsweise ist das verformbare Element 22a in den Bereichen 46 und 48 der Zwischenschicht 36 an Enden fest mit dem Substrat verbunden und ausgebildet, um eine S-förmige Bewegung auszuführen, wie es im Zusammenhang mit dem verformbaren Element 40 beispielhaft erläutert ist. Das benachbart angeordnete verformbare Element 22b ist mit dem Ankerelement 84 verbunden. Das Ankerelement 84 ist in einem Mittenbereich des verformbaren Elements 22b angeordnet und kann an der Abstandsschicht 34a oder der Schicht 32a mit derselben verbunden sein. Das bedeutet, dass das Substrat ein Ankerelement aufweisen kann.

Seitenwände der Zwischenschicht 36, die benachbart zu bewegbaren Enden der verformbaren Elemente 22a oder 22b angeordnet sind, können basierend auf einer Bewegungsform der verformbaren Elemente 22a bzw. 22b geformt sein.

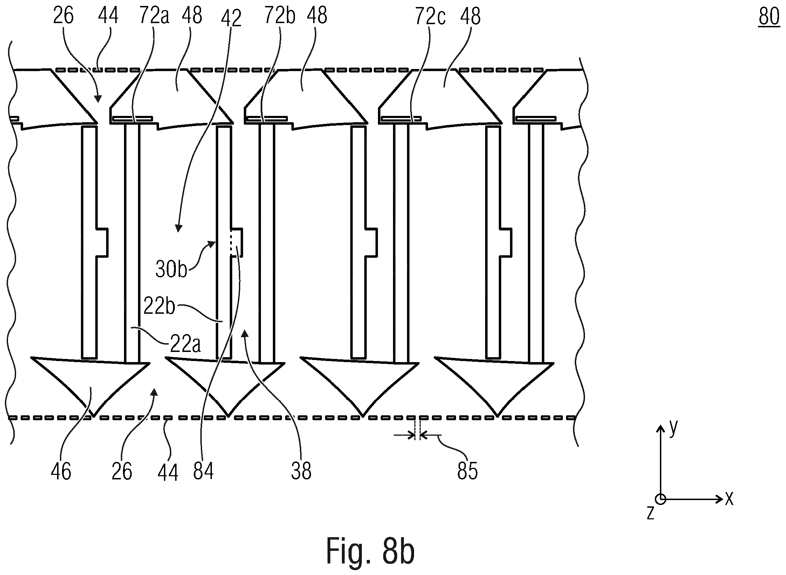

Fig. 8b zeigt eine schematische Aufsicht des MEMS-Wandlers 80, wobei die Abstandsschicht 34b und die Schicht 32b beispielhaft nicht gezeigt sind. Das MEMS 80 umfasst die Stabelemente 44 in Bereichen der Öffnungen 26. Die Bereiche 48 können die Federelemente 72a-c aufweisen. Die Bereiche 48 sind beispielhaft als Aufsicht der Zwischenschicht 36 dargestellt.

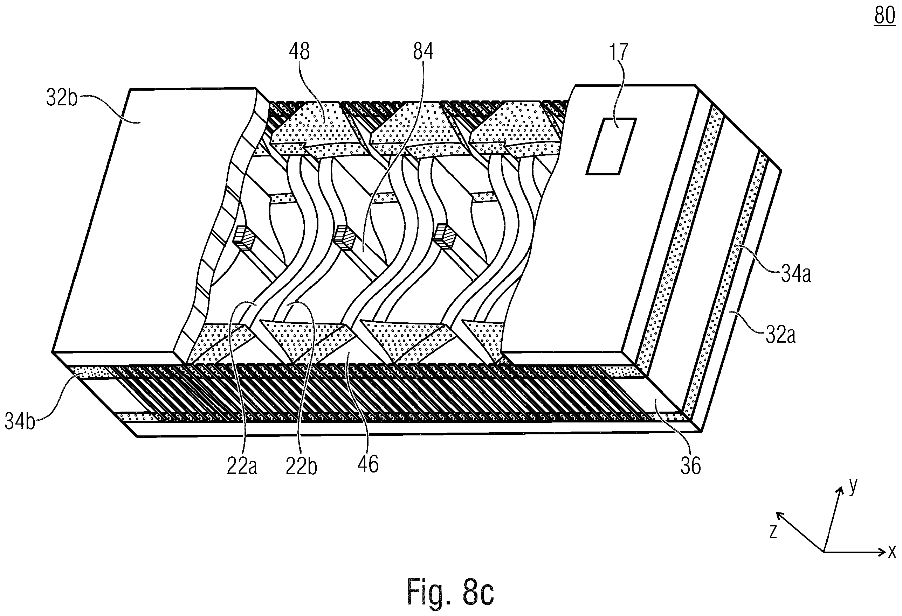

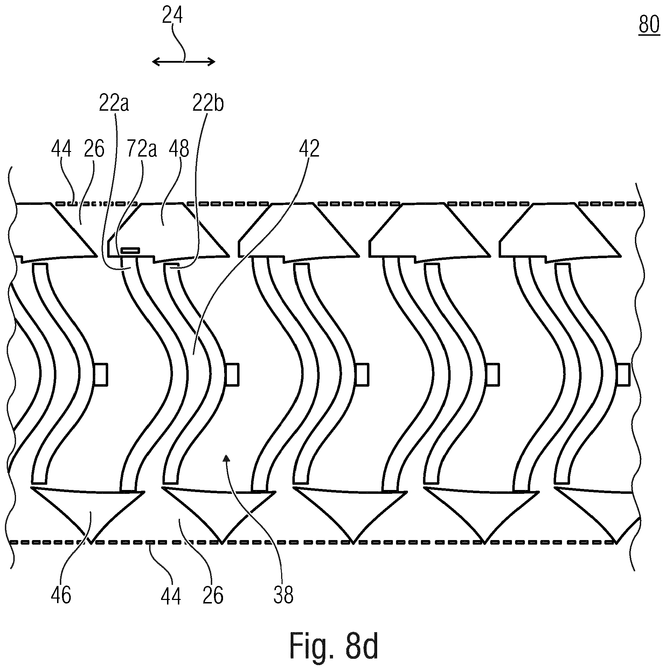

Das Ankerelement 84 kann einstückig mit dem verformbaren Element 22b und/oder einer Schicht des Substrats geformt sein. Wie es in Fig. 8 dargestellt ist, kann das Ankerelement 84 jedoch entlang der z-Richtung über das verformbare Element 22b hinausragen, um die Schichten 32a und 32b miteinander zu verbinden. Dies ermöglicht eine verringerte Schwingungsanfälligkeit der Schichten 32a und 32b. Alternativ kann das Ankerelement 84 auch aus einem anderen Stück und/oder aus einem anderen Material wie das mechanisch verformbare Element 22b gebildet sein. Das benachbart hierzu angeordnete verformbare Element 22a ist beispielsweise beidseitig fest mit dem Substrat in den Bereichen 48 oder 46 verbunden, beispielsweise form- oder kraftschlüssig.

Ein Abstand 85 zwischen Stabelementen 44 kann beispielsweise geringer sein als 1 $\mu$m, als 0,1 $\mu$m oder 0,05 $\mu$m.

Das Ankerelement 84 kann in einem Mittenbereich des verformbaren Elements 22b angeordnet sein. Der Mittenbereich kann bspw. einen geometrischen Schwerpunkt des verformbaren Elementes umfassen. Der Mittenbereich kann bspw. das Balkensegment 30b des verformbaren Elementes 40 sein.

Fig. 8c zeigt eine schematische perspektivische Ansicht des MEMS-Wandlers 80 in einem ausgelenkten Zustand. Äußere Bereiche des verformbaren Elements 22b können sich in eine Richtung hin zu dem verformbaren Element 22a bewegt haben, wobei Orte der äußeren Enden des verformbaren Elements 22a im Wesentlichen unverändert geblieben sind. Ein Mittenbereich des verformbaren Elements 22a kann sich in eine Richtung des verformbaren Elements 22b bewegt haben, wobei ein Ort des Mittenbereichs des verformbaren Elements 22b basierend auf dem Ankerelement 84 im Wesentlichen unverändert geblieben ist.

Fig. 8d zeigt eine schematische Aufsicht auf den MEMS-Wandler 80 in dem ausgelenkten Zustand, wie er in Fig. 8c beschrieben ist. Das Volumen der Kavität 42 ist verglichen mit der Ansicht der Fig. 8b verringert, wohingegen ein Volumen der Teilkavität 38 vergrößert ist. Das Federelement 72a kann zu einer reduzierten Krafteinleitung in das verformbare Element 22a führen, kann jedoch auch nicht angeordnet sein. Zwischen den Balkenstrukturen des ersten elektromechanischen Wandlers und des zweiten elektromechanischen Wandlers bzw. zwischen den Aktoren 22a und 22b kann eine erste Teilkavität 42 angeordnet sein, die an eine Öffnung 26 des Substrats angrenzt.

In anderen Worten zeigen Fig. 8a und 8b eine schematische 3D-Darstellung bzw. eine Aufsicht einer Variante, bei der eine Chipfläche des MEMS-Wandlers sehr effizient ausnutzbar ist. Wie in der Basiskonfiguration, wie sie im Zusammenhang mit den Fig. 2a-c beschrieben ist, können ausschließlich oder vorwiegend Biegeaktoren eingesetzt werden, d. h. auf das zusätzliche starre Plattenelement kann verzichtet werden. Die Kammer 42 wird, wie in Fig. 8a illustriert, durch zwei nicht ausgelenkte S-Aktoren 22a und 22b begrenzt. Der links (negative x-Richtung) begrenzende S-Aktor 22a kann mit seinen beiden Enden in der Zeichnung oben bzw. unten (also entlang der positiven oder negativen y-Richtung) mit dem übrigen Bauelement verbunden sein. Der rechts begrenzende S-Aktor 22b kann an einem Pfosten (Ankerelement) 84 befestigt sein. Die beiden Enden dieses S-Aktors können frei beweglich sein. Der Pfosten 84 kann mit dem oberen und unteren Deckel 32a bzw. 32b fest verbunden sein. Bei anliegendem Signal verbiegen sich beide Aktoren S-förmig. Das in Fig. 8a verdeckt dargestellte Federelement 72a, das durch eine Aussparung beeinflusst ist, kann zur Zugentlastung dienen. Das Federelement ist in der Zeichenebene der Fig. 8b entlang der lateralen Bewegungsrichtung 24 in dem Element 48 angeordnet, so dass das Federelement 72a entlang der lateralen Bewegungsrichtung 24 fest eingespannt ist. Das Federelement 72a kann, wie es beispielsweise in Fig. 8 dargestellt ist, basierend auf den Abstandsschichten 34a und 34b eine feste Verbindung hierzu aufweisen und ebenfalls eingespannt sein. Alternativ können die Schichten 34a und 34b auch so strukturiert werden, dass das Federelement 72a keinen Kontakt zu der Abstandsschicht 34a und/oder 34b aufweist und somit eine höhere Nachgiebigkeit aufweisen kann.

Wie es in den Fig. 8c und 8d dargestellt ist, können die bauchförmigen Verwölbungen des S-Aktors 22a in Richtung des Pfostens 84 bewegt werden, so dass die Mitte des S-Aktors 22a nahezu die Mitte des S-Aktors 22b berührt. Gleichzeitig haben sich die freien Enden des S-Aktors 22b in Richtung der festen Einspannung des S-Aktors 22a bewegt, so dass diese sich ebenfalls nahezu berühren. Die aktuierte Form der beiden S-Aktoren kann näherungsweise gleich oder identisch sein, so dass sich die Kammer 42 bei genügender Auslenkung der Aktoren praktisch oder nahezu vollständig schließen kann. Das ursprüngliche Volumen der Kammer 42 kann also vollständig für die Generation des Volumenstroms oder für dessen Erfassung eingesetzt werden. Im gleichen Maße, wie die Kammer 42 an Volumen verliert, kann die Kammer 38 an Volumen gewinnen, wodurch bei geeigneter Dimensionierung der die Strömungen beeinflussenden Elemente verhindert werden kann, dass eine zu hohe durch dynamische Effekte auftretende Druckdifferenz zwischen den Kammern 38 und 42 die Bewegung der Aktoren beeinträchtigt. Die Elemente 46 und 48 können so ausgestaltet sein, dass der Abstand zu den freien Enden der Aktoren 22b unabhängig von der Auslenkung der Enden klein und/oder in etwa konstant bleibt. Zur Zugentlastung der Aktoren 22a können, wie oben beschrieben, Biegefederelemente 72a angeordnet sein.

Vorangehend beschriebene Ausführungsbeispiele können weitere Aktoren umfassen, die in entstehenden Strömungskanälen angeordnet sind. Die weiteren Aktoren können bspw. nicht der direkten Schallerzeugung dienen, wie es beispielsweise die elektromechanischen Wandler 18 ermöglichen können, sondern zur variablen Einstellung der Strömungseigenschaften nutzbar sein. Damit kann beispielsweise die Dämpfung und folglich die Breite der Resonanzkurve individuell für jede Kammer anforderungsspezifisch und flexibel während des Betriebs des Bauelements (MEMS-Wandler) angepasst werden.

In eingangs aufgeführter Abschätzung wurde die Volumenänderung pro aktiver Fläche ($\Delta V/A$) für einen Membranlautsprecher nach dem Stand der Technik auf 3,75 $\mu$m abgeschätzt. Dies kann, wie nachfolgend ausgeführt, für eine in den Fig. 8a-c dargestellten MEMS-Wandler anhand von für Mikrotechnik-Technologie sinnvollen Dimensionen erneut abgeschätzt wurden, um eine Abschätzung für eine aktive Fläche $\Delta V/A$ zu erhalten. Dazu kann für eine Breite der Aktoren (in Abbildung 8a in x-Richtung) ein Wert von 5 $\mu$m angenommen werden. Die Breite des Pfostens 84 kann einen Wert von ebenfalls 5 $\mu$m aufweisen. Für den Abstand der Aktoren, welche die Seitenwände der Kammer 38 bilden, (etwa in den Fig. 8a und 8b im unausgelenkten Zustand) können 10 $\mu$m angenommen werden. Für einen Abstand der Aktoren, welche die Seitenwände der Kammer 42 bilden, (Fig. 8a und 8b im unausgelenkten Zustand) können 100 $\mu$m angenommen werden. Ein planarer Füllfaktor $F_p$, der angeben kann, welcher Anteil der aktiven Fläche für die Generation eines Volumenstroms nutzbar ist, kann sich dann zu

$$F_p = 100 / (5 + 100 + 5 + 10) = 83 \%$$

ergeben.

$\Delta V/A$ kann ausgedrückt werden als: $\Delta V/A = A \times F_p h / A = F_p h$

In obigem Ausdruck kann h die Höhe der Kammer (bspw. die z-Richtung in Fig. 8a) darstellen. Vereinfacht kann hierfür lediglich die Aktorhöhe angenommen werden. Eine Dicke der Abstandsschichten 34a und 34b kann vernachlässigt werden. Im Vergleich mit den obigen 3,75 $\mu$m für die Membranlautsprecher wird klar, dass bereits eine Aktorhöhe von lediglich 3,75 $\mu$m / $F_p$ (also 4,5 $\mu$m) hinreichend ist, um denselben Volumenstrom pro aktiver Fläche zur Verfügung zu stellen. Mit einer in mikromechanischer Technologie ohne erhöhte Aufwände herstellbaren Aktordicke h von in etwa 50 $\mu$m kann der Wert bereits um mehr als den Faktor 10 höher liegen als der des MEMS-Membranlautsprechers.

In Ausführungsbeispielen gemäß dem MEMS-Wandler 80, die ohne starre Platten ausgeführt sind, können parasitäre Schwingungen aufgrund der deutlich reduzierten Anzahl mechanischer Elemente und mechanischer Verbindungen wesentlich einfacher beherrschbar bzw. reduzierbar sein als in Varianten, die die Plattenelemente und ggf. weitere verformbare Elemente zwischen dem verformbaren Element und dem Plattenelement aufweisen. Ein serielles Hintereinanderschalten von Aktoren, wie beispielsweise in den Fig. 7b und 7c dargestellt, kann zur Erzielung größerer Hübe bzw. größerer Kräfte dienen.

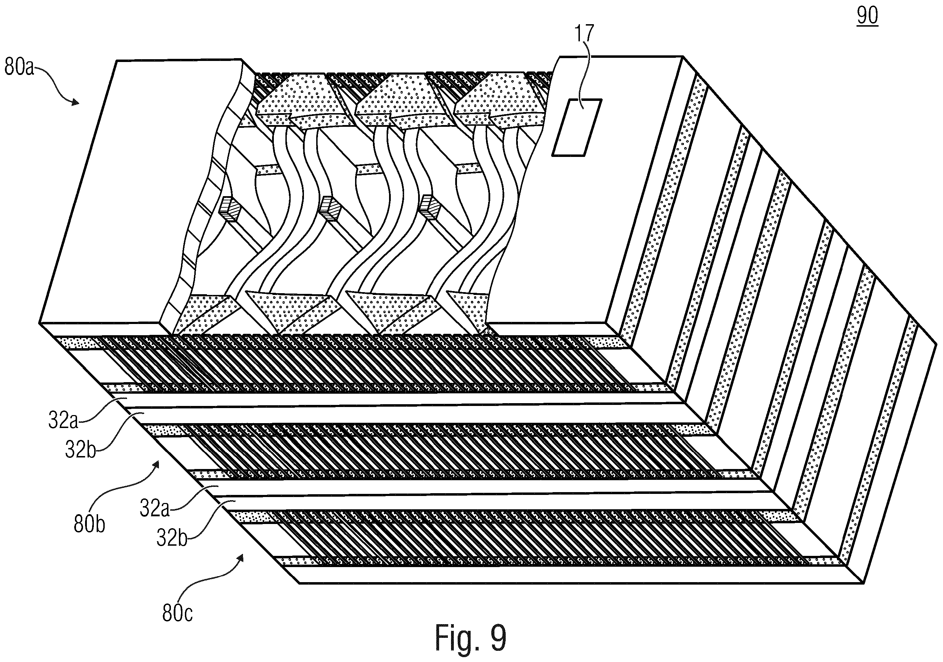

Fig. 9 zeigt eine schematische perspektivische Ansicht eines Stapels 90. Der Stapel 90 umfasst einen MEMS-Wandler 80a, der mit weiteren MEMS-Wandlern 80b und 80c zu dem Stapel 90 verbunden und in dem Stapel 90 angeordnet ist. Die elektromechanischen Wandler des MEMS-Wandlers 80a und eines weiteren MEMS-Wandlers 80b und/oder 80c können gemeinsam ansteuerbar sein. Das bedeutet, dass bei gleichbleibender Chipfläche ein Volumenstrom, der erzeugbar oder erfassbar ist, erhöht ist. Obwohl der Stapel 90 so beschrieben ist, dass er die MEMS-Wandler 80a, 80b und 80c umfasst, können alternativ oder zusätzlich andere MEMS-Wandler 10, 20, und/oder 50 angeordnet sein. Obwohl der Stapel 90 so beschrieben ist, dass er drei MEMS-Wandler umfasst, kann der Stapel 90 auch eine andere Anzahl von MEMS-Wandlern, etwa zwei, vier, fünf, sechs oder mehr MEMS-Wandler umfassen. Die Kavitäten oder Teilkavitäten der MEMS-Wandler bzw. benachbarter MEMS-Wandler, die in dem Stapel 90 angeordnet sind, können miteinander verbunden sein. Die Kavitäten oder Teilkavitäten können bspw. durch Öffnungen in Schichten zwischen einzelnen MEMS-Wandlern verbunden sein. Die elektronische Schaltung 17 kann ausgebildet sein, um einen oder mehrere der MEMS-Wandler anzusteuern, d. h., um die Konvertierung zwischen einer Verformung des verformbaren Elements und einem elektrischen Signal bereitzustellen. Der Stapel 90 kann somit zumindest eine, aber auch mehrere elektronische Schaltungen 17 aufweisen.

In anderen Worten können basierend auf Siliziumtechnologie Scheiben bzw. Chips (MEMS-Wandler) beispielsweise durch Bondverfahren gestapelt werden, so dass sich in diesem Fall, im Gegensatz zu den klassischen Membranlautsprechern, eine weitere Erhöhung des Volumenstroms ergeben kann. Bei einem Einsatz von Technologien zur Abdünnung der einzelnen Scheiben bzw. Chips vor Stapelung kann die Stapelhöhe gering gehalten werden. Eine derartige Technologie kann beispielsweise einen Ätzprozess und/oder einen Schleifprozess umfassen.

Eine Reduzierung einer Schichtdicke der Schichten 32a und/oder 32b, die benachbart zueinander angeordnet sind, kann so weit geführt werden, dass eine oder gar beide dieser Schichten entfernt werden. Alternativ oder zusätzlich kann zur Verringerung der Stapelhöhe ein Herstellungsprozess so ausgeführt werden, dass bestimmte Unter- bzw. Oberdeckel (Schichten 32a bzw. 32b) weggelassen werden. Beispielsweise könnte der Stapel 90 so gebildet sein, dass der MEMS-Wandler 80b und/oder 80c jeweils ohne Schicht 32b ausgeführt sind.

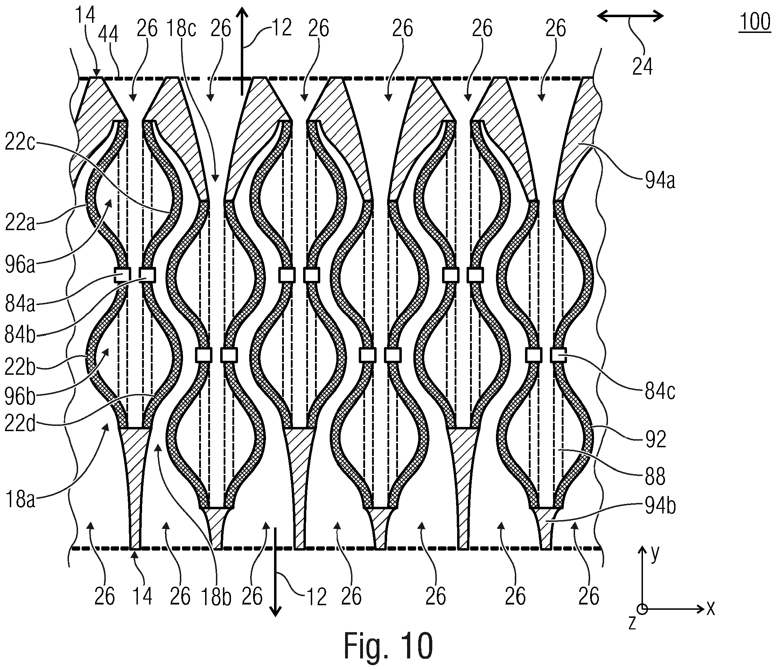

Fig. 10 zeigt eine schematische perspektivische Aufsicht auf einen Ausschnitt eines MEMS-Wandlers 100, bei dem zwischen Seiten des Substrats 14 verformbare Elemente 22a-d angeordnet sind. Die verformbaren Elemente 22a und 22b sind mittelbar über das Ankerelement 84a verbunden. Das bedeutet, dass Enden der verformbaren Elemente 22a und 22b mit dem Substrat, ggf. mit dem Ankerelement 84a fest verbunden und mithin (fest) eingespannt sein können. Das bedeutet, dass die verformbaren Elemente 22a-d oder andere verformbare Elemente gemäß weiterer Ausführungsbeispiele eine Balkenstruktur aufweisen können. Die Balkenstruktur kann an einem ersten und einem zweiten Ende fest eingespannt sein. Eine Einspannung von Enden eines verformbaren Elementes 22a-d bzw. einer Balkenstruktur ermöglicht eine Vorauslenkung der verformbaren Elemente (etwa aufgrund von Schichtspannungsgradienten) zu reduzieren oder deutlich zu reduzieren. Somit können die Spalte zwischen den Deckeln und den Aktoren viel geringer ausfallen, was für einige Anwendungen erhebliche Effizienzvorteile hat

Die verformbaren Elemente 22a-d sind bspw. jeweils beidseitig fest eingespannt. Eine feste Einspannung kann mittels einer Anordnung oder Erzeugung der verformbaren Elemente 22a und/oder 22b an dem Substrat 14 und/oder an einem Ankerelement 84a bzw. 84b erhalten werden. Gestrichelte Linien 88 deuten einen unausgelenkten Zustand an, wohingegen durchgezogene Balken 92 eine ausgelenkte Form der verformbaren Elemente 22a-d andeuten. Ausformungen oder Elemente 94a und 94b des Substrats 14 können eine Positionierung der verformbaren Elemente 22a-d entlang der y-Richtung ermöglichen. Eine paarweise Position von elektromechanischen Wandlern 18a-c kann basierend auf den Elementen 94a und 94b verschoben sein. Benachbart und/oder paarweise zueinander angeordnete elektromechanische Wandler 18a und 18b können entgegengesetzt zueinander verformbar sein.

Das verformbare Element 22a und ggf. ein gegenüberliegendes verformbares Element 22c können ausgebildet sein, um basierend auf der Verformung einen Teilkavitätsabschnitt 96a zu beeinflussen, d. h. zu vergrößern oder zu verkleinern bzw. um basierend auf dem Volumenstrom in dem Teilkavitätsabschnitt 96a eine Verformung auszuführen. Das verformbare Element 22b und ggf. das gegenüberliegend angeordnete verformbare Element 22d können ausgebildet sein, um einen Teilkavitätsabschnitt 96b zu beeinflussen. Die Teilkavitätsabschnitte 96a und 96b können miteinander verbunden sein, etwa in einem Bereich der Ankerelemente 84a und 84b. Die Verformung der verformbaren Elemente 22a-d kann so erhalten werden, dass sich die verformbaren Elemente 22a und 22c bzw. 22b und 22d mit einer voneinander verschiedenen Frequenz verformen, d. h. eine Volumenänderung in dem Teilkavitätsabschnitt 96a kann mit einer Frequenz erfolgen, die von einer Frequenz verschieden ist, mit der sich ein Volumen des Teilkavitätsabschnitts 96b ändert. Wird der MEMS-Wandler beispielsweise als Lautsprecher genutzt, so können basierend auf der frequenzmäßig verschiedenen Volumenänderung verschiedene Frequenzen in den Teilkavitätsabschnitten erhalten werden. Wird der MEMS-Wandler 100 beispielsweise als Mikrophon verwendet, können die Teilkavitätsabschnitten 96a und 96b beispielsweise voneinander verschiedene Resonanzfrequenzen aufweisen. Alternativ können weitere Teilkavitätsabschnitte und weitere verformbare Elemente entlang der y-Richtung angeordnet sein, so dass der MEMS-Wandler 100 beispielsweise weitere Frequenzen erzeugen oder weitere Resonanzfrequenzen aufweisen kann.

Alternativ können die verformbaren Elementen 22a und 22b oder die verformbaren Elemente 22c und 22d auch direkt miteinander verbunden sein. Beispielsweise können Ankerelemente in einem Mittenbereich eines oder mehrerer verformbarer Elemente 22a-d angeordnet sein, um die Verformung der verformbaren Elemente 22a-d zu beeinflussen. Das bedeutet, dass die verformbaren Elemente 22a und 22b unmittelbar miteinander verbunden werden können. Alternativ kann auch ein Federelement oder ein anderes Element zwischen den verformbaren Elementen 22a und 22b angeordnet sein.

Der MEMS-Wandler 100 kann so ausgeführt sein, dass in einem ersten Zeitintervall der Volumenstrom 12 in einer positiven y-Richtung aus Öffnungen 26 und nachfolgend, in einem zweiten Zeitintervall der Volumenstrom 12 in einer negativen y-Richtung aus Öffnungen 26 erhalten wird.

In anderen Worten zeigt Fig. 10 eine Konfiguration in der wiederum, ggf. ausschließlich, S-förmige Aktoren angeordnet sind. Die S-förmigen Aktoren können zur Verdeutlichung des Prinzips in der Abbildung sowohl aktuiert (durchgezogene Linien 92) als auch nichtaktuiert (gestrichelte Linien 88) darstellbar sein. Aktuierter und nicht-aktuierter Zustand können durch entsprechendes Design auch vertauschbar sein. Die S-förmigen Aktoren (verformbare Elemente 22a-d) können sowohl an ihrem einen (oberen) als auch an ihrem anderen (unteren) Ende eingespannt sein. Dazu können die Ankerelemente 84a-b genutzt werden. Die Ankerelemente 84a-b können aus den Schichten 34a, 36 und 34b gebildet sein und mit einer Schicht 32a und/oder 32b verbunden sein. Abstände zwischen den freien Enden der S-förmigen Aktoren und Elementen 94a oder 94b können basierend auf dieser Konfiguration entfallen. Dies kann geringere Umströmungsverluste ermöglichen. Ein Ausgangssubstrat kann so prozessiert werden, dass aus diesem die Aktoren herstellbar sind, wobei das Ausgangssubstrat Schichtspannungsgradienten aufweisen kann bzw. Schichtspannungsgradienten können während der Herstellung der Aktoren eingeführt werden. Eine dadurch induzierte Auslenkung der verformbaren Elementen kann basierend auf der Anordnung der Ankerelemente 84a und/oder 84b reduziert oder verhindert werden. Insbesondere kann die beidseitige Aufhängung der verformbaren Elemente zu einer Verringerung oder Verhinderung einer Auslenkung derselben in Richtung einer der Schichten 32a oder 32b führen. Die Abstandschichten 34a bzw. 34b können dementsprechend dünner ausfallen, was wiederum eine Reduktion der Umströmungsverluste bewirken kann. Je Kammer (Teilkavitätsabschnitt 96a oder 96b) kann durch zwei S-förmige Aktoren begrenzt sein. In dem Beispiel der Fig. 10 können zwei Kammern seriell hintereinander geschaltet sein. Die Anzahl der seriell geschalteten Kammern kann basierend auf einer auf dem Chip zur Verfügung gestellten Fläche unter Berücksichtigung der akustischen Eigenschaften, insbesondere der Resonanzfrequenz der S-förmigen Aktoren bzw. des Aktor-Kammersystems wählbar sein und kann zwischen 1 und einer hohen Zahl, beispielsweise mehr als 3, mehr als 5 oder mehr als 10 variieren.

Die Elemente 94a und 94b können optional angeordnet sein, d. h. der MEMS-Wandler 100 kann auch ohne diese Elemente ausgeführt sein. Wird beispielsweise aufgrund einer speziellen Gestaltung oder Ansteuerung der elektromechanischen Wandler und/oder der verformbaren Elemente ein entsprechender Teil des Aktors nicht ausgelenkt, so kann auf eine Beabstandung mittels der Elemente 94a oder 94b von dem Substrat 14 verzichtet werden. Es kann ein Mehrfach-S-Aktor (wellenförmiger Aktor) ausgeführt werden. Insbesondere ermöglicht dies ein Erhalten niedriger Resonanzfrequenzen basierend auf dieser Anordnung, da eine Resonanzfrequenz des Balkens (verformbaren Elements) mit zunehmender Länge abnehmen kann.

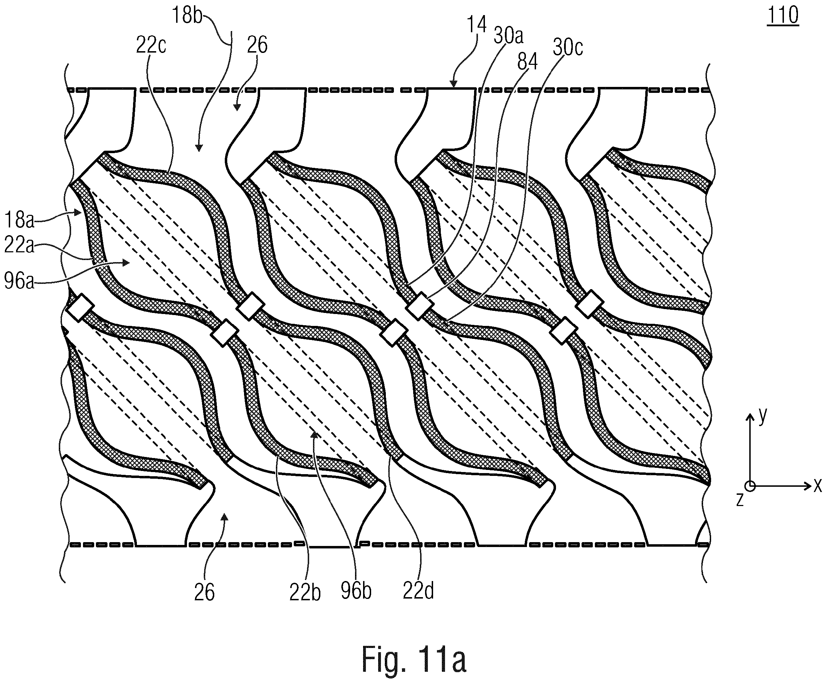

Fig. 11a zeigt eine schematische Aufsicht auf einen Ausschnitt eines MEMS-Wandlers 110, bei dem die elektromechanischen Wandler 18a-b verglichen mit der Konfiguration der Fig. 10 bezogen auf eine laterale Richtung des Substrats 14, beispielsweise die x-Richtung, schräg angeordnet sind. Bei einer verglichen mit dem MEMS-Wandler 100 gleichen Ausdehnung entlang der y-Richtung können die elektromechanischen Wandler 18ab eine längere axiale Ausdehnung aufweisen. Dies kann größere Teilkavitätsabschnitte 96a und/oder 96b und/oder eine höhere Anzahl von seriell hintereinander geschalteten Teilkavitätsabschnitte bzw. verformbaren Elementen ermöglichen.

Ein äußeres Balkensegment 30a eines verformbaren Elements kann mittelbar über das Ankerelement 84 mit einem äußeren Balkensegment 30c eines weiteren Verformbaren Elementes mit einander verbunden sein. Alternativ können die Balkensegmente 30a und 30c auch unmittelbar, d. h., direkt, mit einander verbunden sein.

In anderen Worten zeigt Fig. 11a ein weiteres Ausführungsbeispiel, bei dem die aktive Fläche gegenüber den Ausführungen der Fig. 10 um 45° gedreht ist, wobei ggf. die zur Verfügung stehende Chipfläche in einem höheren Umfang ausnutzbar ist. Trichterförmige Öffnungen 26 können so gestaltet werden, dass der Schall vorzugsweise senkrecht zur Chipkantenfläche, d. h. entlang der y-Richtung in positiver oder negativer Richtung hiervon emittiert werden kann.

Jedes der vorangehend beschriebenen verformbaren Elemente kann auch als eine Vielzahl von miteinander verschalteten verformbaren Elementen gebildet sein.

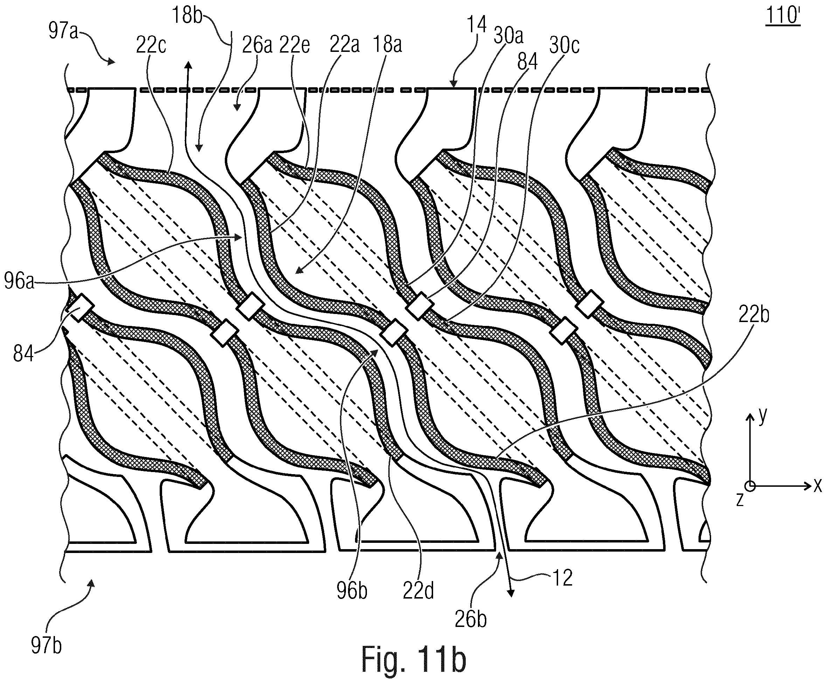

Fig. 11b zeigt eine schematische Aufsicht auf einen Ausschnitt eines MEMS-Wandlers 110', der bspw. als Pumpe einsetzbar ist. Verglichen mit dem MEMS-Wandler 110 aus Fig. 11a können die Teilkavitätsabschnitte 96a und 96b über zwei Öffnungen 26a und 26b mit einer Umgebung des MEMS-Wandlers 110' verbunden sein. Die Teilkavitätsabschnitte 96a und 96b können über die Öffnung 26a mit einer ersten Seite 97a des MEMS-Wandlers 110' verbunden sein und über die Öffnung 26b mit einer zweiten Seite 97a des MEMS-Wandlers 110' verbunden sein. Die erste Seite 97a und die zweite Seite 97b können bspw. gegenüberliegend zueinander angeordnet sein. Alternativ können die Seiten 97a und 97b auch einen Winkel zueinander aufweisen. Bspw. kann eine der Seiten 97a oder 97b eine Seitenfläche des MEMS-Wandlers 110' aufweisen und die andere Seite 97b oder 97a eine Hauptseite (bspw. eine Ober- oder Unterseite) des MEMS-Wandlers 110' umfassen.

Basierend auf eine Verformung der verformbaren Elemente 22a-d kann der Fluidstrom von der ersten Seite 97a zu der zweiten Seite 97b oder andersherum durch den MEMS-Wandler 110' hindurch generierbar sein. Bspw. können die verformbaren Elemente 22a und 22c in einem ersten Zeitintervall verformt und das Volumen des Teilkavitätsabschnitts 96a verkleinert werden. In einem zweiten Zeitintervall kann das Volumen des Teilkavitätsabschnitts 96b verkleinert werden. Basierend auf einer Reihenfolge der Verkleinerung oder Vergrößerung der Volumina kann eine Richtung des Volumenstroms 12 beeinflusst werden. Alternativ können auch mehrere Teilkavitätsabschnitte hintereinander angeordnet sein oder lediglich ein Teilkavitätsabschnitt angeordnet sein.

Vereinfacht ausgedrückt kann die Funktion einer Pumpe erhalten werden, indem der Volumenstrom 12 anstelle von Hin und Her analog zu einem Lautsprecher gemäß einem Durchflussprinzip durch den MEMS-Wandler erzeugt wird. Eine Ein- und eine Austrittsseite des MEMS-Wandlers können gegenüberliegend angeordnet sein, können aber alternativ auch einen Winkel zu einander aufweisen oder an der gleichen Seite örtlich oder fluidisch voneinander beabstandet sein. Die Kavität umfassend die Teilkavitätsabschnitte 96a und 96b kann die Öffnungen 26a und 26b in dem Substrat aufweisen. Zumindest einer der elektromechanischen Wandler 18a oder 18b kann ausgebildet sein, um den Volumenstrom 12 basierend auf dem Fluid bereitzustellen. Bspw. kann zumindest einer der elektromechanischen Wandler 18a oder 18b ausgebildet sein, um das Fluid basierend auf einer Aktuierung des elektromechanischen Wandler durch die erste Öffnung 26a in eine Richtung der Kavität zu befördern oder, um das Fluid basierend auf der Aktuierung durch die zweite Öffnung 26b in eine Richtung weg von der Kavität zu befördern oder andersherum.

Obwohl eine Pumpenfunktion im Zusammenhang mit dem MEMS-Wandler 110' beschrieben ist, können auch andere hier beschriebene Ausführungsbeispiele als Pumpe oder Mikropumpe nutzbar sein, etwa indem eine Anordnung von Öffnungen der Kavität, Teilkavität oder zumindest eines Teilkavitätsabschnitts angepasst wird.

Bei einer gleichzeitigen Auslenkung der verformbaren Elemente 22a und 22e kann in einem dazwischenliegenden Volumen ein Unterdruck (alternativ Überdruck) resultieren, der der Verformung oder Auslenkung entgegenwirkt. Das Volumen kann eine Öffnung aufweisen, bspw. in der Schicht 32a und/oder 32b, so dass ein Druckausgleich in diesem Volumen ermöglicht ist. Dies ermöglicht einen effizienten Betrieb des MEMS-Wandlers 110'.

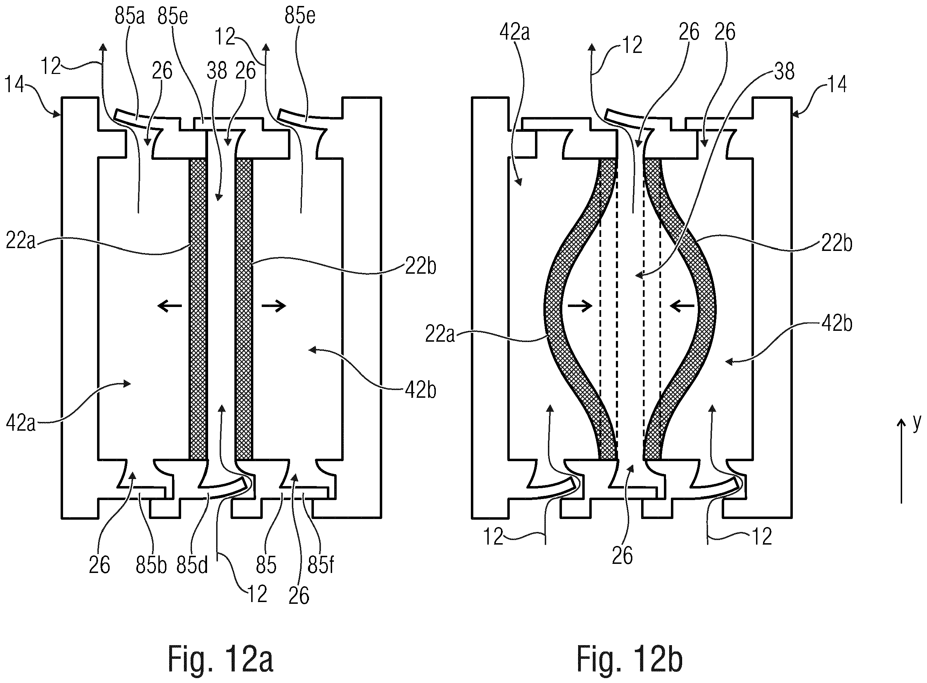

Fig. 12a zeigt eine schematische Ansicht eines MEMS-Wandlers 120, der bspw. als MEMS-Pumpe einsetzbar ist, in einem ersten Zustand. Der MEMS-Wandler 120 weist bspw. zwei verformbaren Elemente 22a und 22b auf, die eine Balkenstruktur ausweisen und beidseitig an dem Substrat 14 eingespannt oder fest eingespannt sind. Alternativ kann der MEMS-Wandler 120 auch mit einem verformbaren Element oder mit mehr als zwei verformbaren Elementen ausgeführt sein.

Fig. 12b zeigt den MEMS-Wandler 120 in einem zweiten Zustand. Basierend auf einer Verformung zumindest eines verformbaren Elementes 22a und/oder 22b kann ausgehend von dem ersten Zustand, wie er in Fig. 12a dargestellt ist, der zweite Zustand erhalten werden. Ausgehend von dem zweiten Zustand kann basierend auf einer Rückverformung des oder der verformbaren Elements oder Elemente der erste Zustand erhalten werden. In dem zweiten Zustand ist die Teilkavität 38 zwischen den verformbaren Elementen 22a und 22b bspw. gegenüber dem ersten Zustand vergrößert. Während eines Übergangs vom ersten in den zweiten Zustand kann ein Unterdruck in der Teilkavität 38 entstehen. Währen eines Übergangs vom zweiten Zustand in den ersten Zustand kann ein Unterdruck in der Teilkavität 38 entstehen.

Zwischen einem verformbaren Element 22a bzw. 22b und dem Substrat 14 ist eine Teilkavität 42a bzw. 42b angeordnet, deren Volumina komplementär zu dem Volumen der Teilkavität 38 verkleinert bzw. vergrößert werden können, wobei ebenfalls komplementär zu der Teilkavität 38 ein Überdruck bzw. Unterdruck basierend auf der Verformung der verformbaren Elemente erhalten werden kann.

In einem Bereich einer jeweiligen Öffnung 26 kann eine Ventilstruktur 85a-f angeordnet sein. Eine oder mehrere Ventilstrukturen 85a-f können bspw. aus einem Material des Substrats 14 gebildet sein. Die Ventilstrukturen können einstückig mit einer oder mehreren Schichten des Substrats 14 gebildet sein und bspw. mittels eines Ätzprozesses erzeugt werden.

Die Ventilstrukturen können ausgebildet sein, um einen Durchfluss des Volumenstroms 12 durch die Öffnung 26 zumindest entlang einer Richtung zu behindern, d. h., zu reduzieren oder zu verhindern. Bspw. können die Ventilstrukturen 85b, 85d und 85f ausgebildet sein, um einen Austritt des Fluids aus der jeweiligen Teilkavität zu reduzieren oder zu verhindern. Alternativ oder zusätzlich können die Ventilstrukturen 85a, 85c und 85e ausgebildet sein, um einen Eintritt des Fluids in die jeweilige Teilkavität zu reduzieren oder zu verhindern. Eine oder mehrere Ventilstrukturen 85a-f können passiv ausgebildet sein, etwa als einseitig eingespannte Biegebalkenstruktur oder Zungenstruktur. Alternativ oder zusätzlich können eine oder mehrere Ventilstrukturen 85a-f aktiv ausgebildet sein, etwa als elektromechanischer Wandler oder verformbares Element. Vereinfacht ausgedrückt, können die Ventilstrukturen 85a-f wie die anderen Aktoren (elektromechanischer Wandler) des MEMS-Wandlers aktuierbar sein.

Die Ventilstruktur 85d kann bspw. ausgebildet sein, um basierend auf einem Unterdruck in der Teilkavität 38 den Volumenstrom 12 in die Teilkavität 38 einströmen zu lassen, während die Ventilstruktur 85c gleichzeitig einen Eintritt des Volumenstrom 12 in die Teilkavität 38 reduziert oder verhindert. Tritt, wie in Fig. 12b gezeigt, ein Überdruck in der Teilkavität 38 auf, so kann die Ventilstruktur 85c ausgebildet sein, um basierend auf dem Überdruck den Volumenstrom 12 aus der Teilkavität 38 ausströmen zu lassen, während die Ventilstruktur 85d gleichzeitig einen Austritt des Volumenstrom 12 aus der Teilkavität 38 reduziert oder verhindert.

Eine Funktion der Ventilstrukturen 85a, 85b bzw. 85e und 85f kann bzgl. der Teilkavitäten 42a bzw. 42b gleich oder vergleichbar sein. Die Ventilstrukturen 85a-f können auch als Rückschlagventile bezeichnet werden und ermöglichen bspw. eine Einstellung einer Vorzugsrichtung des Volumenstrom 12.

Obwohl der MEMS-Wandler so beschrieben ist, dass bspw. der Volumenstrom aus den Teilkavitäten 38, 42a und 42b entlang der gleichen Richtung (positive y-Richtung) und während unterschiedlichen Zeitintervallen, während denen ein Übergang zwischen dem ersten und dem zweiten Zustand erfolgt, strömt, können die Ventilstrukturen auch so angeordnet sein, dass der Volumenstrom aus zumindest einer Teilkavität 38, 42a oder 42b entlang einer anderen Richtung, etwa der negativen y-Richtung strömt.

Obwohl der MEMS-Wandler so beschrieben ist, dass die Ventilstrukturen 85a-f an jeder Öffnung 26 angeordnet sind, können alternativ Ventilstrukturen an keiner oder lediglich einigen Öffnungen 26 angeordnet sein.

Obwohl die Ventilstrukturen für eine Funktion als Rückschlagventil passiv ausgeführt sein können, können die Ventilstrukturen auch aktiv gebildet sein, das bedeutet, sie können ansteuerbar sein und im Sinne von Aktuatoren basierend auf der Ansteuerung einen geöffneten oder geschlossenen Zustand des Ventils bereitstellen. Insbesondere können zwei Ventilstrukturen 85a und 85b, 85c und 85d oder 85e und 85f, die jeweils einer Teilkavität zugeordnet sind, so angesteuert werden, dass Druckpulse in dem Fluidstrom 12 entstehen, etwa durch eine mit dem MEMS-Wandler verbundene Steuereinrichtung. Bspw. kann eine Aktuierung der elektromechanischen Wandler 18 so erfolgen, dass ein Über- oder Unterdruck in dem Fluid innerhalb der Teilkavitäten 42a, 42b aufgebaut wird und erst dann eine Öffnung der Ventilstrukturen 85a-f angesteuert wird.

In anderen Worten kann mit derartigen Druckpulsen auch eine näherungsweise Nachbildung einer niederfrequenten Schallwelle durch kurze Druckpulse erreicht werden. Durch mehrere seriell hintereinander angeordnete Kammern kann dies in nahezu kontinuierlicher Weise geschehen. Ähnlich ist das auch mit parallel nebeneinander liegenden Kammern möglich. Fig. 12a zeigt ein Beispiel im nicht aktuierten Zustand, bei dem jede Kammer oben und unten mit je einem Ventil versehen ist, das aktiv gebildet sein kann. Jedes Ventil kann individuell geöffnet oder geschlossen werden. Auch eine teilweise Öffnung/Schließung ist denkbar. Die Ventilbalken können genauso gestaltet bzw. betrieben werden, wie die beweglichen Seitenwände, d. h., die verformbaren Elemente. Sie können also auf demselben oder gleichen Aktorprinzip beruhen. Dabei können diese Ventil-Biegebalken auch so ausgestaltet werden, dass sie in beide Richtungen bewegbar sind, bzw. die Öffnung (durch entsprechende vom Biegeaktor-Ventil aufzubringende Gegenkraft) bei Fluidfluss, schließen (bis auf einen sehr kleinen Spalt, der für die Bewegung erforderlich ist Mit diesem Aufbau ist die volle Flexibilität zur Steuerung des Fluidflusses in Bezug auf Richtung bzw. Unter-/Überdruck gegeben, und zwar individuell für jede Kammer. Steht die Richtung für den Fluidfluss fest, kann auch mit Anschlägen für die VentilBalken gearbeitet werden ("Rückschlagventil").

In nochmals anderen Worten kann im ersten Zustand die mittlere Kammer (Teilkavität 38) durch die beiden dunkel dargestellten Aktoren (verformbaren Elemente 22a und 22b) expandiert werden, während die beiden äußeren Kammern (Teilkavitäten 42a und 42b) komprimiert werden. Erstere Kammer füllt sich über das Rückschlagventil 85d mit dem Fluid aus dem unteren Bereich. Letztere drücken durch das Rückschlagventil 85a bzw. 85e Fluid in den oberen Bereich. Im zweiten Zustand wird die mittlere Kammer komprimiert. Fluid wird in den oberen Bereich gedrückt. Die äußeren Kammern füllen sich mit dem Fluid aus dem unteren Bereich.

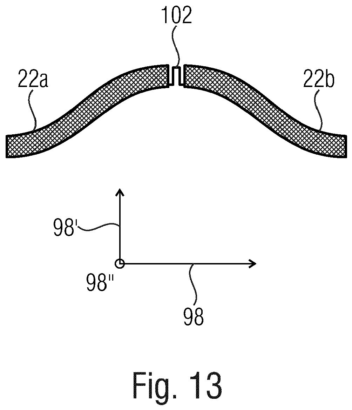

Fig. 13 zeigt eine schematische Ansicht eines ersten verformbaren Elements 22a und eines zweiten verformbaren Elements 22b, die entlang einer lateralen Erstreckungsrichtung 98 des verformbaren Elements 22a und/oder 22b miteinander verbunden sind. Zwischen dem verformbaren Element 22a und dem verformbaren Element 22b ist ein Federelement 102 angeordnet. Das Federelement 102 kann reduzierte mechanisch induzierte Rückstellkräfte in den verformbaren Elementen 22a und 22b bewirken. Beispielsweise kann das Federelement 102 in einer Richtung 98', die senkrecht zu der Richtung 98 angeordnet ist, eine geringe Steifigkeit aufweisen und entlang einer Richtung 98", die senkrecht zu der Richtung 98 und 98' im Raum angeordnet sein kann, eine hohe Steifigkeit. Die verformbaren Elemente 22a und 22b und das Federelement 102 können beispielsweise als das verformbare Element 22a in dem MEMS-Wandler 110 angeordnet sein.

In anderen Worten können zur Zugentlastung der beidseitig eingespannten S-förmigen Aktoren 22a-d an Einspannungsorten oder beispielsweise auch in einem Bereich zwischen Einspannungsorten, etwa mittig, der Aktoren geeignete Federelemente 102 angeordnet werden. Das Federelement 102 ist beispielsweise in der Mitte der Aktoren eingesetzt und ist in der gewünschten Richtung (98') besonders flexibel und in den beiden Richtungen (98 und 98") steif, d. h. es weist eine hohe oder höhere Steifigkeit auf. Das Federelement 102 kann zwischen auslenkbaren Enden der verformbaren Elemente 22a und 22b angeordnet sein. Das Federelement 102 kann entlang der lateralen Bewegungsrichtung 24 eine geringere Steifigkeit aufweisen als in eine Richtung senkrecht zu der lateralen Bewegungsrichtung 24.

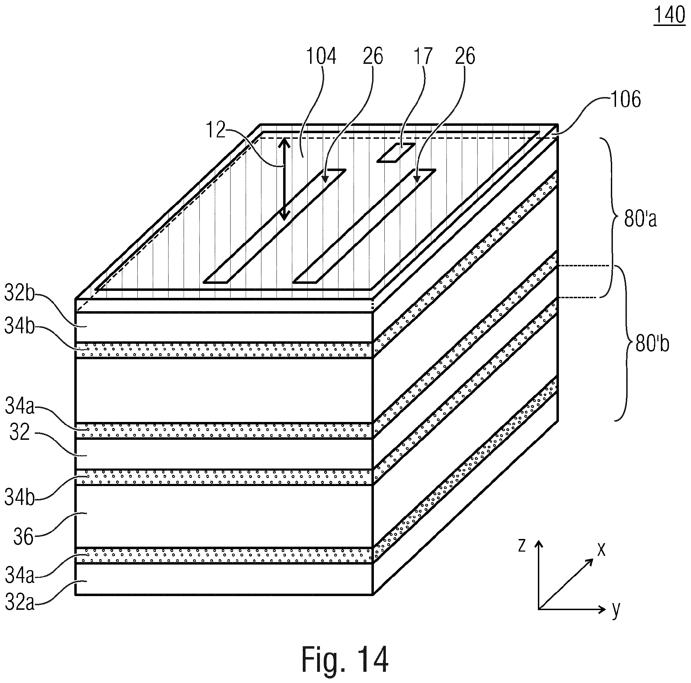

Fig. 14 zeigt eine schematische Ansicht eines Stapels 140 umfassend einen MEMS-Wandler 80'a und einen MEMS-Wandler 80'b, die miteinander verbunden sind und verglichen mit dem MEMS-Wandler 80 eine gemeinsame Schicht 32 aufweisen, das bedeutet, eine Schicht 32a oder 32b des MEMS-Wandlers 80 ist entfernt. Die elektronische Schaltung 17 kann ausgebildet sein, um die MEMS-Wandler 80'a und 80'b gemeinsam anzusteuern. Alternativ hierzu kann jeder der MEMS-Wandler 80'a und 80'b eine zugeordnete elektronische Schaltung aufweisen.

Ferner weist der MEMS-Wandler 80'a in der Schicht 32b die Öffnungen 26 auf, das bedeutet, verglichen mit dem MEMS-Wandler 80 ist eine Abstrahlrichtung des Volumenstroms 12 bzw. einer Eindringrichtung des Volumenstroms 12 senkrecht verkippt. Das bedeutet, dass eine Deckelfläche des MEMS-Wandlers eine Außenseite des Stapels bilden kann, wobei der MEMS-Wandler eine Öffnung in der Deckelfläche aufweisen kann, die einer dem zweiten MEMS-Wandler zugewandten Seite abgewandt angeordnet ist, wobei der Volumenstrom 12 des MEMS-Wandlers 80'a senkrecht oder entgegengesetzt zu dem Volumenstrom des MEMS-Wandlers 80'b aus oder in die Kavität eintritt.

An dem MEMS-Wandler 80'a kann ein Membranelement 104 angeordnet sein. Das Membranelement 104 kann so angeordnet sein, dass ein Austritt des Volumenstroms 12 aus der Kavität und durch das Membranelement 104 hindurch oder ein Eintritt des Volumenstroms 12 in die Kavität 16 zumindest teilweise zu verhindert ist. Die Kavität kann sich auf Bereiche ersteckend, die außerhalb des MEMS-Wandlers 80'a angeordnet sind und zwischen dem MEMS-Wandler 80'a und dem Membranelement 104 angeordnet sind. Basierend auf dem Volumenstrom 12 kann eine Auslenkung des Membranelements 104 bewirkbar sein. Das Membranelement 104 kann bspw. mittels einer Rahmenstruktur 106 an dem MEMS-Wandler 80'a angeordnet sein. Die Rahmenstruktur 106 kann an einer Seite des MEMS-Wandlers 80'a angeordnet sein, etwa an einer Hauptseite der Schicht 32b.

Alternativ kann auch eine Verkippung um einen von 90° verschiedenen Winkel ausgeführt sein. Der MEMS-Wandler 80'b kann Öffnungen an oder in der Schicht 32b aufweisen, so dass der Volumenstrom 12 an zwei Seiten des Stapels 140 in Kavitäten und/oder aus Kavitäten ein- bzw. austreten kann, wobei die Seiten einander entgegengesetzt angeordnet sind.

Alternativ oder zusätzlich kann der Stapel 140 auch einen weiteren oder anderen MEMS-Wandler aufweisen, etwa den MEMS-Wandler 20 oder 80. Beispielsweise kann der MEMS-Wandler 20 zwischen den MEMS-Wandlern 80'a und 80'b angeordnet sein. Dies ermöglicht einen Eintritt oder Austritt des Volumenstroms 12 in oder aus Kavitäten entlang einer Richtung, die senkrecht zu einer entsprechenden Richtung des MEMS-Wandlers 80'a ist.

In anderen Worten können Schallaustrittsöffnungen 26 statt an den Chipseitenflächen auch im unteren Deckel 32a und/oder im oberen Deckel 32b angebracht sein. Fig. 14 zeigt eine entsprechende vereinfachte Darstellung. Die Öffnungen 26 im oberen Deckel 32b sind erkennbar. Ähnliche Öffnungen können sich im unteren Deckel 32b befinden, sind jedoch basierend auf der perspektivischen Ansicht nicht erkennbar. Die Schicht 32 kann ebenfalls Öffnungen aufweisen, das bedeutet, Kavitäten, Teilkavitäten und/oder Teilkavitätsabschnitte der MEMS-Wandler 80'a und 80'b können miteinander verbunden sein. Über die Öffnungen in der Schicht 32 können vertikal (entlang der z-Richtung) übereinanderliegende Kammern miteinander verbunden sein.

Ein Gitter umfassend ein oder mehrere Stabelemente (Gitterstege) 44, das zur Einstellung der Dämpfung und insbesondere als Schutz vor Partikeln ausgebildet sein kann, kann auch in der in Fig. 14 beschriebenen Variante einfach realisiert werden. Beispielsweise können die Öffnungen 26 in den oberen Deckel 32b bzw. den unteren Deckel 32a durch ein nass- oder trockenchemisches Ätzverfahren ausgebildet werden. Vor der Ätzung kann in einer zusätzlich aufgebrachten dünnen Schicht, welche eine geeignet hohe Selektivität gegenüber der Ätzung der Öffnungen hat, das gewünschte Gitter strukturiert werden. Für die Ätzung der Öffnungen 26 kann nun ein Ätzverfahren mit geeigneter hoher Isotropie bzw. lateraler Unterätzung gewählt werden, so dass es zur Unterätzung der Gitterstege 44 kommen kann. Beispielhaft kann das Gitter in einer Siliziumoxid- oder Nitridschicht hergestellt sein und die Deckel aus Silizium, welche dann mittels tiefenreaktiven Ionenätzen (Deep Reactive Ion Etching - DRIE) strukturiert werden können. Dieser Prozess kann so eingestellt werden, dass Unterätzungen in der Größenordnung von Mikrometern erreichbar sind. Alternativ kann beispielsweise eine nasschemische Ätzung mit Tetramethylammoniumhydroxid (TMAH) und/oder Kaliumhydroxid (KOH) bzw. Nitridsäure (Nitric Acid - HNA) ausgeführt werden.

Bei entsprechender trichterförmige Gestaltung der Öffnungen im unteren Deckel 32a und im oberen Deckel 32b kann so die Schallaustrittsfläche einen größeren Anteil der Chipfläche umfassen und ggf. verglichen mit MEMS-Wandlern, die einen Austritt an einer Seitenfläche aufweisen, wie etwa der MEMS-Wandler 80, größer gestaltet werden. Diese Option bietet in Bezug auf die akustischen Eigenschaften und in Bezug auf die Dämpfung weiterer Gestaltungsspielraum. Eine Kombination von Schallaustrittsöffnungen in den Deckeln 32a und 32b und den Seitenflächen zwischen den Deckelflächen 32a und 32b ist ein Merkmal weiterer Ausführungsbeispiele. Eine bevorzugte Variante für hochintegrierte Systeme kann die Anbringung von Öffnungen im Deckel 32b umfassen, um den Schall nach oben abzugeben und die Anbringung der Druckausgleichsöffnungen an der Seite umfassen, um das Bauelement einfach, beispielsweise auf eine gedruckte Leiterplatte aufbringen zu können.

Generell können die Schalleintrittsöffnungen bzw. Schallaustrittsöffnungen 26 so gestaltet werden, dass die akustischen Eigenschaften und/oder die Dämpfungseigenschaften gezielt eingestellt werden. Die unteren und/oder oberen Schichten 32a und 32b können prinzipiell ebenfalls schwingungsfähig sein. Die Schwingung dieser Elemente kann durch geeignete zusätzliche Verbindungselemente in den dazwischenliegenden Schichten 34a und 34b bzw. 36 unterdrückt bzw. reduziert werden, etwa durch die Ankerelemente 84. Die Unterdrückung oder Reduzierung kann umfassen, die Schwingung in einen Frequenzbereich zu verschieben, der außerhalb des Hörschalls liegt. Alternativ oder zusätzlich kann die Schwingung der Schichten 32a und/oder 32b auch gezielt zur Optimierung der akustischen Abstrahlung implementiert sein, wobei ebenfalls gezielte Verbindungen in den Schichten einsetzbar sein können und zusätzlich die Steifigkeit bzw. die akustischen Eigenschaften der Schichten 32a und 32b durch entsprechende Strukturierung (durchgehende Öffnungen oder Sacklöcher) einstellbar sein können.

Außerdem ist es möglich, auf den oberen Deckel 32b eine Membran aufzubringen, welche dann durch den Volumenstrom 12 der Kammern zu einer Schwingung angeregt wird. Dies ist schematisch durch die gestrichelte Linie 104 angedeutet. In einem einfachen Fall kann dazu auf dem oberen Deckel 32b ein Abstandshalter in Form eines Rahmens oder Spacers (Abstandshalter) 106 angeordnet sein, an dem die Membran 104 angeordnet oder aufgespannt sein kann. Die Herstellung einer solchen Membran 104 kann mit bekannten mikromechanischen Prozessen erfolgen. Alternativ kann die Membran 104 auch im Inneren der Kavität oder Teilkavität angeordnet sein und/oder lediglich eine oder einen Anteil der Öffnungen 26 bedecken.

Für manche der vorangehend beschriebenen Ausführungsbeispiele der MEMS-Wandler (etwa MEMS-Lautsprecher-Bauelemente) kann gelten, dass es Kammern gibt, welche unabhängig von einigen, mehreren oder allen anderen Kammern einen Teilvolumenstrom erzeugen können, etwa in Teilkavitäten oder Teilkavitätsabschnitten. Es können Kammern realisiert sein, welche aus in lateraler und/oder vertikaler Richtung zusammenhängenden Teilkammern bestehen (lateral siehe beispielsweise Fig. 10 und 11) (vertikal siehe beispielsweise Fig. 14), wobei Ausführungsbeispiele auch eine Kombination hiervon zeigen. Solche zusammenhängenden Teilkammern (etwa die Teilkavitätsabschnitte 94a und 94b) können genutzt werden, um einen von anderen Kammern oder Teilkammern unabhängigen oder abhängigen Teilvolumenstrom zu erzeugen. Ein Fall, in welchem eine Kammer (Teilkavität) einen Volumenstrom unabhängig voneinander erzeugen kann, kann als Mono-Kammer bezeichnet werden. Eine Kammer, die basierend auf mehreren Teilkammern (Teilkavitätsabschnitte) einen Volumenstrom erzeugen kann, kann als Komposit-Kammer bezeichnet werden.

Vorangehend beschriebene Ausführungsbeispiele sind so modifizierbar, dass beide Arten von Kammern beliebig kombinierbar sind. Es sind also Ausführungsbeispiele möglich, bei welchen ausschließlich Mono-Kammern oder ausschließlich Komposit-Kammern angeordnet sind. Alternativ sind Ausführungsbeispiele realisierbar, bei denen beide Kammerarten angeordnet sind.

In andere Worten können bei Verwendung von ausschließlich Mono-Kammern die Resonanzfrequenzen aller Aktor-/Kammersysteme identisch sein oder auch unterschiedlich gestaltet werden. So können beispielsweise bestimmte Frequenzbereiche in der Schallabstrahlung durch eine vermehrte Anzahl entsprechender Mono-Kammern hervorgehoben werden. Insbesondere kann durch entsprechende Verteilung der Resonanzfrequenzen und die Breite der Resonanzkurven via der Dämpfung z. B. durch die Dimensionierung der Gitteröffnungen oder allgemein der Schallaustrittsöffnungen bzw. der Strömungskanäle eine Gestaltung des Frequenzverlaufs (Schalldruckpegel als Funktion der Frequenz) erreicht werden. Vor allem die Glättung des Frequenzverlaufs spielt dabei eine wesentliche Rolle.

Teilkavitäten und/oder Teilkavitätsabschnitte können basierend auf räumlichen Ausdehnungen der Volumina, einer Geometrie der elektromechanischen Wandler und/oder einer Frequenz mit der die elektromechanischen Wandler betrieben werden, den Volumenstrom mit unterschiedlicher Frequenz aussenden und/oder auf die Erfassung bestimmter Frequenzen des Volumenstroms optimiert sein.

In einem weiteren Ausführungsbeispiel sind ausschließlich Mono-Kammern verwendet. Die Schallaustrittsöffnungen können ausschließlich seitlich angeordnet sein. Drei Chips/Scheiben (MEMS-Wandler) können übereinander gestapelt sein. Der obere Chip kann für eine Schallabstrahlung in einem ersten (etwa hohen Frequenzbereich) optimiert sein. Ein zweiter, etwa mittlerer, MEMS-Wandler kann einen zweiten Frequenzbereich (etwa mittlere Frequenzen) angepasst sein. Ein dritter MEMS-Wandler kann für einen dritten Frequenzbereich angepasst sein, etwa für tiefe Frequenzen. Damit kann ein Drei-Wege-Lautsprecher erhalten werden. Die Anordnung der drei Kanäle (drei MEMS-Wandler) könnte ebenso in einem Chip geschehen, indem lateral eine erste Anzahl $N_1$ von Kammern für die hohen Frequenzen, eine zweite Anzahl $N_2$ von Kammern für mittlere Frequenzen und eine dritte Anzahl $N_3$ für tiefe Frequenzen verwendet wird. Dieses Prinzip ist für ein N-Wegesystem in lateraler und bei Stapelung auch in vertikaler Richtung einfach erweiterbar. In einem weiteren Ausführungsbeispiel ist ein N-Wegesystem so ausgestaltet, dass der Schall über Fouriersynthese der entsprechenden Harmonischen mit den Frequenzen $N*f_1$ erzeugt werden, wobei $f_1$ die niedrigste Frequenz darstellt.

Das bedeutet, dass ein MEMS-Wandler mit zumindest einem weiteren MEMS-Wandler zu einem Stapel angeordnet sein kann, wobei ein Stapel bspw. durch eine Anordnung von zumindest zwei MEMS-Wandlern entlang einer lateralen Richtung (etwa der x-Richtung) und/oder einer Dickenrichtung (etwa der z-Richtung) erhalten werden kann. Alternativ können die MEMS-Wandler auch beabstandet von einander angeordnet sein. Die Kavität des MEMS-Wandlers und die Kavität des zumindest einem weiteren (zweiten) MEMS-Wandlers können eine von einander verschiedene Resonanzfrequenz aufweisen.

Bei einem Aktorbetrieb, d. h., die verformbaren Elemente werden aktiv verformt, kann ein N-Wege Lautsprecher erhalten werden, wobei N eine Anzahl von MEMS-Wandlern mit voneinander verschiedenen Resonanzfrequenzen bezeichnet. Bei einem Sensorbetrieb können bspw. voneinander verschiedene Frequenzbereiche des Volumenstroms mit verschiedenen MEMS-Wandlern erfasst werden. Dies ermöglicht bspw. eine Fouriersynthese des Volumenstroms. Bspw. kann die Steuervorrichtung 128 ausgebildet sein, um die Verformung der verformbaren Elemente eines oder mehrerer der elektromechanischen Wandler des MEMS-Wandlers und des weiteren MEMS-Wandlers zu erfassen. Die Steuervorrichtung kann ausgebildet sein, um eine Fouriersynthese (Fourieranalyse) basierend auf den elektrischen Signalen zu berechnen und ein Ergebnis auszugeben.

Die eben dargestellten Beispiele unter Verwendung von Mono-Kammern können ebenso bei Verwendung von Komposit-Kammern realisiert werden, wobei die einzelnen Teilkammern einer Komposit-Kammer identische Resonanzfrequenzen aufweisen.

Bei Verwendung von Komposit-Kammern können die zusammenhängenden Teilkammern aber auch unterschiedliche Frequenzen durch entsprechende Lage der Resonanzmaxima unterstützen. So könnten beispielsweise drei Teilkammern ein Drei-Wege-System darstellen. Der z. B. in der hinteren Teilkammer (erster Abschnitt entlang einer axialen Ausdehnung) tieffrequent modulierte Luftstrom würde in der mittleren Teilkammer (zweiter Abschnitt entlang einer axialen Ausdehnung) zusätzlich eine mittelfrequente und im vorderen Teil der Kammer (dritter Abschnitt entlang einer axialen Ausdehnung) zusätzlich eine hochfrequente Modulation erfahren.

Bei hohen Frequenzen kein ein erforderlicher Hub, also eine Auslenkung der elektromechanischen Wandler geringer sein, als bei tiefen Frequenzen, um den gleichen Schalldruck zu erzeugen. Die Kammern oder Teilkammern, welche für hohe Frequenzen eingesetzt werden, können damit mit geringerem Kammervolumen bzw. Abstand der die Kammer eingrenzenden aktorischen Seitenwände gestaltet werden.

Beim Betrieb kann zwischen Kammern gleicher Frequenz ein Phasenversatz über die Ansteuerung eingebracht werden, so dass die Wellenfront verkippt wird und nicht senkrecht zur Oberfläche austritt (Phased-Array).

In allen bisher und im Folgenden vorgestellten Varianten ist jede Kammer von mindestens einer zweiten Kammer umgeben, in welche zum Druckausgleich Luft einströmt, wenn in die erste Kammer Luft einströmt bzw. anders herum. Offensichtlich ist dies vor allem dann, wenn zwischen diesen Kammern keine Trennwände existieren, da ein Aktor bei seiner Bewegung das Volumen der einen Kammer vergrößert und dabei gleichzeitig das Volumen der anderen Kammer verringert bzw. anders herum.

Für den Einsatz z. B. als Lautsprecher in Hörgeräten oder In-Ear-Kopfhörern wird häufig die Außenluft (also die außerhalb des Ohres) nicht durch den Lautsprecher bewegt. Vielmehr wird lediglich durch die Schwingung z. B. einer Membran das Volumen im Ohrkanal periodisch variiert. Dies kann bei allen dargestellten und im Folgenden vorgestellten Varianten erfolgen, indem die entsprechenden Öffnungen, die bei den dargestellten Varianten entweder auf einer Chipoberseite, einer Chipunterseite oder an einer Chipseitenflächen liegen, verschlossen bleiben. Dazu ist lediglich an diesen Stellen auf die Strukturierung der Stabgitter zu verzichten.

Generell gilt und für alle Lautsprechereinsatzgebiete gilt, dass Stabgitter an bestimmten Stellen oder komplett auch durch eine geschlossene Membran ersetzt werden können. Damit wird die Partikelempfindlichkeit maximal reduziert und der Betrieb insbesondere auch in kontaminierenden bzw. korrosiven Gasen und Flüssigkeiten ermöglicht.

Im Folgenden werden Maßnahmen im Design und dem Betrieb der Biegeaktoren vorgestellt, welche zum Ziel haben den gewünschten Frequenzgang möglichst gut darstellen zu können.

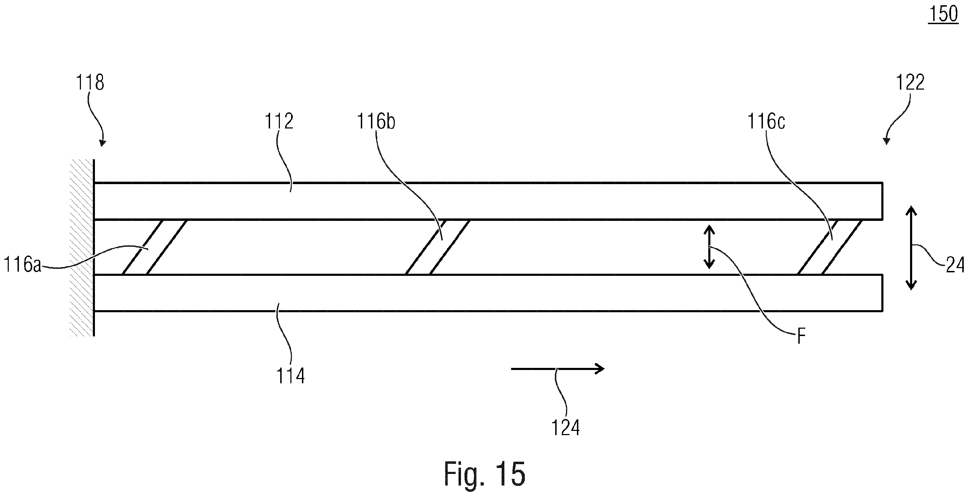

Durch Einbinden mehrerer zusätzlicher Federelemente, welche den Biegeaktor in einzelne Elemente einteilt, kann die effektive Steifigkeit der Aktoren und damit die Resonanzfrequenz verringert werden. Beispielhaft sei auf Abbildung 15 verwiesen, wo ein einzelnes Federelement eingesetzt wurde, um den Biegeaktor in zwei Elemente zu teilen. Die Einteilung in zwei oder mehr Elemente ist zum Erreichen einer Resonanzfrequenz im niedrigen Frequenzbereich des Hörschalls wichtig, da die Biegeaktoren ohne eine solche Maßnahme bei üblichen Dimensionen der Biegeaktoren (z. B. Breite 5 μm, Länge 2 mm, Material Silizium) Eigenfrequenzen im kHz-Bereich aufweisen. Alternativ oder zusätzlich kann gezielt ein zusätzliches Masseelement am Biegeaktor oder auch an der ggf. vorhandenen starren Platte vorgesehen werden, um die Resonanzfrequenz zu verringern. Ein solches Element kann in einfacher Weise bei der Strukturierung der Schicht 36 vorgesehen werden. Die Wirkungsweise einer zusätzlichen Masse $\Delta m$ kann an einem Modell des Harmonischen Oszillators erläutert werden.

Die Schwingungsamplitude $A(\omega)$ eines Elements der Masse $m$, das über eine Feder der Steifigkeit $k$ aufgehängt ist, ist bei sinusförmiger Anregung mit einer Kraft der Amplitude $F_0$ gegeben als:

$$A(\omega) = \frac{F_0}{m} \frac{1}{\sqrt{\left(\omega - \omega_0^2\right)^2 + \left(\frac{c}{m}\omega\right)^2}} \qquad \text{(Glg. 3)}$$

Dabei ist $\omega$ die Kreisfrequenz der Anregung und $c$ die Dämpfungskonstante. Wird der Resonator im quasistatischen Bereich betrieben, so ist die Amplitude unabhängig von der Masse. Es gilt für $\omega \ll \omega_0$:

$$A(\omega) \approx F_0 / k \qquad \text{(Glg. 4)}$$

Eine zusätzliche Masse $\Delta m$ ändert also die Eigenfrequenz $\omega_0$ auf den niedrigeren Wert $\omega_{0-}$, die Amplitude der Schwingung bleibt allerdings unverändert. Anders stellt sich die Situation dar, wenn der Biegeaktor im Bereich seiner Eigenfrequenz betrieben wird. Für $\omega \approx \omega_0$ kann der erste Term in der Wurzel von Glg. 3 gegen den zweiten Term vernachlässigt werden und es gilt:

$$A(\omega) \approx F_0 / (c\ \omega_{0-}) \qquad \text{(Glg. 5)}$$

Da $\omega_{0-}$ umgekehrt proportional zur Wurzel aus der Masse des Schwingers ist, bewirkt eine Erhöhung der Masse eine entsprechende Verringerung von $\omega_{0-}$ und somit eine Zunahme der Amplitude. Der Mehrgewinn an Amplitude ergibt sich unter der Bedingung c $\omega_{0-}$ < k. Weiter oben wurde bereits die Möglichkeit beschrieben, dass die Biegebalken so aufgebaut werden, dass sie sich je nach Adressierung bzw. Signal in die eine oder andere Richtung verbiegen können. Damit ist die Rückstellkraft nicht mehr notwendigerweise über die mechanische Federwirkung bei Verbiegung des Balkens aufzubringen. Umso geringer die Steifigkeit eines solchen Biegebalkens gewählt wird, umso größer ist bei fester einkoppelbarer Energie die Auslenkung.

Während alle Überlegungen sich auf den Hörschallbereich bezogen haben, ist es vorstellbar das Bauelement auch für die Generation von Ultraschall auszulegen. Prinzipiell ist es auch denkbar, dass statt Aktoren Balken mit positionssensorischen Elementen versehen werden (z. B. piezoresistiv, piezoelektrisch, kapazitiv etc.), um dann ein Bauelement als Mikrophon zur Verfügung zu stellen.

Für den Kern der Herstellung der MEMS-Lautsprecher in Siliziumtechnologie kann auf bekannte Waferbond-verfahren und tiefes reaktives Ionenätzen zurückgegriffen werden. Die Herstellung der Aktoren hängt vom gewählten Wirkprinzip ab und wird zunächst ausgeblendet. Dieser Teil kann modular in den folgenden beispielhaften Ablauf eingebunden werden. Die folgende Darstellung bezieht sich auf ein Bauelement mit ausschließlich seitlichen Öffnungen für den Luftstrom.

Als Ausgangsmaterial werden BSOI (Bonded Silicon on Insulator, verbundenes Silizium auf einem Isolator) Scheiben eingesetzt. Die Trägerscheibe (Handle-Wafer) bildet den unteren Deckel 32a des MEMS-Lautsprecher-Bauelements. Die vergrabene Oxidschicht der BSOI-Scheibe kann später als Abstandsschicht 34a fungieren. Die aktive Schicht der BSOI-Scheibe kann der Schicht 36 entsprechen. Die Trägerscheibe kann eine Dicke von 500 bis 700 $\mu$m aufweisen und kann bei Bedarf - evtl. am Ende des Prozesses - weiter abgedünnt werden. Die vergrabene Oxidschicht kann eine Dicke von 50 nm bis 1 $\mu$m aufweisen. Die aktive Schicht der BSOI-Scheibe kann eine Dicke von 1 bis 300 $\mu$m aufweisen. Die Schicht 36 wird bspw. vorzugsweise mit tiefem reaktiven Ionenätzen (DRIE) strukturiert. Nach dieser Strukturierung kann die vergrabene Oxidschicht (34a) mindestens lokal im Bewegungsbereich der Aktoren entfernt oder wenigstens abgedünnt werden. Dies kann nasschemisch, z. B. mit BOE (Buffered Oxide Etch - gepufferte HF-Lösung) oder trockenchemisch, z. B. mittels gasförmiger HF (Flusssäure), erfolgen. Nach zumindest teilweiser Entfernung der Abstandsschicht 34a im Bewegungsbereich der Aktoren kann z. B. über eine Gasphasenabscheidung (chemische Gasphasenabscheidung, chemical vapour deposition - CVD oder Atomlagenabscheidung, atomic layer deposition - ALD) eine reibungsarme Schicht abgeschieden werden, welche den Spalt zwischen der Schicht 34a und den Aktoren (verformbaren Elementen) verschließt bzw. stark verringert. Alternativ können schon bei der Bondung der Scheiben für die Herstellung der BSOI-Scheiben durch Abscheidung und Strukturierung geeigneter Schichten Bereiche definiert werden, in denen keine Bondung erfolgt, wie es bspw. in US 7,803,281 B2 beschrieben ist. Ein solches Verfahren kann für oberen und unteren Deckel eingesetzt werden. Die Schicht 34b wird bspw. vorzugsweise mittels reaktivem Ionenätzen (RIE) strukturiert. Mit diesen beiden Strukturierungen sind alle Elemente in der Schicht 36 und 34b wie in den entsprechenden Abbildungen dargestellt, hergestellt. Dies schließt auch die stabförmige Gitterstruktur ein.

Die vorab beschriebene Abscheidung einer reibungsarmen Schicht kann auch für den oberen Deckel (Schicht 32b) eingesetzt werden. Diese wird dann z. B. auf den Deckel vor Bonding aufgebracht. Auf die Abstandsschicht 34b kann dann verzichtet werden. Bspw. kann eine reibungsarme Schicht durch Abscheidung eines Materials erhalten werden. Ein Reibungswert kann bspw. 10 %, 20 % oder 50 % geringer sein, als bei einem Material der Schichten 32a, 34a, 34b oder 32b.

Die Schicht 36 kann bei entsprechender Dotierung auch als elektrischer Leiter verwendet werden. Vor allem dann, wenn Aktoren mit unterschiedlichen Frequenzen angeregt werden sollen, ist eine vertikale elektrische Isolation in Schicht 36 vorteilhaft. Dies kann z. B. durch sogenannte gefüllte Gräben, wie in [8] beschrieben, erreicht werden. Auch die Verwendung von offenen Gräben zur elektrischen Isolation stellt eine Möglichkeit dar.

Auf eine zweite Scheibe, die als eine Siliziumscheibe mit einer typischen oder möglichen Dicke von 500 bis 700 $\mu$m gebildet sein kann und bspw. den oberen Deckel 32b bilden wird, wird eine Schicht aufgebracht und strukturiert. Diese Schicht entspricht der Abstandsschicht 34b. Die Dicke dieser Schicht entspricht vorzugsweise der der vergrabenen Oxidschicht. Als Material für die Abstandsschicht stehen alle Materialien zur Verfügung, welche das später zu erfolgende Bonden der zweiten Scheibe auf die BSOI-Scheibe ermöglicht. Beispielhaft ist hier genannt Siliziumoxid, vorzugsweise thermisches Oxid für das Direktbonden von Siliziumoxid auf Silizium. Alternativ kann für das Direktbonden auch Polysilizium eingesetzt werden. Eine weitere Alternative besteht darin in die zweite Scheibe geeignete Vertiefungen zu ätzen, so dass aus der Scheibe die Funktion sowohl des oberen Deckels 32b als auch die Funktion der Abstandsschicht 34b abgebildet wird. Auf diese Vertiefungen kann mindestens im Bereich der Aktorbewegung verzichtet werden, wenn die Scheibe an diesen Stellen mit einer geeignet reibungsarmen Schicht beschichtet wird, so dass auf den Abstand zwischen Aktor (beweglichem Element) und Deckel (Schichten 32a und/oder 32b) verzichtet werden kann. Auf eine weitere Schicht auf der zweiten Scheibe - abgesehen von Hilfsschichten (Maskierung) für die Strukturierung - kann dann abgesehen werden. Damit ist außerdem die Direktbondung von Silizium auf Silizium möglich.

Neben dem Direktbonden ist es auch möglich Klebebondverfahren einzusetzen, so dass dann die Abstandsschicht 34b aus einem polymeren Material (z. B. BCB) besteht. Denkbar, aus Gründen der nicht vorhandenen CMOS-Kompatibilität aber nicht bevorzugt, sind außerdem Au-Si eutektische Bondverfahren oder anodische Bondverfahren (Na-Ionen haltige Schichten).

Nach Bonden der beiden Scheiben ist der Kern der Herstellung im Scheibenverbund abgeschlossen. Nicht ausgeführt wurde die Herstellung der elektrischen Verdrahtung und Kontakte und eventuell erforderliche elektrische Isolationsstrukturen. Diese Elemente können durch bekannte Standardprozesse nach dem Stand der Technik zur Verfügung gestellt werden: Herstellung von Leitbahnen z. B. mittels Sputtern und Strukturierung von AlSiCu, vertikale Isolationen durch Abscheidung und Strukturierung von Oxiden, laterale Isolationen durch offene oder gefüllte Isolationsgräben, welche die Schicht 36 komplett durchdringen.

Die Vereinzelung der Bauelemente mit seitlich angebrachten Öffnungen erfordert insbesondere den Schutz der Stabgitter. Dies wird z. B. ermöglicht, indem das Bauelement innerhalb eines Rahmen mit diesem z. B. über vier dünne Stege verbunden ist. Dazu sind unterer Deckel 32a und oberer Deckel 32b, sowie die Schichten 34a, 36 und 36b entsprechend zu strukturieren. Für diese Strukturierung kommen vor allem anisotrope Ätzverfahren, wie z. B. TMAH, KOH und DRIE in Frage. Speziell für die Strukturierung entlang der Stabgitter ist die DRIE-Strukturierung der Schicht 36 die bevorzugte Variante. Zum Herauslösen der Bauelemente aus dem Scheibenverbund werden die Stege zerstört. Dies kann z. B. mechanisch oder mittels Laserbearbeitung erfolgen.

Auch ist es denkbar den unteren Deckel 32a für die Vereinzelung nicht zu strukturieren, sondern nur die Schichten 34a, 36, 34b und 32b. Speziell die Schicht 36 kann mittels DRIE strukturiert werden, um den senkrechten Verlauf der Stabgitter zu realisieren. Von der Chipoberfläche aus ergibt sich dann ein Graben, welcher auf dem unteren Deckel 32b endet. Dieser Graben kann jetzt mit einem polymeren Material (z. B. Photolack) gefüllt werden. Das Polymer dient zum Schutz vor Verschmutzung beim anschließenden Säge-Vereinzelungsprozess. Nach dem Sägen werden die Bauelemente gespült und gereinigt, um den Sägeschlamm zu entfernen. Anschließend wird das Polymer durch geeignete Lösungsmittel oder in einem Sauerstoffplasma entfernt.

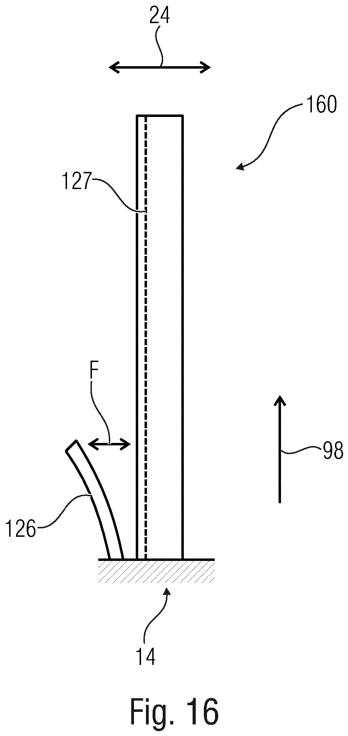

Werden statt der seitlichen Öffnungen Öffnungen im unteren und oberen Deckel verwendet, so ist die Herstellung zu erweitern, wie bereits im Kontext der Abbildung 16 beschrieben. Für die Vereinzelung können untere und obere Öffnung z. B. durch eine Folie geschützt werden, sodass Sägeprozesse oder Laserschneiden möglich sind. Alternativ können die Öffnungen auch durch ein polymeres Material, z. B. Photolack, für den Vereinzelungsprozess verschlossen werden und im Anschluss durch ein Lösungsmittel oder im Sauerstoffplasma wieder entfernt werden.

Die Stapelung von Bauelementen erfolgt vorzugsweise im Scheibenverbund durch Bondverfahren. Die elektrische Kontaktierung kann dann entweder durch elektrische Kontakte (Bondpads) in der jeweiligen Schicht 36 erfolgen oder bei Verwendung von TSVs (Through-Silicon-Vias) auch über sogenannte Bumps auf der Chipunterseite. Zur elektrischen Verbindung der gestapelten Einzelchips können ebenfalls TSVs verwendet werden. Bei nicht gestapelten Chips können ebenfalls TSVs und Bumps eingesetzt werden.

Um eine höhere Stabilität der Stabgitter 54 zu erreichen, können die Abstandsschichten 34a und 34b im Bereich der Stabgitter unstrukturiert bleiben.

Im Folgenden sind bevorzugte Ausgestaltungsvarianten für die Herstellung der lateralen Biegeaktoren beschrieben.

Prinzipiell können bekannte elektrostatische, piezoelektrische, thermomechanische und elektrodynamische Wirkprinzipien für die Aktuation der Biegebalken eingesetzt werden.

Ein einfaches elektrostatisches Wirkprinzip kann für einen Teil der oben gezeigten Bauelementvarianten auch ohne aktive Biegebalken realisiert werden. Der MEMS-Wandler 50 kann so ausgeführt sein, dass starre Plattenelemente 62a und 62b als Kondensatorplatten ausgeführt sind oder Kondensatorplatten aufweisen, die sich aufgrund einer elektrischen Potentialdifferenz so weit aufeinander zu bewegen, bis die durch die dann als Biegefeder agierenden Elemente 64 eine entsprechende mechanische Gegenkraft aufweisen.

Alternativ können die Biegebalken über eine zusätzlich angeordnete, feste Gegenelektrode direkt ausgelenkt werden. Auch der Einsatz von Kammelektroden zur Erhöhung der Kräfte bzw. der Auslenkung ist denkbar.

Ein weiteres elektrostatisches Prinzip beruht auf dem Einsatz eines einseitig eingespannten Balkens, der an seiner Einspannung einen sehr geringen Abstand zu einer Elektrode hat und sich dieser Elektrodenabstand mit zunehmendem Abstand von der Einspannung vergrößert. Dabei kann der Abstand an der Einspannung Null betragen. Liegt zwischen dem Biegebalken und der Elektrode eine elektrische Spannung an, so schmiegt sich ein durch die Höhe der elektrischen Spannung und die Steifigkeit des Balkens bestimmter Teil des Biegebalkens an die Elektrode. Der Raum zwischen Balken und Elektrode bildet bezogen auf das hier beschriebene Prinzip die Kammer 42a welche in ihrem Volumen wie beschrieben verändert werden kann.

Ein Grundprinzip derartiger Aktuatoren ist bspw. in der Literatur beschrieben. In [9] werden beispielsweise vertikal auslenkende Aktoren vorgestellt. Die Variation des Elektrodenabstands wird durch gezielte Einbringung von Schichtspannungen bei der Herstellung der Biegebalken realisiert. Für das im Rahmen dieser Anmeldung beschriebene Bauelement könnten Aktoren nach diesem Prinzip leicht durch entsprechende Strukturierung der Schicht 36 realisiert werden. Zusätzlich zu der ohnehin erforderlichen Strukturierung der Schicht 36 ist eine Isolationsschicht zwischen die Elektrode und den Biegebalken aufzubringen, was durch bekannte Verfahren der Mikrosystemtechnik einfach umzusetzen ist. Das Einbringen einer Schichtspannung ist nicht erforderlich, da die Biegebalken bereits durch die Strukturierung die gewünschte Form erhalten. In der hier beschriebenen Art sind die Aktoren lateral auslenkbar und damit für das weiter oben beschriebene Bauelementprinzip einsetzbar.

Bezüglich Integration und Skalierbarkeit für hohe Stückzahlen bietet das elektrostatische Wirkprinzip eine hohe Anzahl von Vorteilen. Es werden keine externen Komponenten, wie Magnete oder Spulen benötigt und es sind keine für Reinräume und insbesondere CMOSkompatible Reinräume kontaminationskritische Materialien erforderlich. Der bis dato verfolgte Membranansatz weist jedoch einige Nachteile auf. Dazu zählt, dass mit einer einzelnen schwingenden Membran oder Platte der gesamte Hörschallbereich nur ungenügend abgedeckt werden kann. Der Ansatz die Membran oder die Membranen quasistatisch zu betreiben löst dieses Problem allerdings aufgrund der fehlenden Resonanzüberhöhung zu Lasten der Auslenkung und damit zu Lasten des erreichbaren Volumenstroms bzw. des erreichbaren Schallpegels. Letztere hängen für ein festes Volumen, wie z. B. für In-Ear-Kopfhörer wie folgt zusammen [11]:

$$SPL = 20log_{10}\left(-1.4\frac{P_0 \Delta V}{P_{ref}V_0}\right) \quad \text{(Glg. 6)}$$

SPL steht dabei für "Sound Pressure Level" (Schalldruck), $P_0$ ist der Normaldruck, $\Delta V$ ist die erzielbare Volumenänderung durch den Lautsprecher, $P_{ref}$ ist der Referenzdruck, der ein Maß für die Hörschwelle angibt, er beträgt 20 µPa, $V_0$ ist im Fall von In-Ear-Kopfhörern oder Hörgeräten das Volumen der Ohrhöhle und entspricht etwa 2 cm³.

In Bezug auf MEMS-Lautsprecher ist es also erstrebenswert einen möglichst hohen Volumenstrom pro Chipfläche bzw. pro Volumen des gesamten Lautsprechers zu erreichen. Elektrodynamische Wandler können beispielsweise sehr hohe Membranauslenkungen erzielen und damit einen hohen Volumenstrom. Das Volumen des Gesamtaufbaus ist aufgrund der erforderlichen Permanentmagnete jedoch sehr groß. Für Lautsprecher in Mobiltelefonen, die perspektivisch in einer Dimension immer weniger Platz bieten, erscheint dieser Ansatz generell beschränkend.

Piezoelektrische Biegeaktuatoren erfordern die Abscheidung einer piezoelektrischen Schicht auf einem Substrat. Diese piezoelektrische Schicht könnte zum Beispiel der Schicht 58 aus Fig. 3 entsprechen, welche dann seitlich zur bspw. Silizium umfassende oder daraus bestehenden Schicht 56 angeordnet ist. Die Herstellung solcher Aktoren ist mit oberflächenmikromechanischen Prozessen möglich.

Laterale thermomechanische Aktoren in Form eines kalten und eines warmen Arms, wie z. B. in [10] beschrieben, können sehr einfach integriert werden, indem bei der weiter oben beschriebenen DRIE-Strukturierung der Schicht 36 die entsprechenden Geometrien berücksichtigt werden.

Eine weitere Variante für thermomechanische Aktoren besteht in der Verwendung von Bimorphen, welche durch elektrischen Strom erwärmt werden. Für die Herstellung eines solchen Bimorphs könnte beispielsweise nach Strukturierung der Schicht 36 eine Oxidschicht konform abgeschieden werden, so dass auch alle Seitenwände beschichtet werden. Diese Oxidschicht könnte dann überall außer an der einen Seitenwand des Biegeelements durch Maskierungs- und Ätzverfahren entfernt werden.

Der Einsatz eines elektrodynamischen Wirkprinzips ist einfach für die beidseitig eingespannten Biegebalken umsetzbar. Bei Stromfluss durch die Balken oder durch eine separat aufgebrachte Leiterstruktur erfahren die Balken in einem Magnetfeld eine Kraft, welche zur Auslenkung führt. Die Richtung des Stromflusses kann für die einzelnen Balken gemäß der gewünschten Auslenkungsrichtung gewählt werden. Die optionale Herstellung der Leiterbahnen erfolgt mit Standardprozessen der Oberflächenmikromechanik. Die zusätzliche Topographie ist in dem Fall bei der Wahl der Dicke der Abstandsschicht 34b zu berücksichtigen.

Die bevorzugte Ausführung für den Biegeaktor ist ein lateraler elektrostatischer Aktuator, welcher auf der Nutzung sehr geringer Elektrodenabstände beruht und damit bei geringen Spannungen arbeiten und betrieben werden kann. Solche lateralen Aktoren sind bspw. in EP 2 264058 A1 beschrieben. Diese Technologie erlaubt die Herstellung aller oben beschriebenen Biegeaktor- und Bauelementvarianten und kann in einfacher Weise modular in den oben beschriebenen Kernteil des Herstellungsprozesses der Bauelemente integriert werden.

Im Folgenden wird Bezug genommen auf die Umströmungsverluste bei der Bewegung der Seitenwände, d h., der verformbaren Elemente. Unter der Annahme laminarer Strömung kann in einem einfachen Modell gezeigt werden, dass die Umströmungsverluste, etwa Volumenströme von Kammer 42a zu Kammer 38a in Fig. 2a im Vergleich zu den Nutz-Volumenströmen, also der Volumenstrom, der nach außen dringt bzw. von außen nach innen dringt, geeignet gering gehalten werden können, wenn die Abstandsschichten 34a und 34b im Vergleich zur Dicke der Schicht 36 klein sind. Dasselbe gilt für den Abstand am gegebenenfalls freien Ende eines Biegebalkens zur lateral begrenzenden Struktur. Letzteres kann bei beidseitig eingespannten Biegeaktoren entfallen. Werden für diese Konfiguration im Modell einer laminaren Strömung durch rechteckige Rohre die Strömungsverluste berechnet, so kann sich bezogen auf den Nutz-Volumenstrom ein Verlust durch Umströmungen von etwa 3 % ergeben, wenn für die Dimensionen Folgendes angenommen wird:

Biegeaktor: Länge 1 mm, Höhe: 30 $\mu$m, Breite 10 $\mu$m

Kammer: Für die Berechnung des Strömungswiderstands nach außen wurde eine mittlere Breite von 50 $\mu$m angenommen. Dies unterschätzt den Strömungswiderstand bei großer Auslenkung der Biegeaktoren.

Schichtdicken der Abstandshalter 34a und 34b: je 0.5 $\mu$m

Die angenommenen Dimensionen sind lediglich beispielhaft zu verstehen und sehr gut mit mikromechanischen Technologien realisierbar. Die Annahme laminarer Strömung könnte aufgrund der geringen Breite der Aktoren (oben: 10 $\mu$m), welche der Rohrlänge entspricht, inkorrekt sein. Diese Annahme ist jedoch eine Worst-Case-Annahme, da beim Auftreten von Turbulenzen der Strömungswiderstand zunimmt. Um solche Turbulenzen zu motivieren können die Biegeaktoren in der Schicht 36 mit geeigneten lateral ausgebildeten Elementen versehen werden. Als geeignet sind Anordnungen anzusehen, welche bei Umströmung Wirbel bilden. Alternativ oder ergänzend kann eine bewusste Aufrauhung der zur Kammer zeigenden Fläche des Deckels 32a und 32b die Ausbildung einer turbulenten Strömung befördern.

Fig. 15 zeigt eine schematische Seitenschnittansicht eines verformbaren Elements 150, das eine erste Schicht 112 und eine zweite Schicht 114 aufweist, die über Verbindungselemente 116 miteinander beabstandet und verbunden sind, wobei die Verbindungselemente 116a-c mit einem Winkel von $\neq$ 90° zu der Schicht 114 und der Schicht 112 angeordnet sind. Beispielsweise können die Schichten 112 und 114 eine Elektrode aufweisen. Alternativ kann jeweils eine Elektrode an den Schichten 112 und/oder 114 angeordnet sein. Basierend auf einem Anlegen eines elektrischen Potentials kann eine abstoßende oder anziehende Kraft zwischen den Schichten 112 und 114 erzeugt werden. Die anziehende oder abstoßende Kraft kann zu einer Verformung der Elemente 116a-c führen, so dass ein von einem eingespannten Ende 118 abgewandtes auslenkbares Ende 122 des verformbaren Elements 144 entlang der lateralen Bewegungsrichtung 24 auslenkbar ist.

Das bedeutet, dass das verformbare Element 150 eine erste Schicht 114 und eine zweite Schicht 116 aufweisen kann, wobei zwischen der ersten Schicht 114 und der zweiten Schicht 116 Abstandshalter 116a-c angeordnet sein können. Die Abstandshalter 116a-c können in eine Neigungsrichtung 124 schräg zu einem Verlauf der Schichten 112 und 114 angeordnet sein. Eine Anziehungskraft zwischen den Schichten 112 und 114 kann eine Biegung des verformbaren Elements 150 bewirken.

Das verformbare Element 150 kann entlang der Neigungsrichtung eben oder einfach gekrümmt ausgebildet sein. Alternativ kann das verformbare Element bzw. die Schichten 112 und/oder 114 auch zumindest zwei diskontinuierlich aneinander angeordnete Abschnitte aufweisen, etwa einem Sägezahnmuster folgend.

Fig. 16 zeigt eine schematische Aufsicht auf ein verformbares Element 160, das benachbart zu einer Elektrode 126 angeordnet ist. Das verformbare Element 160 kann eine weitere Elektrode 127 aufweisen oder die weitere Elektrode 127 sein. Basierend auf einem angelegten elektrischen Potential zwischen der Elektrode 126 und der weiteren Elektrode 127 des verformbaren Elements 160 kann eine elektrostatische oder elektrodynamische Kraft F erzeugt werden. Basierend auf der elektrostatischen oder elektrodynamischen Kraft F kann eine Verformung des verformbaren Elements 160 bewirkt werden.

In einem von dem Volumenstrom oder dem elektrischen Potential, d. h. der Kraft F, unbeeinflussten Zustand des verformbaren Elements 160 kann ein Abstand zwischen dem verformbaren Element 160 und der Elektrode 126 entlang der axialen Ausdehnungsrichtung 98 des verformbaren Elements veränderlich sein. In einem Bereich, an dem der mechanische Wandler bzw. das verformbare Element 160 eine Verbindung mit dem Substrat 14 aufweist, kann der Abstand minimal sein. Dies ermöglicht eine hohe Steuerbarkeit der Verformung des verformbaren Elements 160. Alternativ kann der Abstand zwischen der Elektrode 126 und dem verformbaren Element 160 entlang der Ausdehnungsrichtung 98 beliebig variabel oder konstant sein.

Elektromechanische Wandler können gemäß Ausführungsbeispielen als elektrostatische Wandler, als piezoelektrische Wandler, als elektromagnetische Wandler, als elektrodynamische Wandler, als thermomechanische Wandler oder als magnetostriktive Wandler gebildet sein.

Basierend auf einer erzeugbaren Kraft kann eine Verformung des verformbaren Elements bewirkt werden oder eine Verformung des verformbaren Elements feststellbar bzw. bestimmbar sein.

Anhand der folgenden Figuren werden einige vorteilhafte Ausführungen der elektronischen Schaltung erläutert.

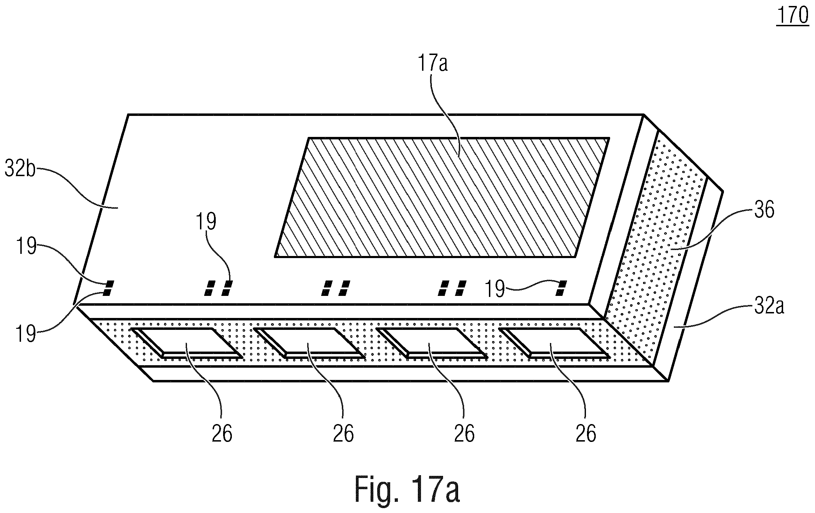

Fig. 17a zeigt eine schematische perspektivische Ansicht eines MEMS-Wandlers 170 gemäß einem Ausführungsbeispiel. Der Einfachheit halber umfasst die Schicht 36 auch die beispielsweise im Zusammenhang mit der Fig. 2a beschriebenen Schichten 34a und 34b. Die Kavität des MEMS-Wandlers 170 ist über Öffnungen 26 mit einer äußeren Umgebung des MEMS-Wandlers 170 verbunden. Die elektronische Schaltung 17 kann mehrteilig ausgeführt sein, so dass ein erster Teil 17a der elektronischen Schaltung 17 auf oder in der Schicht 32b angeordnet ist. Ein weiterer Teil 17b der elektronischen Schaltung 17 kann in einer anderen Schicht, etwa der Schicht 32a und/oder 36 angeordnet sein.

Der MEMS-Wandler 170 kann an oder in der Schicht 32b Durchkontaktierungen 19 aufweisen, die beispielsweise als Kontaktpins für die Eingabe und/oder Ausgabe elektrischer Signale an oder von der elektronischen Schaltung 17 dienen.

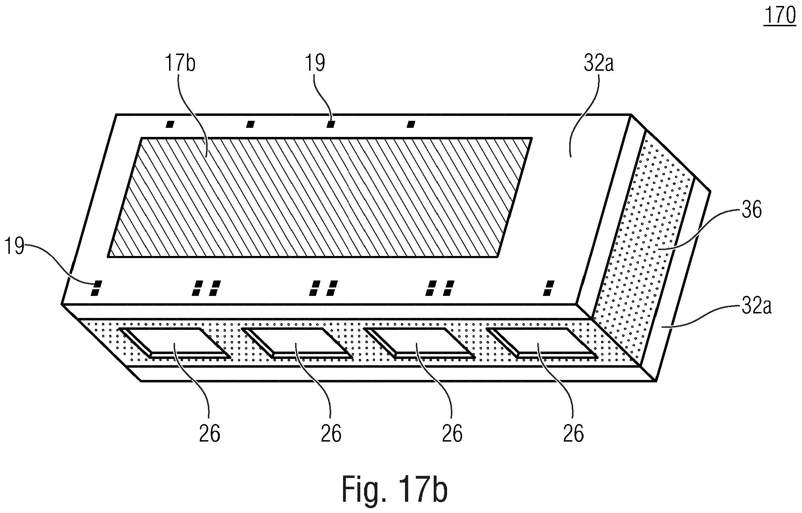

Fig. 17b zeigt eine weitere schematische Ansicht des MEMS-Wandlers 170, bei dem die Seite 32a im Vordergrund ist. Ein zweiter Teil 17b der elektronischen Schaltung 17 kann auf oder in der Schicht 32a angeordnet sein. Die Schicht 32a kann ebenfalls Durchkontaktierungen 19 aufweisen. Einige der Durchkontaktierungen können eine gleiche Funktionalität aufweisen. Die Durchkontaktierungen können bspw. für die Durchkontaktierung durch den gesamten Schichtstapel genutzt werden, eine, manche oder alle aber auch nur für Teile des Stapels, etwa bis zu den verformbaren Elementen. In einem Beispiel können also manche der Durchkontaktierungen 19 die Kontaktierungen zu den verformbaren Elementen des Wandlers darstellen und andere der Durchkontaktierungen 19 solche, die die elektronische Schaltung des Deckelwafers mit der elektronischen Schaltung auf dem Bodenwafer verbinden.

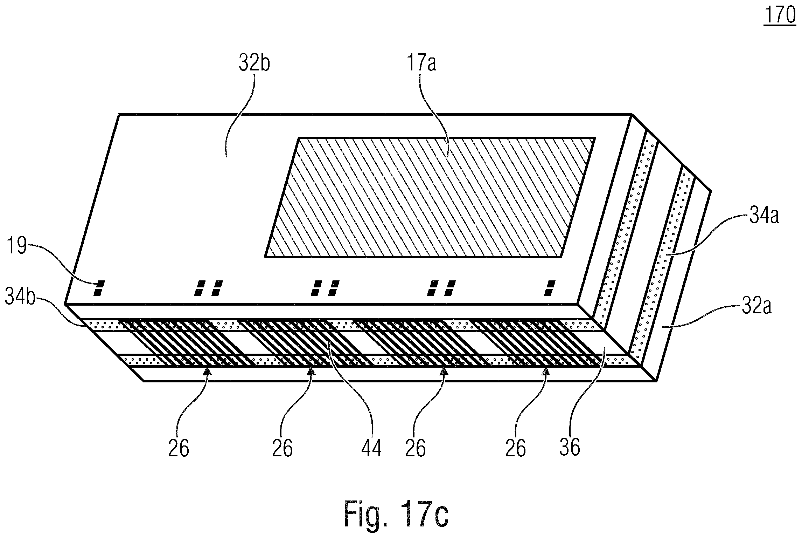

Fig. 17c zeigt eine schematische perspektivische Ansicht des MEMS-Wandlers 170 analog der Ansicht in Fig. 17a, wobei die Öffnungen so ausgestaltet sind, dass Gitterstege 44 angeordnet sind, um einen Eintritt von Partikeln zumindest zu erschweren.

Die Anordnung der elektronischen Schaltung 17 in Schichten des MEMS-Wandlers, etwa des MEMS-Wandlers 170, ermöglicht zum einen den Erhalt einer hochintegrierten Struktur, die eine Verringerung oder Vermeidung von externen Schaltungsstrukturen ermöglicht, so dass die Gesamtvorrichtung klein ausgeführt werden kann. Ferner ermöglicht die Anordnung der elektronischen Schaltung in einer Schicht des Schichtstapels den Erhalt kurzer Signalwege, was sowohl für den Bereich der elektromagnetischen Verträglichkeit als auch für Zeitaspekte und Leistungsaspekte der Vorrichtung vorteilhaft ist.

Obwohl eine Anordnung der elektronischen Schaltung in lediglich einer der Schichten 32a oder 32b möglich ist, kann eine Aufteilung der elektronischen Schaltung und eine Anordnung derselben in oder an beiden Schichten 32a und 32b Vorteile dahin gehend bieten, dass benachbarte elektronische Komponenten über jeweils möglichst kurze Wege durch die elektronische Schaltung 17 angesteuert werden. Eine Aufteilung der elektronischen Schaltung 17 ermöglicht ferner, dass die elektronischen Schaltungen 17a und 17b unterschiedliche oder komplementäre Funktionen implementieren und/oder in komplementären Technologien realisiert sein, etwa MEMS und CMOS. Zwar können die elektronischen Schaltungen 17a und 17b miteinander und/oder mit dem elektromechanischen Wandler in elektrischer Verbindung stehen. Die Struktur des MEMS-Wandlers 170 als Schichtaufbau ermöglicht es, die einzelnen Schichten getrennt voneinander zu fertigen, etwa in voneinander verschiedenen Halbleiterfertigungsprozessen. Dies ermöglicht die Implementierung unterschiedlicher von dem Halbleiterfertigungsprozess zumindest beeinflussten Funktionen in unterschiedlichen Schichten und mithin in unterschiedlichen elektronischen Schaltungen, so dass in einem Chip des MEMS-Wandlers 170 basierend auf unterschiedlichen Halbleiterfertigungsprozessen erhaltene Schaltungsstrukturen 17a und 17b angeordnet sein können.

Dies kann auch so verstanden werden, dass eine erste elektronische Schaltung 17a in einer ersten Deckelschicht des Schichtstapels angeordnet ist und dass eine zweite elektronische Schaltung 17b in einer zweiten Deckelschicht des Schichtstapels des Substrats angeordnet ist. Jede der beiden elektronischen Schaltungen 17a und 17b ist entlang einer Richtung senkrecht zu der in-plane-Bewegungsebene angeordnet. Das verformbare Element kann während einer Verformung desselben zumindest zeitweise zwischen der ersten und der zweiten elektronischen Schaltung 17a und 17b angeordnet sein. Durch Durchkontaktierungen und/oder andere Schaltungselemente kann die elektronische Schaltung 17 bzw. die Schicht, in der diese angeordnet ist, mit einer Außenseite des MEMS-Wandlers zumindest elektrisch verbunden sein oder die Außenseite des MEMS-Wandlers darstellen. Dies ermöglicht eine Kontaktierbarkeit des MEMS-Wandlers mit einer Leitungsanordnung, etwa auf einer Platine.

In anderen Worten ermöglichen Ausführungsbeispiele den Erhalt eines Gesamtbauelements umfassend unterschiedliche Funktionalitäten, die in dem MEMS-Wandler integriert sind. Dazu zählen z. B. die integrierte elektronische Ansteuerung eines MEMS-Lausprechers und die Ansteuerung/Auslese eines integrierten Beschleunigungssensors. Eine Herstellung einzelner Funktionselemente kann in unterschiedlichen Fertigungsprozessen verwendet oder implementiert werden, die gegebenenfalls während der Fertigung eines Wafers nicht oder schwer kombinierbar sind. Durch Aufteilung dieser Funktionalitäten auf zwei Schichten, etwa der obere und der untere Wafer 32a und 32b, können die Fertigungsverfahren mit größerer Flexibilität ausgewählt und auf Ebene der Funktionalität kombiniert werden, d. h. in dem MEMS-Wandler 170 zusammengefügt werden. Zum Beispiel kann der obere Wafer (Deckel) elektronische Schaltungselemente für den Niedervoltbereich enthalten, d. h. kleine elektronische Strukturen, die einen geringen Platzbedarf aufweisen und eine hohe Geschwindigkeit ermöglichen. Der untere Wafer (Boden) kann beispielsweise eine D/A-Wandlung für hohe Spannungen zum Betrieb eines Lautsprechers umfassen, d. h. große Transistorstrukturen mit hohem Platzbedarf, die langsam sind. Ebenso kann für Speicherelemente, Lichtquellen oder zusätzlich integrierte MEMS-Sensoren/Aktoren ein entsprechender Vorteil erhalten werden. Die Integration komplementärer Funktionalität kann beliebig unterteilt werden.

In nochmals anderen Worten kann in den Schichten 32a und/oder 32b eine integrierte elektronische Schaltung 17 bzw. 17a und/oder 17b realisiert sein. Diese kann beispielsweise dazu dienen, die Aktoren für die Schallerzeugung anzusteuern und die jeweilige elektrische Spannung gemäß dem gewünschten Schalldruck einzustellen, sowie die elektrische Anregung bei der gewünschten Frequenz zu realisieren. Auch eine gegebenenfalls erforderliche Wandlung eines digitalen elektrischen Eingangssignals in ein analoges Ansteuersignal ist mit der integrierten Schaltung, etwa in Form eines D/A-Wandlers oder per Pulsweitenmodulation (PWM) möglich. Im Falle der Mikrofonfunktion kann in dieser Schaltung beispielsweise die elektrische Signal(vor)verarbeitung - unter Beibehaltung kurzer Signalwege - oder auch eine A/D-Wandlung realisiert werden. Für die elektrische Kontaktierung der integrierten elektronischen Schaltung 17 mit den Aktoren/Sensoren im Chipinneren sind Durchkontaktierungen 19 vorstellbar, die in der Schicht 32a und/oder 32b angeordnet sein können. Diese können die Schicht 34a bzw. 34b durchdringen, so dass individuelle durchgängige elektrische Verbindungen von der integrierten Schaltung bis zu den einzelnen Aktoren (Balken) implementiert sein können. Für jeden Balken können beispielsweise zwei elektrische Kontakte vorgesehen sein. Auf einer Seite des Stapels können die Durchkontaktierungen 19 über integrierte Leiterbahnen mit der elektronischen Schaltung 17 verbunden sein, was in den Figuren nicht dargestellt ist.

Herstellungsbezogen wird beispielsweise eine Silizium-CMOS-Scheibe (CMOS = komplementärer Metalloxidhalbleiter) auf die Schicht 34b gebondet. Diese Scheibe bildet chipbezogen die Schicht 32b und schließt die Kammern (Kavität) nach oben hin ab. Die Durchkontaktierungen in der Silizium-CMOS-Scheibe können bereits vor dem Bonden hergestellt sein (Fall a). In diesem Fall kann es erforderlich sein, dass auch die Durchkontaktierungen der Schicht 34b bereits vor dem Bonden vorhanden sind. Alternativ ist es auch möglich (Fall b), dass beide Durchkontaktierungen erst nach dem Bonden hergestellt werden. Vereinfacht dargestellt kann dazu nach dem Bonden an den entsprechenden Stellen unter Zuhilfenahme einer Maskierung eine Ätzung bis zu den elektrisch zu kontaktierenden Stellen der Balken durchgeführt werden und das so entstandene Sackloch mit einem elektrisch leitenden Material, etwa ein Metall oder dotiertes Halbleitermaterial, wieder aufgefüllt werden. Gegebenenfalls können die Seitenwände des Sacklochs vor Füllung mit dem elektrisch leitfähigen Material mit einer isolierenden Schicht belegt werden, etwa Siliziumoxid und/oder Siliziumnitrid.

Obwohl die Figuren 17a bis 17c so dargestellt sind, dass die elektronische Schaltung in zwei Schichten angeordnet ist, die die Kavität 14 umschließen, kann die elektronische Schaltung auch lediglich an einer Schicht angeordnet sein, etwa an einer Schicht, die dazu konfiguriert ist, mit einer Platine oder dergleichen in Verbindung gebracht zu werden, das bedeutet, eine Bodenschicht. Alternativ oder zusätzlich können zusätzliche funktionale Elemente in zumindest einer dieser Schichten realisiert sein, wobei diese Schicht dann Durchkontaktierungen aufweisen kann. Alternativ oder zusätzlich zu den Anordnungen von Durchkontaktierungen mittels selektivem Ätzen kann auch eine Durchkontaktierung durch den gesamten Schichtstapel vorgesehen sein, etwa mittels sogenannter "TSV (Through-Silicon Via; Silizium-Durchkontaktierung), so dass funktionale Strukturen auf der Bodenschicht und der Deckelschicht elektrisch verbunden sein können. Es kann ein Herstellen der Abstandsschicht 34b auf der Rückseite des Deckelwafers, d. h. der Schicht 32b erfolgen, und diese gemeinsamen Schichten dann mit dem Wafer gebondet werden. Alternativ oder zusätzlich kann eine Abstandsschicht 34a auf der Schicht 32a hergestellt werden und dieser Schichtverbund durch anschließendes Bonden mit anderen Schichten zusammengefügt werden. Die jeweiligen aneinander hergestellten Schichten können vor dem Bonden mit anderen Bestandteilen kontaktiert werden. Prinzipiell können aber Durchkontaktierungen in den Schichten 32a und 32b bzw. daran angeordnete Schichten 34a und 34b an einer beliebigen Stelle des Prozessablaufs vorgesehen sein.

Alternativ oder zusätzlich kann es ebenfalls vorgesehen sein, dass ein MEMS-Wandler, wie vorhin beschrieben, so in der Kavität angeordnet wird, dass die Kavität aus einem vergleichsweise dicken Wafer herausgeformt wird, so dass auf zumindest einige der Schritte zur Durchführung eines Waferbondens verzichtet werden kann.

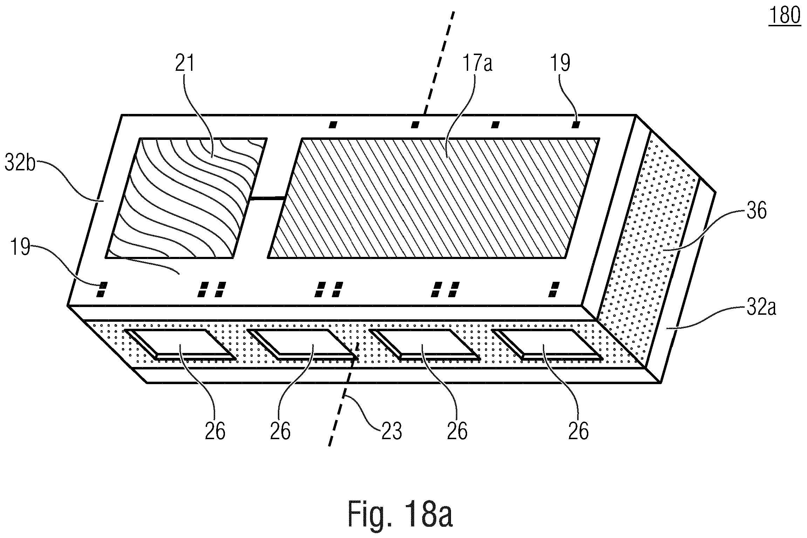

Fig. 18a zeigt eine schematische perspektivische Ansicht eines MEMS-Wandlers 180, bei dem die Schicht 32b dem Betrachter zugewandt ist, wie es beispielsweise in der Fig. 17a der Fall ist.

Benachbart zu der elektronischen Schaltung 17a kann die Schicht 32b ein Funktionselement oder MEMS-Struktur 21 aufweisen, die eine oder mehrere MEMS-Funktionen aufweisen kann. Beispielsweise kann die MEMS-Struktur 21 einen Inertialsensor, ein Magnetometer, einen Temperatur- und/oder Feuchtigkeitssensor, einen Gassensor oder eine Kombination daraus umfassen. Alternativ oder zusätzlich kann es sich um einen beliebigen Sensor, einen beliebigen Aktor, eine Drahtlos-Kommunikationsschnittstelle, eine Lichtquelle, einen Speicherbaustein, einen Prozessor und/oder einen Navigationsempfänger handeln. Die elektronische Schaltung 17a kann ausgebildet sein, um die MEMS-Struktur 21 anzusteuern und/oder auszuwerten.

Das bedeutet, dass die Schicht 32a, in der sich die elektronische Schaltung 17a befindet, ein Funktionselement, d. h. die MEMS-Struktur 21, umfassen kann. Das Funktionselement kann mit der elektronischen Schaltung verbunden sein, wobei die elektronische Schaltung 17a ausgebildet sein kann, um das Funktionselement anzusteuern oder auszuwerten.

Eine Drehung des MEMS-Wandlers 180 um eine Achse 23 kann zu einer Ansicht gemäß Fig. 17b führen, wobei die an der Seite 32a angeordnete Schaltung 17b beispielsweise ausgebildet sein kann, um die elektromechanischen Wandler des MEMS-Wandlers 180 im Inneren der Kavität auszuwerten und/oder anzusteuern. Auch hier können die elektronischen Schaltungen 17a und 17b komplementäre Funktionen aufweisen.

In anderen Worten kann der Bodenwafer eine integrierte elektronische Schaltung 17b aufweisen, die vorzugsweise komplementäre Aufgaben zu Schaltung 17a übernimmt. Das bedeutet, der MEMS-Wandler 180 kann konfiguriert sein, um so an einer Schaltungsstruktur oder Platine angeordnet zu werden, dass die MEMS-Struktur 21 dieser Struktur abgewandt angeordnet ist.

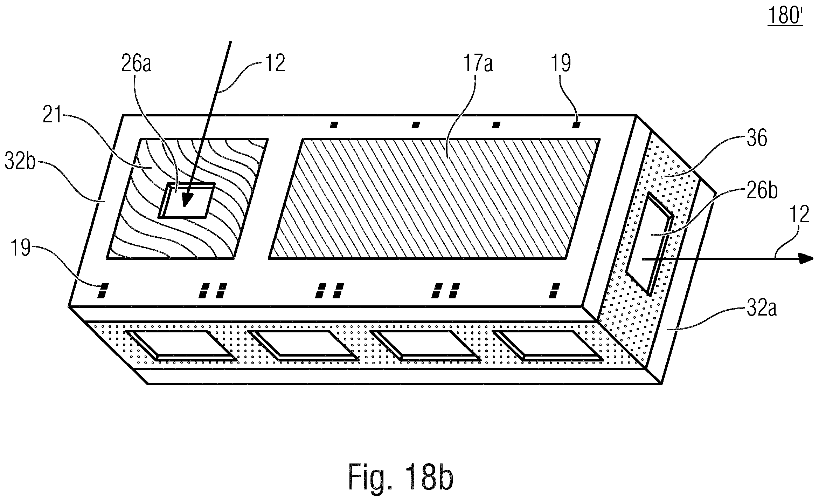

Fig. 18b zeigt eine schematische perspektivische Ansicht eines MEMS-Wandlers 180', der gegenüber dem MEMS-Wandler 180 dahin gehend modifiziert ist, dass eine Öffnung 26a in der Schicht 32b angeordnet ist und sich beispielsweise innerhalb des MEMS-Blocks 21 befindet.

Dies kann beispielsweise derart erfolgen, dass die elektronische Schaltung 17a sowohl ausgebildet ist, um die elektromechanischen Wandler im Inneren der Kavität anzusteuern und/oder auszulesen und ausgebildet ist, um die MEMS-Struktur 21 anzusteuern und/oder auszuwerten.

Der MEMS-Wandler 180' kann eine weitere Öffnung 26b in dem Substrat aufweisen. Die Öffnung 26b kann beispielsweise so angeordnet sein, dass der Fluidstrom 12 eine große Distanz durch den MEMS-Wandler 180 zurücklegt. Beispielsweise kann die Öffnung 26b an einer oder in einer der MEMS-Struktur 21 maximal entfernten Seitenwand des MEMS-Wandlers 180' angeordnet sein. Die MEMS-Struktur (Funktionselement) 21 kann beispielsweise als gassensorisches Funktionselement ausgebildet sein, das in der Deckelschicht 32b angeordnet ist. Das gassensorische Funktionselement 21 kann ausgebildet sein, um mit dem Fluidstrom 12 zu interagieren, wenn dieser die Öffnung 26a passiert und, um den Fluidstrom 12 sensorisch zu erfassen. Anders ausgedrückt, kann das Funktionselement 21 ausgebildet sein, um Eigenschaften des Fluidstroms 12 zu erfassen.

Die vorangehend erläuterte komplementäre Funktionalität zweier Teile der elektronischen Schaltung kann beispielsweise bezogen auf das Gassensorelement auf einer Seite des Stapels, etwa der Unterseite, und eine elektronische Ansteuerung auf einer gegenüberliegenden Seite des Stapels sein. Beispielsweise kann es sein, dass der Herstellungsprozess für das Gassensorelement, etwa gefertigt in MEMS-Technologie, nicht oder mit hohem Aufwand in den Herstellungsprozess für die elektronische Schaltung, etwa gefertigt in CMOS-Technologie, auf der Vorderseite integrierbar ist, so dass die Aufteilung der Funktionalitäten in komplementäre Paare die Verwendung von Standardherstellungsprozessen ermöglicht. Dies kann so erfolgen, dass einer dieser Standardprozesse genutzt wird, um die Ansteuerfunktion des Gassensors auf einem Wafer und die Ansteuerfunktion für die elektromechanischen Wandler in einem anderen Wafer zu implementieren, die später mittels Waferbonden mit einander zu dem MEMS-Wandler, etwa dem MEMS-Wandler 180, verbunden werden.

Beispielsweise kann das gassensorische Funktionselement 21 die Öffnung 26a umschlie-ßen, um einen möglichst großen Kontakt einer Oberfläche mit dem Fluidstrom 12 bereitzustellen. Alternativ oder zusätzlich ist es vorstellbar, dass das gassensorische Funktionselement zumindest teilweise in die Öffnung hineinragt, etwa wenn ein Element zur Messung einer Strömungsgeschwindigkeit oder dergleichen angeordnet ist.

In anderen Worten weist die Schicht 32b eine Öffnung auf. Eine zusätzliche Öffnung befindet sich im Schichtstapel auf der beispielsweise rechten Seite. Die fluidisch interagierenden Elemente in der Schicht 36, d. h. die verformbaren Elemente oder die Plattenelemente, sind in diesem Beispiel so ausgeführt, dass sie zusammen mit der Deckelschicht und mit der Bodenschicht 32a und 32b eine Mikropumpe ergeben. Zum Beispiel könnte über die Öffnung 26a die Umgebungsluft angesaugt und zur Öffnung 26b ausgestoßen werden. In dem Block 21 befindet sich beispielsweise ein Gassensor, der durch die Nähe zur Öffnung quasi-kontinuierlich mit Umgebungsluft versorgt wird. Dadurch kann der Sensor deutlich schneller auf Veränderungen in der Umgebungsluft reagieren, als in dem Fall, dass ein Gasaustausch nur basierend auf der Fusion erfolgt.

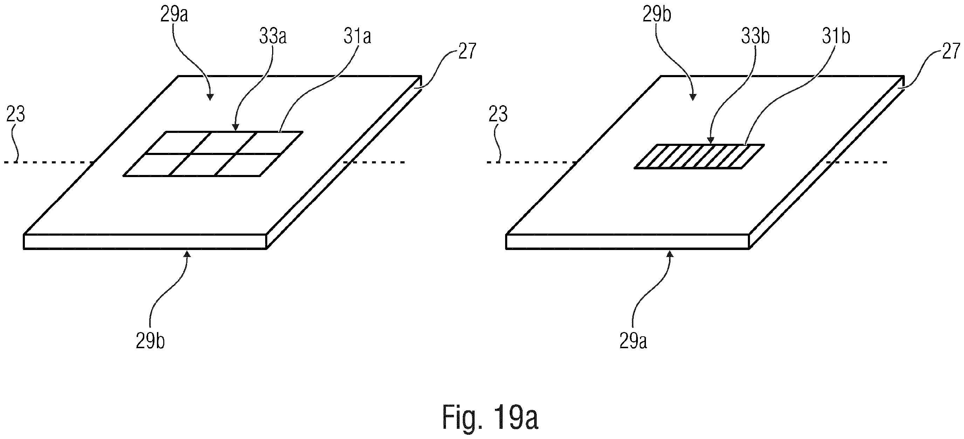

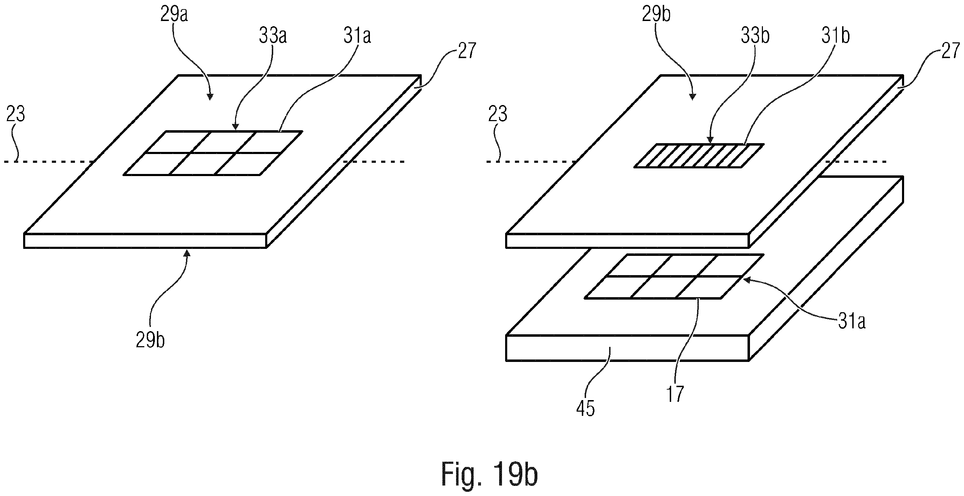

Fig. 19a zeigt eine schematische Ansicht einer Schicht 27, die Teil eines Schichtstapels eines MEMS-Wandlers gemäß Ausführungsbeispielen sein kann. Die Schicht 27 umfasst eine erste Hauptseite 29a und eine zweite Hauptseite 29b, wobei die linke Seite der Fig. 19 so dargestellt ist, dass die Hauptseite 29a sichtbar ist und in der rechten Seite der Fig. 19 durch Drehung der Schicht 27 um die Achse 23 die Seite 29b sichtbar ist. Die Seite 29a weist eine elektronische Struktur 31a auf, die ein erstes Abstandsraster 33a aufweist. Die zweite Seite 29b weist ebenfalls eine elektronische Struktur 31b auf, die ein hiervon verschiedenes Abstandsraster 33b aufweist. Die elektronischen Strukturen 31a und 31b können innerhalb der Adaptionsschicht 27 mit einander elektrisch verbunden sein, so dass durch die Adaptionsschicht 27 eine Anpassung oder Umsetzung des ersten Abstandsrasters 33a in das zweite Abstandsraster 33b und umgekehrt ermöglicht ist. Die Abstandsraster 33a und 33b können beispielsweise angepasst sein, um eine Kontaktierung einer jeweiligen Seite 29a oder 29b mit einer bestimmten Art von elektronischen Schaltungen zu vereinfachen oder zu ermöglichen. So kann beispielsweise eines der beiden Abstandsraster 33a oder 33b konfiguriert sein, um mit einer häufig genutzten oder Standard-Rasterung von Bauelementen oder Platinen kompatibel zu sein, während das andere Abstandsraster beispielsweise einen geringeren Strukturabstand aufweist, um eine kompakte Ausgestaltung von Schaltungsstrukturen in dem MEMS-Wandler zu ermöglichen. Alternativ hierzu können Abstände in dem Abstandsraster 33b auch größer sein als in dem Abstandsraster 33a.

Die Schicht 27 kann eine beliebige Schicht in dem Schichtstapel sein, beispielsweise eine Schicht, die eine Kontaktierung zu anderen Komponenten ermöglicht, beispielsweise die Schichten 32a und/oder 32b in Fig. 17a. Das bedeutet, dass die Schicht 27 eine Deckelschicht des Stapels sein kann.

Alternativ ist es ebenfalls möglich, dass die Schicht 27 ausschließlich als Adaptionsschicht zur Anpassung der Abstandsraster konfiguriert ist, das bedeutet, die elektronische Schaltung 17 nicht aufweist. Unabhängig hiervon kann die Schicht 27 eine Deckelschicht des Stapels sein, etwa wenn die elektronische Schaltung 17 nur an einer Seite des Stapels angeordnet ist.

Die Schicht 27 kann als Deckelschicht eines Stapels angeordnet werden, was eine Nutzung derselben Interposer d. h., Umsetzer ermöglicht. Das bedeutet, sie kann auch die Funktion eines Interposers erfüllen. Auf diese Schicht kann dann z. B. über Lötverfahren oder andere geeignete Verbindungsverfahren zur Herstellung der elektrischen Kontakte, ein weiterer Chip oder Schaltungsträger mit elektronischer und/oder sensorischer Funktionalität aufgebracht werden, der das entsprechende Abstandsraster aufweist, In Ausführungsbeispielen ist vorgesehen, dass auf diese Art, d. h., durch die zusätzlich kontaktierten Chips die ein Teil oder gar die gesamte elektronische Funktionalität für die Ansteuerung / Auslese des Wandlers realisiert wird bzw. alle zusätzlichen sensorischen Funktionalitäten zur Verfügung gestellt werden.

Durch MEMS-Technologie ist es möglich, das gassensorische Element nicht nur in der Nähe der Öffnung 26a zu platzieren, sondern gegebenenfalls auch direkt über der Öffnung, d. h., das gassensorische Element kann in die Öffnung hineinragen. Hierzu kann das sensorische Element in der Öffnung 26a aufgehängt werden. Eine Pumpe mit einer zuvor beschriebenen Funktionalität kann auch beispielsweise einen Teil des verfügbaren Chipvolumens nutzen. Damit kann ein anderer Teil, etwa ein für einen Lautsprecher und/oder ein Mikrofon vorgesehener Bereich hierfür genutzt werden. Auch kann das Chipvolumen oder ein Teil davon als Ultraschallwandler eingesetzt werden oder für eine andere hierin beschriebene Funktion.

Möglich ist der Einsatz der beschriebenen Technologie auch als Mikrodosiereinheit mit integrierter Sensorik und Signalverarbeitung. Beispielsweise kann ein Glukosesensor als MEMS-Element mit einer Mikropumpe integriert sein, die mit einem integrierten, in die Schicht 36 realisierten Reservoir verbunden ist. Sobald der Glukosesensor auf einen kritischen Blutzuckerspiegel schließen lässt, kann die Pumpe die notwendige Insulinmenge aus dem Reservoir abgeben. Ähnliches ist für andere Medikamente und/oder Wirkstoffe wie beispielsweise Schmerzmittel denkbar, etwa als Morphin- oder Hydromorphonpumpe.

In anderen Worten beruht der neuartige Ansatz auch darauf, dass die Schallaufnahme bzw. die Schallwiedergabe durch einen MEMS-Chip erfolgt, bei dem die fluidisch aktiven Elemente nicht an der Chipoberfläche, sondern im Inneren des Chips untergebracht sind. Dadurch bleiben die Flächen an der Ober- und Unterseite des Chips vollständig oder zumindest in großen Teilen verfügbar für die monolithische Integration weiterer Sensoren, Aktoren und elektronischer Schaltkreise. Die Darstellungen hierin beziehen sich im Wesentlichen auf den Einsatz dieses Prinzips. Die Erfindung beschränkt sich jedoch nicht darauf, sondern ist allgemein für alle MEMS-Lautsprecher und MEMS-Mikrophone einsetzbar, bei denen die Schallerzeugung bzw. -verarbeitung im inneren eines Chipvolumens erfolgt.

Fig. 19b zeigt eine vorteilhafte Verwendung der Adaptionsschicht aus Fig. 19a. Wie bereits erwähnt, kann die Adaptionsschicht als Halbleiterschicht ausgeführt sein, das bedeutet, Halbleitermaterialien umfassen, etwa Silizium oder Galliumarsenid. An der ersten Schichthauptseite 29a kann die Adaptionsschicht 27 die erste elektronische Struktur 31a mit dem ersten Abstandsraster 33a aufweisen. An der zweiten, gegenüberliegenden Schichthauptseite 29b kann die Adaptionsschicht 27 die zweite elektronische Struktur 31b mit einem zweiten Abstandsraster 33b aufweisen. Die erste und die zweite elektronische Struktur 31a und 31b sind mit einander elektrisch verbunden, so dass eine Kontaktierung einer elektrischen Schaltung, etwa der Schaltung 17 auf einer der Seiten 29a oder 29b eine Umsetzung des Abstandsrasters 33a oder 33b auf das andere Abstandsraster 33b oder 33a ermöglicht.

Die Adaptionsschicht 27 kann in einem MEMS-Schichtstapel angeordnet sein, etwa in einem hierin beschriebenen MEMS-Wandler. Der MEMS-Schichtstapel kann eine Schaltungsschicht 45 mit einer elektronischen Schaltung, etwa der elektronischen Schaltung 17, aufweisen, die eines der Abstandsraster 33a oder 33b aufweist. Die Schicht 45 kann bspw. die Schicht 32b oder eine andere Schicht mit einer elektronischen Schaltung sein. Die elektronische Schaltung 17 kann somit mit der ersten oder zweiten elektronischen Struktur 31a oder 31b elektrisch verbunden werden, so dass elektrische Schaltung 17 über die andere Schichthauptseite 29b und mithin mit einem anderen Abstandsraster kontaktierbar ist, weshalb die Schicht 29 auch als Interposer verwendet werden kann. Ein mit der Adaptionsschicht 27 verbundener Schichtstapel kann ein hierin beschriebener Schichtstapel in Abwesenheit der elektronischen Steuerung 17 sein. Der Schichtstapel kann mittels der Adaptionsschicht 27 mit dem Schichtstapel verbunden werden, um so eine Auswertung und/oder Ansteuerung der verformbaren Elemente vorzunehmen. Wird bspw. der MEMS-Wandler 20 betrachtet, so könnte anstelle der elektronischen Schaltung auch die elektronische Struktur zum Verbinden mit der elektronischen Schaltung angeordnet sein, das bedeutet, die Schicht 32b könnte als Adaptionsschicht gebildet sein.

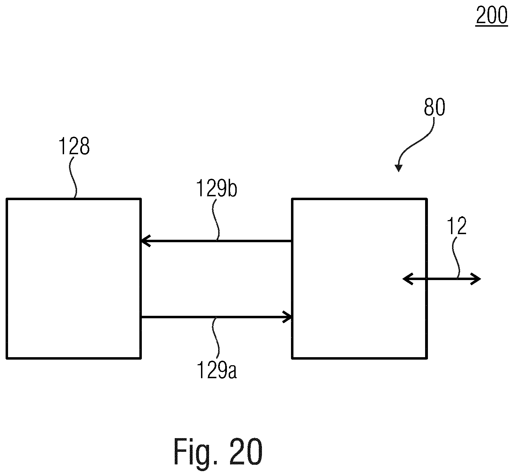

Fig. 20 zeigt ein schematisches Blockschaltbild eines MEMS-Systems 200, das den MEMS-Wandler 80 aufweist, der mit einer Steuervorrichtung 128 verbunden ist, die ausgebildet ist, um eine Verarbeitung der dem MEMS-Wandler 80 bereitzustellenden Signale und/oder der von dem MEMS-Wandler empfangenen Signale auszuführen. Bspw. kann die elektronische Schaltung den elektrodynamischen Wandler der MEMS-Vorrichtung 80 ansteuern und/oder elektrische Signale von den elektrodynamischen Wandlern der MEMS-Vorrichtung 80 empfangen. Informationen, wie eine entsprechende Ansteuerung zu erfolgen hat und/oder eine Auswertung kann in der Steuervorrichtung 128 erfolgen.

Weist der MEMS-Wandler 80 beispielsweise eine Vielzahl von elektromechanischen Wandler 18 auf, kann die Steuervorrichtung 128 ausgebildet sein, um Informationen für die Vielzahl von elektromechanischen Wandlern bereitzustellen, etwa für eine gemeinsame elektronische Schaltung und/oder oder für individuelle elektronische Schaltungen, so dass sich ein erster und ein benachbarter zweiter elektromechanischer Wandler während eines ersten Zeitintervalls zumindest lokal aufeinander zubewegen. Die Steuervorrichtung 128 kann ausgebildet sein, um die zumindest eine elektronische Schaltung einer Vielzahl von elektromechanischen Wandlern so anzusteuern, dass sich der erste elektromechanische Wandler und ein dritter elektromechanischer Wandler, der benachbart zu dem ersten elektromechanischen Wandler angeordnet ist während eines zweiten Intervalls aufeinander zubewegen, der erste elektromechanische Wandler kann zwischen dem zweiten und dem dritten elektromechanischen Wandler angeordnet sein. Beispielsweise kann es sich hier um die elektromechanischen Wandler 18a-c handeln, wobei der elektromechanische Wandler 18b der erste elektromechanische Wandler sein kann.

Zusätzlich kann die Steuervorrichtung 128 ausgebildet sein, um ein elektrisches Signal, das auf einer Verformung des verformbaren Elementes basiert, von der elektronischen Schaltung zu empfangen und auszuwerten. Bspw. kann die Steuervorrichtung 128 ausgebildet sein, um eine Frequenz oder eine Amplitude der Verformung zu bestimmen. Das bedeutet, das System 200 kann als Sensor und/oder Aktor betrieben werden.

Das System 200 kann beispielsweise als MEMS-Lautsprecher betrieben werden, wobei der Volumenstrom 12 eine akustische Schallwelle oder eine Ultraschallwelle sein kann.

Alternativ kann das System 200 als MEMS-Pumpe ausgeführt sein. Eine Kavität des Substrats kann eine erste Öffnung 26 und eine zweite Öffnung 26 in dem Substrat 14 aufweisen. Der elektromechanische Wandler 18 kann ausgebildet sein, um den Volumenstrom 12 basierend auf dem Fluid bereitzustellen. Der elektromechanische Wandler kann ausgebildet sein, um das Fluid basierend auf einer Aktuierung des elektromechanischen Wandlers 18 durch die erste Öffnung 26 in eine Richtung der Kavität zu befördern oder, um das Fluid basierend auf der Aktuierung durch die zweite Öffnung in eine Richtung weg von der Kavität zu befördern.

Alternativ kann das System 200 als MEMS-Mikrophon betrieben werden, wobei basierend auf der Verformung des verformbaren Elements ein elektrisches Signal an einem Anschluss des elektromechanischen Wandlers 80 oder eines anderen angeschlossenen elektromechanischen Wandlers erhaltbar ist. Basierend auf dem Volumenstrom 12 kann die Verformung des verformbaren Elements bewirkbar sein.

Alternativ oder zusätzlich können hierin beschriebene MEMS-Wandler als MEMS-Ventil oder MEMS-Dosiersystem, ausgeführt sein. Ein MEMS-Dosiersystem kann bspw. für implantierbare Medikamentation und/oder Insulinpumpen einsetzbar sein.

Obwohl das System 200 so beschrieben ist, dass die Steuervorrichtung 128 mit dem MEMS-Wandler 80 verbunden ist, kann auch ein anderer MEMS-Wandler angeordnet sein, etwa der MEMS-Wandler 10, 20, 50, 100, 110, 170, 180, 180', 230 und/oder 240. Alternativ oder zusätzlich können auch mehrere MEMS-Wandler gemäß vorangehend beschriebenen Ausführungsbeispielen angeordnet sein. Alternativ oder zusätzlich kann ein Stapel von MEMS-Wandler MEMS-Wandlern angeordnet sein, etwa der Stapel 90 oder 140. Alternativ oder zusätzlich können zumindest zwei MEMS-Wandler angeordnet sein. Zumindest ein erster MEMS-Wandler und ein zweiter MEMS-Wandler können Kavitäten oder Teilkavitäten und/oder elektromechanische Wandler mit voneinander verschiedenen Resonanzfrequenzen aufweisen, etwa eine Kammer mit 500 Hz-Aktoren, eine weitere Kammer oder eine weitere (Teil-)Kavität mit 2 kHz-Aktoren, etc.).

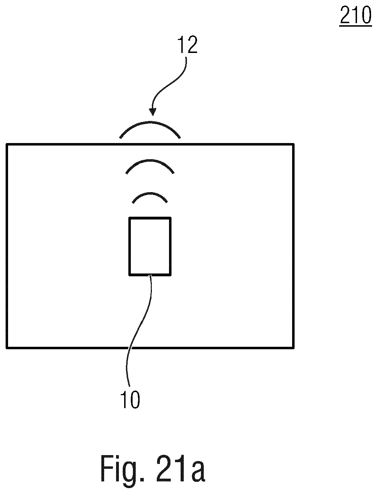

Die vorangehend erläuterten MEMS-Wandler können in Vorrichtungen eingesetzt werden. Ein Beispiel für eine solche Vorrichtung 210 gemäß einem Ausführungsbeispiel ist in Fig. 21a gezeigt. Die Vorrichtung 210 umfasst bspw. den MEMS-Wandler 10, kann aber alternativ oder zusätzlich auch einen anderen hierin beschriebenen MEMS-Wandler 20, 50, 100, 110, 170, 180, 180', 230 und/oder 240 umfassen. Der MEMS-Wandler ist bspw. als MEMS-Lautsprecher zu Erzeugung eines akustischen Fluidstroms 12 in Form von Schallwellen gebildet, wobei die Vorrichtung 210 als mobile Musikwiedergabevorrichtung oder als Kopfhörer ausgebildet ist.

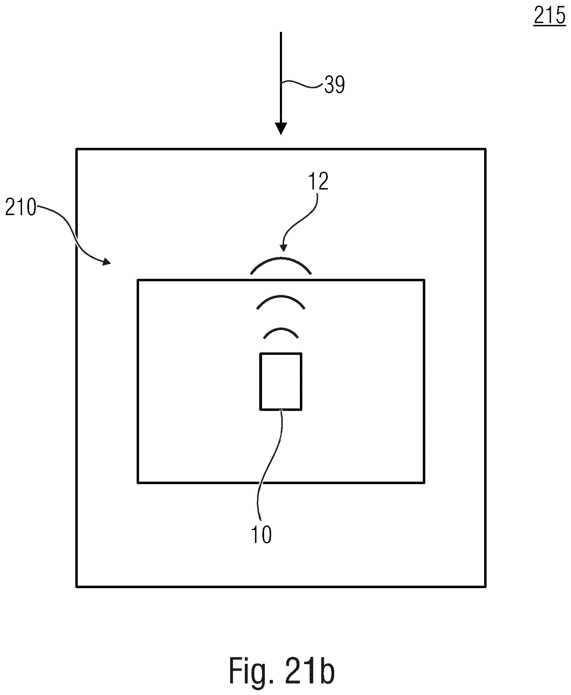

Fig. 21b zeigt ein schematisches Blockdiagramm eines Systems 215 gemäß einem Ausführungsbeispiel, das bspw. die Vorrichtung 210 umfassen kann. Der MEMS-Wandler 10 und/oder ein anderer angeordneter MEMS-Wandler kann als Lautsprecher ausgeführt sein und ausgebildet sein, um aufbauend auf einem Ausgabesignal 39 ein akustisches Signal in Form des Fluidstroms 12 wiederzugeben. Bei dem Ausgabesignal kann es sich um ein analoges oder digitales Signal handeln, das akustische Informationen, etwa umfassend Sprachinformationen aufweist. Das System 215 kann bspw. als Universalübersetzer und/oder als Navigationsassistenzsystem sowohl für indoor-Anwendungen (innerhalb von Gebäuden) als auch outdoor-Anwendungen (außerhalb von Gebäuden) ausgebildet sein. Hierfür kann das System 215 weitere Komponenten, etwa ein Mikrophon und/oder eine Vorrichtung zur Positionsbestimmung sowie eine Recheneinheit, etwa eine CPU aufweisen. Das Mikrophon kann ebenfalls als hierin beschriebener MEMS-Wandler gebildet sein. Die Recheneinheit kann ausgebildet sein, um einen Sprachinhalt in einer ersten Sprache, der mittels des Mikrophons erfasst wird, in eine zweite Sprache zu überführen, um das Ausgabesignal 39 mit der zweiten Sprache bereitzustellen.

Alternativ kann die Recheneinheit ausgebildet sein, um basierend auf einer bestimmten Position das Ausgabesignal 39 so bereitzustellen, dass es eine Sprachinformation bezüglich der ermittelten Position aufweist, was dann von dem MEMS-Wandler 10 wiedergegeben werden kann.

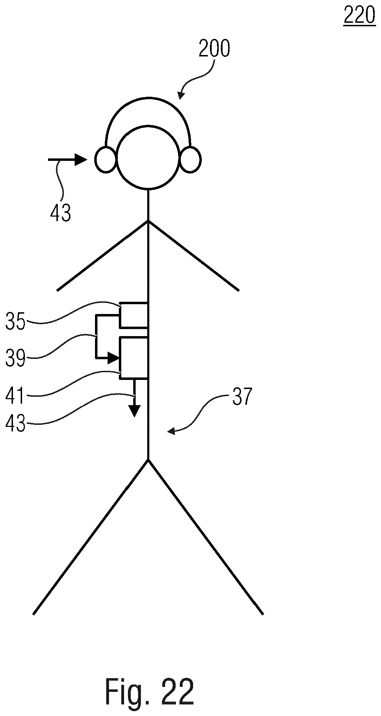

Fig. 22 zeigt eine schematische Darstellung eines Gesundheitsassistenzsystems 220 gemäß einem Ausführungsbeispiel. Das Gesundheitsassistenzsystem 220 umfasst eine Sensoreinrichtung 35 zum Erfassen einer Vitalfunktion eines Körpers 37 und zum Ausgeben eines Sensorsignals 39 basierend auf der erfassten Vitalfunktion. Das Gesundheitsassistenzsystem 220 umfasst eine Verarbeitungseinrichtung 41 zum Verarbeiten des Sensorsignals 39 und zu Bereitstellen eines Ausgabesignals basierend auf der Verarbeitung. Das Gesundheitsassistenzsystem 220 umfasst einen Kopfhörer, etwa die Vorrichtung 200 umfassend einen MEMS-Wandler gemäß einem der hierin beschriebenen Ausführungsbeispiele. Der MEMS-Wandler ist als Lautsprecher ausgeführt ist und umfasst eine Drahtlos-Kommunikationsschnittstelle zum Empfangen des Ausgabesignals 43. Der Lautsprecher 200 ist ausgebildet, um ein akustisches Signal basierend auf dem Ausgabesignal 43 wiederzugeben. Vorteile bietet eine Realisierung des Lautsprechers als Kopfhörer, insbesondere als In-Ear Kopfhörer. Bspw. kann die überwachte Vitalfunktion dem Benutzer angesagt werden, etwa ein Pulsschlag während dem Sport. Es ist auch möglich, dass eine aus der Vitalfunktion abgeleitete Größe ausgegeben wird, wie die Über- oder Unterschreitung eines Schwellwerts oder dergleichen.

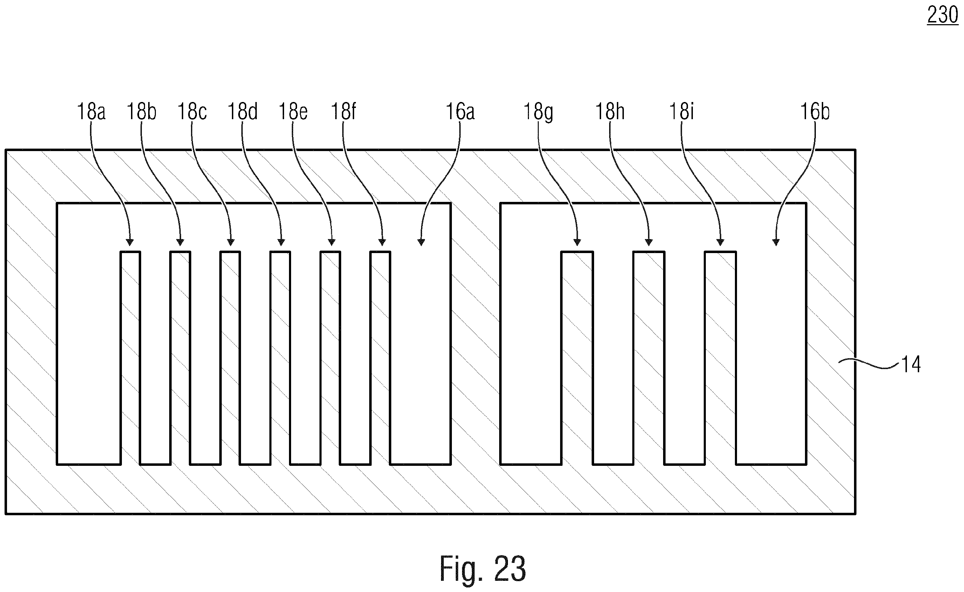

Fig. 23 zeigt eine schematische Aufsicht auf einen MEMS-Wandler 230, der eine Vielzahl von elektromechanischen Wandlern 18a bis 18i aufweist, wobei die elektromechanischen Wandler 18a bis 18f lateral nebeneinander versetzt zueinander in einer ersten Kavität 16a und die elektromechanischen Wandler 18g bis 18i lateral nebeneinander versetzt zueinander in einer zweiten Kavität 16b angeordnet sind. Die Kavitäten 16a und 16b können eine Öffnung in einer nicht dargestellten Boden- und/oder Deckelfläche des Substrats 14 aufweisen. Der MEMS-Wandler 230 kann als Lautsprecher und/oder Mikrofon einsetzbar sein, was sowohl für einzelne elektromechanische Wandler 18a bis 18i als auch für die elektromechanischen Wandler 18a bis 18f oder 18g bis 18i einer jeweiligen Kavität 16a und 16b gilt. Die Lautsprecher und/oder Mikrophone können auch so ausgestaltet werden, dass es für die Abgabe bzw. Aufnahme von Schallwellen über Vibrationen optimiert ist. Z. B. kann er mit dem menschlichen Körper, idealerweise nahe an einem Knochen, platziert werden, um mittels Körperschall Informationen zu übertragen bzw. aufzunehmen. In diesem Fall ist eine bevorzugte Variante die, in der sich alle Aktoren in jeweils die gleiche Richtung bewegen, das bedeutet unabhängig von einem Ansatz, dass eine Kammer zwei bewegliche Wände aufweist. Die elektromechanischen Wandler 18a bis 18i umfassen einseitig eingespannte Balkenelemente. Der MEMS-Wandler 230 umfasst die nicht dargestellte elektronische Schaltung 17.

In anderen Worten enthält die linke Kammer, Kavität 16a, lateral oder vertikal bewegliche Biegeaktoren, die vorzugsweise in Phase schwingen und so den Chip in Vibration versetzen, um damit Schall zu übertragen. Die rechte Kammer, Kavität 16b, enthält drei laterale oder vertikale Biegeaktoren, die ebenfalls vorzugsweise in Phase schwingen, die jedoch durch ihre Dimensionierung (Dicke, Länge oder Breite) einen anderen Frequenzbereich als die linke Kammer abbilden.

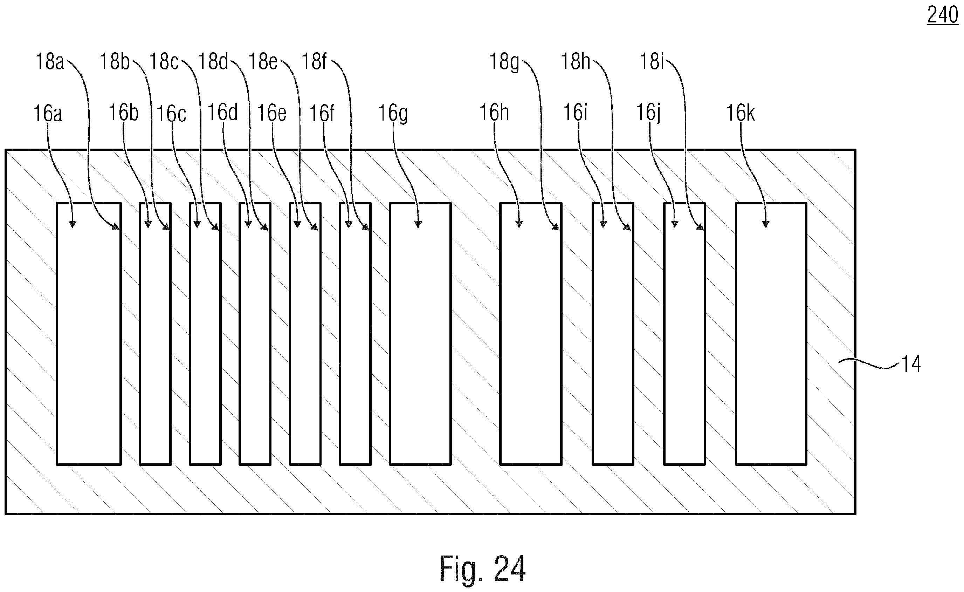

Fig. 24 zeigt eine schematische Aufsicht auf einen MEMS-Wandler 240, der eine Vielzahl von elektromechanischen Wandlern 18a bis 18i aufweist, wobei die elektromechanischen Wandler 18a bis 18f lateral nebeneinander versetzt zueinander angeordnet sind und jeweils benachbarte Kavitäten 16a bis 16k oder Teilkavitäten von einander beabstanden. Die elektromechanischen Wandler 18a bis 18i umfassen beidseitig eingespannte Balkenelemente. Der MEMS-Wandler 240 umfasst die nicht dargestellte elektronische Schaltung 17.

Obwohl die Ausführungsbeispiele der Fig. 23 und 24 so dargestellt sind, dass der MEMS-Wandler 230 ausschließlich einseitig eingespannte Balkenelemente aufweist und der MEMS-Wandler 240 ausschließlich beidseitig eingespannte Balkenelemente aufweist, sind die Ausführungsformen auch beliebig mit einander kombinierbar, so dass je Kavität 16a oder 16b unabhängig von einander gleichartige elektromechanische Wandler oder innerhalb einer Kavität verschiedenartige elektromechanische Wandler angeordnet sein können.

In anderen Worten zeigt Fig. 24 ein gleiches Prinzip wie in Fig. 23, jedoch sind diesmal beidseitig eingespannte Biegeaktoren eingesetzt.

Weitere Ausführungsbeispiele beziehen sich auf ein Verfahren zum Herstellen eines MEMS-Wandlers. Das Verfahren umfasst ein Bereitstellen eines Substrats, das einen Schichtstapel mit einer Mehrzahl von Schichten aufweist, die eine Mehrzahl von Substratebenen bilden, und das eine Kavität in dem Schichtstapel aufweist. Das Verfahren umfasst ein Erzeugen eines elektromechanischen Wandlers, in dem Substrat, so dass dieser mit dem Substrat in der Kavität verbunden ist und ein sich in zumindest einer Bewegungsebene der Mehrzahl von Substratebenen verformbares Element aufweist, wobei eine Verformung des verformbaren Elements in der Bewegungsebene und der Volumenstrom des Fluids kausal zusammenhängen. Das Verfahren umfasst ein Anordnen einer elektronischen Schaltung in einer Schicht des Schichtstapels, so dass die elektronische Schaltung mit dem elektromechanischen Wandler verbunden ist, und ausgebildet ist, um eine Konvertierung zwischen einer Verformung des verformbaren Elements und einem elektrischen Signal bereitzustellen.

Obwohl vorangehend beschriebene Ausführungsbeispiele sich darauf beziehen, dass der Volumenstrom erzeugbar ist, indem sich zwei elektromechanische Wandler aufeinander zubewegen, kann der Volumenstrom auch basierend oder in kausaler Wechselwirkung mit einer Bewegung eines elektromechanischen Wandlers gegenüber einer starren Struktur, beispielsweise dem Substrat, erhalten werden. Das bedeutet, dass ein Volumen einer Teilkavität oder eines Teilkavitätsabschnitts von einem einzelnen elektromechanischen Wandler beeinflusst sein kann.

Vorangehend beschriebene Ausführungsbeispiele, die ein verformbares Element aufweisen, das ausgebildet ist, um eine mehrfache Krümmung auszuführen und/oder mit einem Plattenelement verbunden ist, können verglichen mit der Konfiguration, wie sie im Zusammenhang mit der Fig. 1 beschrieben ist, nutzbar sein, um einen deutlich höheren Volumenstrom zu erzeugen oder um auf einen Volumenstrom deutlich sensibler zu reagieren.

Ausführungsbeispiele ermöglichen den frequenzabhängigen Verlauf des Schalldrucks flexibel einstellbar zu machen, um insbesondere auch den häufig angestrebten Fall eines möglichst flachen Frequenzverlaufs zu ermöglichen.

Um eine frequenzabhängige Schalldruckkurve mit möglichst wenigen Kammern des MEMS-Wandlers selbigen möglichst flach zu gestalten, ist es vorteilhaft, wenn die Güte der schwingungsfähigen Biegebalken gering ist, d. h. die Biegebalken weisen eine breite Resonanzkurve auf. Dazu kann die Einspannung der Balken so ausgeführt werden, dass mittels eines dämpfenden Materials die Balkenschwingung zusätzlich gedämpft wird. Die Einspannung des Balkens wird dazu bevorzugt aus einem nichtkristallinen Material hergestellt. Dazu zählen Siliziumoxid, Polymere, wie z. B. SU8 oder andere Resiste. Eine Dämpfung der Balkenschwingung kann auch elektrisch erreicht werden. Beispielsweise fließt während der freien Balkenschwingung bei einem elektrostatischen oder piezoelektrischen Aktor bei anliegender Spannung aufgrund der Veränderung der Kapazität ein periodisch wechselnder Strom. Durch geeignet vorgesehene elektrische Widerstände entsteht eine Verlustleistung, die zur Dämpfung der Schwingung führt. Auch ein vollständiger elektrischer Schwingkreis (d. h. zusätzlich wird eine integrierte oder externe Spule vorgesehen) ist möglich. Eine Dämpfung kann auch erreicht werden, indem zusätzliche Strukturen an den Biegebalken realisiert werden, welche einen signifikanten Strömungswiderstand für das Fluid beim Ein- bzw. Ausströmen in bzw. aus der Kammer darstellen.

Gerade für die Darstellung niedriger Resonanzfrequenzen - zur Erzeugung bzw. Detektion von tiefen Frequenzen - kann es vorteilhaft sein, die Masse der Biegebalken zu erhöhen. Um dabei die Steifigkeit nicht wesentlich zu erhöhen, werden dafür vorzugsweise im Bereich der größten Schwingungsamplituden zusätzliche Strukturen angebracht. Im Falle eines einseitig eingespannten Balkens ist der optimale Ort bzw. der Bereich der größten Schwingungsamplituden das Ende des Biegebalkens. Im Fall eines zweiseitig eingespannten Balkens ist dies die Mitte des Balkens.

In anderen Worten beruht eine Erkenntnis der vorliegenden Erfindung darauf, dass durch Kompression bzw. Expansion von Kammern, d. h. Teilkavitäten oder Teilkavitätsabschnitten, die in einem Siliziumchip gebildet werden können, ein Volumenstrom generiert wird oder erfassbar wird. Jede Kammer kann mit einem Einlass oder Auslass versehen sein, durch den ein Fluid, etwa Luft, ein- bzw. ausströmen kann. Die Kammern können entlang einer Richtung senkrecht zu der lateralen Bewegungsrichtung (beispielsweise oben und unten) durch einen festen Deckel verschlossen sein. Mindestens eine der seitlichen Wände jeder Kammer ist beweglich oder verformbar ausgebildet und kann durch einen Aktor so verschoben werden, dass sich das Volumen dieser Kammer verringert oder erhöht.

Vorangehend beschriebene Ausführungsbeispiele von MEMS-Wandlern können elektrische Verbindungen, Bondpads oder dergleichen aufweisen, die der Übersichtlichkeit halber in den Figuren nicht gezeigt sind.

Vorangehend beschriebene Ausführungsbeispiele beziehen sich auf Mehrwegelautsprecher oder N-Wege-Lautsprecher die basierend auf unterschiedlichen Resonanzfrequenzen von zumindest zwei Kavitäten oder Teilkavitäten erhalten werden können. Die elektromechanischen Wandler und die Kavitäten oder Teilkavitäten können so auf einander abgestimmt sein, dass ein Schalldruckpegel (SPL) zumindest abschnittsweise, eine Funktion der Resonanzfrequenz ist, d. h. mehrere Aktorkammern können verschiedenen Frequenzverläufe aufweisen (SPL = f(Frequenz)). Das bedeutet, dass Werte von Schalldruckpegeln, die basierend auf der Verformung der verformbaren Elemente und basierend auf der Teilkavitäten erhalten werden, einen Zusammenhang mit einer Frequenz des Volumenstroms, der aus oder in die jeweilige Teilkavität strömt, aufweisen. Der Zusammenhang kann als Funktion darstellbar sein, wobei die Funktion bspw. linear sein kann, etwa SPL = x*Frequenz + b, wobei x und b Variablen sind. Alternativ kann die Funktion auch nichtlinear sein, etwa quadratisch, exponentiell oder basierend auf einer Wurzelfunktion. Der funktionelle Zusammenhang kann ohne weiteres auf verschiedene Teilkavitäten oder Kavitäten, die in verschiedenen MEMS-Wandlern angeordnet sind, übertragen werden. Somit kann die Frequenz des Volumenstroms einen frequenzabhängigen Verlauf eines Drucks in dem Fluid beschreiben.

Die Siliziumchips der MEMS-Wandler können so gestaltet und so aus dem Scheibenverbund, der während einer Fertigung auf Wafer-Level erhalten wird, herausgelöst werden, dass sie für die jeweilige Anwendung eine angepasste Form aufweisen. So kann der Chip z. B. für die Anwendung als Lautsprecher in Hörgeräten oder In-Ear-Kopfhörern beispielsweise rund oder, was für den Verbrauch an Siliziumfläche auf der Scheibe besser geeignet ist, hexagonal gestaltet werden.

Die technische Ausgestaltung der monolithischen Integration von mikroelektronischen Komponenten bzw. Mikrosystemen auf der Oberfläche und Unterfläche eines MEMS-Lautsprechers, welcher das Chipvolumen zur Schallerzeugung nutzt, kann durch mehrere Aspekte ergänzt werden. Die mikroelektronischen Komponenten, etwa die MEMS-Strukturen können in eine oder mehrere Schichten integriert werden. Die elektronische Steuer- bzw. Auswerteschaltung, d. h. die elektronische Schaltung 17, kann einen Digital-Analog-Umsetzer und/oder einen PWM-Generator aufweisen.

Eine Signalaufbereitung kann beispielsweise durch digitale Signalprozessierung erfolgen, die Teil der elektronischen Schaltung 17 sein kann. Die elektronische Schaltung 17 kann ferner Taktgeber und/oder Oszillatoren umfassen, kann eine Hochvolterzeugung, d. h. einen DC/DC-Konverter, eine Ladungspumpe oder einen Hochsetzsteller aufweisen, kann einen Decoder, etwa für digitale Audiosignale wie den "sound wire"-Standard oder einen MP3-Decoder aufweisen. Ferner kann die elektronische Schaltung einen Verstärker oder eine Strombank umfassen. Die elektronische Schaltung 17 kann einen Prozessor, etwa eine CPU aufweisen, der beispielsweise für Text-to-Speech- oder Speech-to-Text-Algorithmen nutzbar ist. Ferner kann die elektronische Schaltung 17 einen Halbleiterspeicher wie etwa einen RAM- oder einen Flash-Speicher aufweisen. Die MEMS-Wandler können MEMS-Sensoren wie etwa Beschleunigungssensoren, Temperatursensoren, Drehratensensoren, Lagesensoren oder Magnetfeldsensoren umfassen, die von Teilen der elektronischen Schaltung 17 oder weiterer Schaltungskomponenten angesteuert oder ausgelesen werden. Ferner können Temperatur- oder Feuchtigkeitssensoren oder Gassensoren vorgesehen werden. Alternativ oder zusätzlich ist es möglich, Funkschnittstellen und/oder Drahtlosschnittstellen für eine Nahfeldkommunikation vorzusehen, etwa eine Bluetooth-Schnittstelle. Auch ist es möglich, dass eine Mobilfunkschnittstelle, etwa für LTE und/oder eSIM (eingebettete SIM), eine Maschine-Maschine-Schnittstelle wie iBeacon oder physical Web vorgesehen ist. Alternativ oder zusätzlich kann ein Empfänger für globale Navigationssatellitensysteme wie GBS integriert sein. Die MEMS-Wandler können als MEMS-Mikrofone und/oder MEMS-Lautsprecher oder dergleichen ausgeführt sein.

Die MEMS-Wandler ermöglichen eine Systemfunktion, d. h. eine Kombination mit weiteren Funktionen oder Anwendungen. Hierzu gehören beispielsweise MP3-Player, Unterhaltungstechnik, Streaming, Headset, Walkie-Talkie und dergleichen. Auch ist eine Anwendung in Hörgeräten möglich. Gesundheitsassistenzsysteme wie Fittnesstracker, die eine Aufzeichnung, einen Vergleich, eine Optimierung und/oder eine Ansage von Vitaldaten ermöglichen, wozu eine Schrittzählung, eine Körpertemperatur, eine Atemfrequenz und/oder ein Energieverbrauch gehören, sind ebenfalls vorstellbar. Systeme können sich auch auf Navigationsassistenzsysteme beziehen, die beispielsweise im öffentlichen Raum und/oder innerhalb von Gebäuden über Sprache-zu-Text- (Speech-to-Text) und/oder Text-zu-Sprache- (Text-to-Speech) Technologien oder Algorithmen Unterstützung ermöglichen. Dies kann sich beispielsweise auf eine bestimmte Personengruppe, wie etwa Blinde beziehen und/oder auf bestimmte Gebäude, wie etwa öffentliche Gebäude, Militärgebäude, Polizeigebäude oder Feuerwehrgebäude. In Steuerungssystemen können hierin beschriebene Ausführungsbeispiele beispielsweise zur Steuerung von Kommunikation und Geräten per Gesten und Sprache eingesetzt werden. Hier kann beispielsweise eine Mensch-Maschine-Schnittstelle über Speech-to-Text und/oder Text-to-Speech-Technologien und/oder Algorithmen vorgesehen sein. Auch sind Anwendungen in der Universalübersetzung vorstellbar, etwa für eine simultane Übersetzung im Sprachbereich. Anwendungen in der virtuellen Realität sind ebenfalls implementierbar, beispielsweise eine rein akustische virtuelle Realität, etwa für blinde Menschen, eine unterstützende visuelle virtuelle Realität, die auch als "augmented reality" bezeichnet wird, ein hörbares Internet, hörbare soziale Netzwerke oder dergleichen. Alternativ oder zusätzlich ist eine Audio-Signatur implementierbar, die eine Personalisierung der Sprachkommandos ermöglicht. Hierunter fallen beispielsweise sicherheitsrelevante Logins, etwa für ein hörbares Internet und eine Mensch-Maschine-Kommunikation. Eine Erkennung sicherheitskritischer akustischer Ereignisse, etwa ein Hilferuf ist ebenfalls implementierbar. Die selbigen Ausführungen gelten uneingeschränkt auch für MEMS-Mikrofone zur Aufnahme akustischer Signale.

Obwohl manche Aspekte im Zusammenhang mit einer Vorrichtung beschrieben wurden, versteht es sich, dass diese Aspekte auch eine Beschreibung des entsprechenden Verfahrens darstellen, sodass ein Block oder ein Bauelement einer Vorrichtung auch als ein entsprechender Verfahrensschritt oder als ein Merkmal eines Verfahrensschrittes zu verstehen ist. Analog dazu stellen Aspekte, die im Zusammenhang mit einem oder als ein Verfahrensschritt beschrieben wurden, auch eine Beschreibung eines entsprechenden Blocks oder Details oder Merkmals einer entsprechenden Vorrichtung dar.

Die oben beschriebenen Ausführungsbeispiele stellen lediglich eine Veranschaulichung der Prinzipien der vorliegenden Erfindung dar. Es versteht sich, dass Modifikationen und Variationen der hierin beschriebenen Anordnungen und Einzelheiten anderen Fachleuten einleuchten werden. Deshalb ist beabsichtigt, dass die Erfindung lediglich durch den Schutzumfang der nachstehenden Patentansprüche und nicht durch die spezifischen Einzelheiten, die anhand der Beschreibung und der Erläuterung der Ausführungsbeispiele hierin präsentiert wurden, beschränkt sei.

**Literatur**

[1] Albach, Thorsten Sven: Magnetostriktive Mikroaktoren und deren Anwendung als Mikrolautsprecher, Dissertation, Universität Erlangen-Nürnberg (2013).

[2] Roberts, Robert C. et al.: Electrostatically Driven Touch-Mode Poly-SiC Microspeaker, Sensors, IEEE 2007 (2007), p. 284-287.

**EP 3 612 493 B1**

[3] Kim, H. et al.: Bi-directional Electrostatic Microspeaker with Two Large-Deflection Flexible Membranes Actuated by Single/Dual Electrodes, Sensors, IEEE 2005 (2005), p. 89-92.

[4] Rehder, J.; Rombach, P.; Hansen, O.: Magnetic flux generator for balanced membrane loudspeaker. In: Sensors and Actuators A: Physical 97 (2002), Nr. 8, p. 61-67.

[5] Neri, F.; Di Fazio, F.; Crescenzi, R.; Balucani, M.: A novel micromachined loudspeaker topology. In: 61 st Conf. on Electronic Components and Technology, ECTC, IEEE 2011 (2011), p. 1221-1227.

[6] Neumann, J. J., Gabriel, K. J.: CMOS-MEMS Acoustic Devices, in: Advanced Micro and Nanosystems, Vol. 2. CMOS-MEMS. Edited by H. Baltes et al., Wiley-VCH Verlag, Weinheim (2005).

[7] Lerch R.; Sessler, G.; Wolf, D.: Technische Akustik, Springer Verlag (2009).

[8] Schenk, H. et al.: A resonantly excited 2D-micro-scanning-mirror with large deflection, Sensors and Actuators A 89 (2001), p. 104-111.

[9] Rosa, M. A. et al.: A novel external electrode configuration for the electrostatic actuation of MEMS based devices, J. Micromech. Microeng.(2004), p. 446-451.

[10] Kumar, V.; Sharma, N. N.: Design and Validation of Silicon-on-Insulator Based U Shaped thermal Microactuator, Int. J. Materials, Mechanics and Manufacturing, Vol. 2, No. 1 (2014), p. 86-91.

[11] Cheng, Ming-Cheng et al.: A lilicon microspeaker for hearing instruments, J. Micromech. Microeng. 14 (2004), p. 859-866

**Patentansprüche**

1. MEMS-Wandler zum Interagieren mit einem Volumenstrom (12) eines Fluids mit:

   einem Substrat (14), das einen Schichtstapel mit einer Mehrzahl von Schichten (32ab, 34a-b, 36) aufweist, die eine Mehrzahl von Substratebenen bilden, und das eine Kavität (16) in dem Schichtstapel aufweist;
   einem elektromechanischen Wandler (18; 18a-f), der mit dem Substrat (14) in der Kavität (16) verbunden ist und ein sich in zumindest einer Bewegungsebene der Mehrzahl von Substratebenen verformbares Element (22; 22a-f; 30; 40; 150; 160) aufweist, wobei eine Verformung des verformbaren Elements (22; 22a-f; 30; 40; 150; 160) in der Bewegungsebene und der Volumenstrom (12) des Fluids kausal zusammenhängen; **gekennzeichnet durch**
   eine elektronischen Schaltung (17; 17a-b), die in zumindest einer Schicht (32a-b) des Schichtstapels angeordnet ist, wobei die elektronische Schaltung (17; 17a-b) mit dem elektromechanischen Wandler (18; 18a-f) verbunden ist, und die ausgebildet ist, um eine Konvertierung zwischen einer Verformung des verformbaren Elements (22; 22a-f; 30; 40; 150; 160) und einem elektrischen Signal bereitzustellen;
   wobei die elektronische Schaltung (17; 17a-b) ausgebildet ist, um ein elektrisches Ansteuersignal ($In_1$) in eine Auslenkung des verformbaren Elements (22; 22a-f; 30; 40; 150; 160) zu konvertieren, wobei die Schaltung einen schaltenden Verstärker umfasst, der ausgebildet ist, um ein digitales pulsweitenmoduliertes Ansteuersignal für das verformbare Element (22; 22a-f; 30; 40; 150; 160) bereitzustellen.

2. MEMS-Wandler gemäß Anspruch 1, bei dem die elektronische Schaltung (17; 17a-b) zumindest eines aus einem Digital-Analog-Wandler zum Konvertieren einer digitalen Version des Ansteuersignals ($In_1$) in eine analoge Version des Ansteuersignals ($Out_1$), einem Analog-Digital-Wandler zum Umsetzen einer analogen Version des elektrischen Ausgangssignals ($In_1$) in eine digitale Version des elektronischen Ausgangssignals ($Out_1$), einem Signaldecoder, einem Prozessor, einem Halbleiterspeicher, einer Drahtloskommunikationsschnittstelle für eine Nahfeldkommunikation und einer Mobilfunkschnittstelle umfasst; und

3. MEMS-Wandler gemäß Anspruch 1 oder 2, bei dem das verformbare Element (22; 22a-f; 30; 40; 150; 160) ein erstes verformbares Element (22; 22a-f; 30; 40; 150; 160) ist, wobei der MEMS-Wandler zumindest ein zweites verformbares Element (22; 22a-f; 30; 40; 150; 160) umfasst, wobei die elektronische Schaltung (17; 17a-b) ausgebildet ist, um das elektrische Ansteuersignal (In) in eine Auslenkung des ersten und des zweiten verformbaren

**42**

Elements (22; 22a-f; 30; 40; 150; 160) zu konvertieren, oder um die Verformung des ersten und des zweiten verformbaren Elements (22; 22a-f; 30; 40; 150; 160) in das elektrische Ausgangssignal (Out₂) zu konvertieren.

4. MEMS-Wandler gemäß einem der vorangehenden Ansprüche, wobei das verformbare Element (22; 22a-f; 30; 40; 150; 160) aktiv gebildet ist und ausgebildet ist, um mit dem Volumenstrom (12) zu interagieren; oder ein mit dem verformbaren Element (22; 22a-f; 30; 40; 150; 160) verbundenes und steif ausgebildetes Plattenelement (62; 62a-c) ausgebildet ist, um mit dem Volumenstrom (12) zu interagieren

5. MEMS-Wandler gemäß einem der vorangehenden Ansprüche, bei dem die Schicht (32a-b) in der die elektronische Schaltung (17; 17a-b) angeordnet ist, mit einer Außenseite des MEMS-Wandlers elektrisch verbunden ist oder die Außenseite des MEMS-Wandlers ist, und mit einer Leitungsanordnung kontaktierbar ist.

6. MEMS-Wandler gemäß einem der vorangehenden Ansprüche, bei dem die Schicht (32a-b) in der die elektronische Schaltung (17; 17a-b) angeordnet ist eine erste Deckelschicht ist, wobei die elektronische Schaltung (17; 17a-b) eine erste elektronische Schaltung (17a) ist, und bei dem eine zweite elektronische Schaltung (17b) in einer zweiten Deckelschicht des Substrats (14) angeordnet ist.

7. MEMS-Wandler gemäß einem der vorangehenden Ansprüche, bei dem der Schichtstapel eine Adaptionsschicht (27) aufweist, und die an einer ersten Schichthauptseite (29a) eine erste elektronische Struktur (31a) mit einem ersten Abstandsraster (33a) aufweist und die an einer zweiten, gegenüberliegenden Schichthauptseite (29b) eine zweite elektronische Struktur (31b) mit einem zweiten Abstandsraster (33b) aufweist, wobei die erste elektronischen Struktur und die zweite elektronische Struktur in der Adaptionsschicht mit einander verbunden sind, wobei die Adaptionsschicht (27) eine Schicht des Schichtstapels ist, die eine Kontaktierung zu einer anderen Komponente ermöglicht.

8. MEMS-Wandler gemäß Anspruch 7, bei dem die Adaptionsschicht (27) eine Deckelschicht des Stapels ist, in der die elektronische Schaltung (17; 17a-b) zumindest teilweise angeordnet ist, oder eine Deckelschicht des Stapels ist, die elektrisch ausschließlich als Adaptionsschicht gebildet ist.

9. MEMS-Wandler gemäß einem der vorangehenden Ansprüche, bei dem die Schicht (32a-b) in der die elektronische Schaltung (17; 17a-b) angeordnet ist, ein Funktionselement (21) umfasst, das mit der elektronischen Schaltung (17; 17a-b) verbunden ist, wobei die elektronische Schaltung (17; 17a-b) ausgebildet ist, um das Funktionselement (21) anzusteuern oder auszuwerten; wobei das Funktionselement einen Sensor, einen Aktor, eine Drahtlos-Kommunikationsschnittstelle, eine Lichtquelle, einen Speicherbaustein und/oder einen Navigationsempfänger umfasst.

10. MEMS-Wandler gemäß einem der vorangehenden Ansprüche, bei dem die Schicht (32a-b) in der die elektronische Schaltung (17; 17a-b) angeordnet ist eine erste Deckelschicht ist, wobei die elektronische Schaltung (17; 17a-b) eine erste elektronische Schaltung (17a) ist, und bei dem eine zweite elektronische Schaltung (17b) und ein Funktionselement (21) in einer zweiten Deckelschicht des Substrats (14) angeordnet ist, wobei die zweite elektronische Schaltung (17b) mit dem Funktionselement (21) verbunden ist, und ausgebildet ist, um das Funktionselement (21) anzusteuern oder auszuwerten; wobei das Funktionselement einen Sensor, einen Aktor, eine Drahtlos-Kommunikationsschnittstelle, eine Lichtquelle, einen Speicherbaustein, einen Prozessor und/oder einen Navigationsempfänger umfasst.

11. MEMS-Wandler gemäß Anspruch 9 oder 10, bei dem das Funktionselement (21) zumindest einen Inertialsensor, ein Magnetometer, einen Temperatursensor, einen Feuchtigkeitssensor, einen Gassensor oder eine Kombination daraus umfasst.

12. MEMS-Wandler gemäß Anspruch 11, bei dem das Funktionselement (21) zumindest eines aus einem MEMS-Sensor und einem MEMS-Aktor umfasst.

13. MEMS-Wandler gemäß einem der vorangehenden Ansprüche, bei dem die elektronische Schaltung (17; 17a-b) in einer Deckelschicht des Substrats (14) angeordnet ist, und bei der ferner ein gassensorisches Funktionselement in der Deckelschicht angeordnet ist, wobei die Deckelschicht eine Öffnung (26a) aufweist, die ausgebildet ist, um den Fluidstrom (12) passieren zu lassen, wobei das gassensorische Funktionselement ausgebildet ist, um mit dem Fluidstrom (12) sensorisch zu interagieren.

14. MEMS-Wandler gemäß Anspruch 13, bei dem die Öffnung von dem gassensorischen Funktionselement (21) in der Deckelschicht umschlossen ist; oder bei dem das gassensorische Funktionselements (21) zumindest teilweise in die Öffnung (26a) hineinragt.

15. MEMS-Wandler gemäß einem der vorangehenden Ansprüche, bei dem die elektronische Schaltung (17; 17a-b) entlang einer Richtung senkrecht zu der Bewegungsebene angeordnet ist, und ein Ort der elektronischen Schaltung (17; 17a-b), wenn dieser in die Bewegungsebene projiziert wird, einem Ort entspricht, an dem sich das verformbare Element (22; 22a-f; 30; 40; 150; 160) während der Verformung zumindest zeitweise befindet.

16. MEMS-Wandler gemäß einem der vorangehenden Ansprüche, bei dem der elektromechanische Wandler (18; 18a-f) ausgebildet ist, um ansprechend auf eine elektrische Ansteuerung (Out, Outi) der elektronischen Schaltung (17; 17a-b) kausal eine Bewegung des Fluids in der Kavität (16) zu bewirken und/oder um ansprechend auf die Bewegung des Fluids in der Kavität (16) kausal ein elektrisches Signal ($Out_2$) mit der elektronischen Schaltung (17; 17a-b) bereitzustellen.

17. Vorrichtung mit einem MEMS-Wandler gemäß einem der vorangehenden Ansprüche, bei der der MEMS-Wandler als MEMS-Lautsprecher ausgebildet ist, wobei die Vorrichtung als mobile Musikwiedergabevorrichtung oder als Kopfhörer ausgebildet ist.

18. System mit einem MEMS-Wandler gemäß einem der Ansprüche 1 bis 17, wobei der MEMS-Wandler als Lautsprecher ausgeführt ist und ausgebildet ist, um aufbauend auf einem Ausgabesignal ein akustisches Signal wiederzugeben.

19. Gesundheitsassistenzsystem (220) mit:

einer Sensoreinrichtung (35) zum Erfassen einer Vitalfunktion eines Körpers (37) und zum Ausgeben eines Sensorsignals (39) basierend auf der erfassten Vitalfunktion;
eine Verarbeitungseinrichtung (41) zum Verarbeiten des Sensorsignals (39) und zu Bereitstellen eines Ausgabesignals (43) basierend auf der Verarbeitung; und
einen Kopfhörer (200) umfassend einen MEMS-Wandler gemäß einem der Ansprüche 1 bis 16, wobei der MEMS-Wandler als Lautsprecher ausgeführt ist und der eine Drahtlos-Kommunikationsschnittstelle zum Empfangen des Ausgabesignals (43) umfasst und ausgebildet ist, um aufbauend hierauf ein akustisches Signal wiederzugeben.

20. Verfahren zum Bereitstellen eines MEMS-Wandler zum Interagieren mit einem Volumenstrom (12) eines Fluids mit folgenden Schritten:

Bereitstellen eines Substrats (14), das einen Schichtstapel mit einer Mehrzahl von Schichten (32a-b, 34a-b, 36) umfasst, die eine Mehrzahl von Substratebenen bilden; Erzeugen einer Kavität (16) in dem Schichtstapel und eines elektromechanischen Wandlers (18; 18a-f), in dem Substrat (14), so dass dieser mit dem Substrat (14) in der Kavität (16) verbunden ist und ein sich in zumindest einer Bewegungsebene der Mehrzahl von Substratebenen verformbares Element (22; 22a-f; 30; 40; 150; 160) aufweist, wobei eine Verformung des verformbaren Elements (22; 22a-f; 30; 40; 150; 160) in der Bewegungsebene und der Volumenstrom (12) des Fluids kausal zusammenhängen; **gekennzeichnet durch**
eine elektronische Schaltung (17; 17a-b) in zumindest einer Schicht (32a-b) des Schichtstapels angeordnet ist, so dass die elektronische Schaltung (17; 17a-b) mit dem elektromechanischen Wandler (18; 18a-f) verbunden ist, und ausgebildet ist, um eine Konvertierung zwischen einer Verformung des verformbaren Elements (22; 22a-f; 30; 40; 150; 160) und einem elektrischen Signal bereitzustellen;
so dass die elektronische Schaltung (17; 17a-b) ausgebildet ist, um ein elektrisches Ansteuersignal ($In_1$) in eine Auslenkung des verformbaren Elements (22; 22a-f; 30; 40; 150; 160) zu konvertieren, wobei die Schaltung einen schaltenden Verstärker umfasst, der ausgebildet ist, um ein digitales pulsweitenmoduliertes Ansteuersignal für das verformbare Element (22; 22a-f; 30; 40; 150; 160) bereitzustellen.

**Claims**

1. MEMS transducer for interacting with a volume flow (12) of a fluid, comprising:

a substrate (14) which comprises a layer stack having a plurality of layers (32a-b, 34a-b, 36) which form a plurality of substrate planes, and which comprises a cavity (16) within the layer stack;

an electromechanical transducer (18; 18a-f) connected to the substrate (14) within the cavity (16) and comprising an element (22; 22a-f; 30; 40; 150; 160) which is deformable within at least one plane of movement of the plurality of substrate planes, deformation of the deformable element (22; 22a-f; 30; 40; 150; 160) within the plane of movement and the volume flow (12) of the fluid being causally correlated; **characterized in that** an electronic circuit (17; 17a-b) arranged within at least one layer (32a-b) of the layer stack, the electronic circuit (17; 17a-b) being connected to the electromechanical transducer (18; 18a-f) and being configured to provide a conversion between a deformation of the deformable element (22; 22a-f; 30; 40; 150; 160) and an electric signal; wherein the electronic circuit (17; 17a-b) is configured to convert an electric control signal ($In_1$) to a deflection of the deformable element (22; 22a-f; 30; 40; 150; 160), wherein the circuit includes a switching amplifier configured to provide a digital pulse-width modulated control signal for the deformable element (22; 22a-f; 30; 40; 150; 160).

2. MEMS transducer according to claim 1, wherein the electronic circuit (17; 17a-b) includes at least one of a digital-to-analog converter for converting a digital version of the control signal ($In_1$) to an analog version of the control signal ($Out_1$), an analog-to-digital converter for converting an analog version of the electric output signal ($In_1$) to a digital version of the electric output signal ($Out_1$), a signal decoder, a processor, a semiconductor memory, a wireless communication interface for near-field communication, and a mobile radio interface.

3. MEMS transducer according to claim 1 or 2, wherein the deformable element (22; 22a-f; 30; 40; 150; 160) is a first deformable element (22; 22a-f; 30; 40; 150; 160), the MEMS transducer including at least a second deformable element (22; 22a-f; 30; 40; 150; 160), the electronic circuit (17; 17a-b) being configured to convert the electric control signal (In) to a deflection of the first and second deformable elements (22; 22a-f; 30; 40; 150; 160) or to convert the deformations of the first and second deformable elements (22; 22a-f; 30; 40; 150; 160) to the electric output signal ($Out_2$).

4. MEMS transducer according to one of the preceding claims, wherein the deformable element (22; 22a-f; 30; 40; 150; 160) is actively formed and is configured to interact with the volume flow (12); or a plate element (62; 62a-c) connected to the deformable element (22; 22a-f; 30; 40; 150; 160) and configured to be rigid is configured to interact with the volume flow (12).

5. MEMS transducer according to one of the preceding claims, wherein the layer (32a-b) which has the electronic circuit (17; 17a-b) arranged therein is electrically connected to an outer side of the MEMS transducer, or is the outer side of the MEMS transducer and may be contacted to a lead assembly.

6. MEMS transducer according to one of the preceding claims, wherein the layer (32a-b) which has the electronic circuit (17; 17a-b) arranged therein is a first lid layer, the electronic circuit (17; 17a-b) being a first electronic circuit (17a), and wherein a second electronic circuit (17b) is arranged in a second lid layer of the substrate (14).

7. MEMS transducer according to one of the preceding claims, wherein the layer stack comprises an adaptation layer (27) comprising, at a first layer main side (29a), a first electronic structure (31a) having a first distance raster (33a) and comprising, at a second, oppositely located layer main side (29b), a second electronic structure (31b) having a second distance raster (33b), the first electronic structure and the second electronic structure being connected to each other in the adaptation layer, wherein the adaptation layer (27) is a layer of the layer stack enabling contacting with another component.

8. MEMS transducer according to claim 7, wherein the adaptation layer (27) is a lid layer of the stack and has the electronic circuit (17; 17a-b) at least partly arranged therein, or is a lid layer of the stack which is electrically configured as an adaptation layer exclusively.

9. MEMS transducer according to one of the preceding claims, wherein the layer (32a-b) which has the electronic circuit (17; 17a-b) arranged therein includes a functional element (21) connected to the electronic circuit (17; 17a-b), the electronic circuit (17; 17a-b) being configured to control or evaluate the functional element (21); wherein the functional element (21) comprises a sensor, an actuator, a wireless communication interface, a light source, a memory component and/or a navigation receiver.

10. MEMS transducer according to one of the preceding claims, wherein the layer (32a-b) which has the electronic circuit (17; 17a-b) arranged therein is a first lid layer, the electronic circuit (17; 17a-b) being a first electronic circuit (17a), and wherein a second electronic circuit (17b) and a functional element (21) are arranged in a second lid layer of the substrate (14), the second electronic circuit (17b) being connected to the functional element (21) and configured to control or evaluate the functional element (21); wherein the functional element comprises a sensor, an actuator, a wireless communication interface, a light source, a memory component, a processor and/or a navigation receiver.

11. MEMS transducer according to claim 9 or 10, wherein the functional element (21) includes at least an inertial sensor, a magnetometer, a temperature sensor, a humidity sensor, a gas sensor or a combination thereof.

12. MEMS transducer according to claim 11, wherein the functional element (21) includes at least one of a MEMS sensor and a MEMS actuator.

13. MEMS transducer according to one of the preceding claims, wherein the electronic circuit (17; 17a-b) is arranged in a lid layer of the substrate (14), and wherein, in addition, a gas-sensing functional element is arranged in the lid layer, the lid layer comprising an opening (26a) configured to allow the fluid flow (12) to pass, the gas-sensing functional element being configured to sensorically interact with the fluid flow (12).

14. MEMS transducer according to claim 13, wherein the opening is encompassed by the gas-sensing functional element (21) in the lid layer; or wherein the gas-sensing functional element (21) at least partly projects into the opening (26a).

15. MEMS transducer according to one of the preceding claims, wherein the electronic circuit (17; 17a-b) is arranged along a direction perpendicular to the plane of movement, and a location of the electronic circuit (17; 17a-b), when said location is projected into the plane of movement, corresponds to a location where the deformable element (22; 22a-f; 30; 40; 150; 160) is at least partly located during deformation.

16. MEMS transducer according to one of the preceding claims, wherein the electromechanical transducer (18; 18a-f) is configured to cause a movement of the fluid within the cavity (16) in response to electrical controlling (Out, $Out_1$) of the electronic circuit (17; 17a-b), and/or to causally provide an electric signal ($Out_2$) to the electronic circuit (17; 17a-b) in response to the movement of the fluid within the cavity (16).

17. Device comprising a MEMS transducer according to one of the preceding claims, wherein the MEMS transducer is configured as a MEMS loudspeaker, the device being configured as a mobile music reproduction device or as headphones.

18. System comprising a MEMS transducer according to one claim 1 to 17, wherein the MEMS transducer is configured as a loudspeaker and is designed to reproduce an acoustic signal on the basis of an output signal.

19. Health assistance system (220) comprising:

a sensor means (35) for sensing a vital function of a body (37) and for outputting a sensor signal (39) on the basis of the sensed vital function;
a processing means (41) for processing the sensor signal (39) and for providing an output signal (43) on the basis of said processing; and
headphones (200) including a MEMS transducer according to claims 1 to 16, the MEMS transducer being configured as a loudspeaker and including a wireless communication interface for receiving the output signal (43), and being configured to reproduce an acoustic signal on the basis thereof.

20. Method of providing a MEMS transducer for interacting with a volume flow (12) of a fluid, comprising:

providing a substrate (14) which comprises a layer stack having a plurality of layers (32a-b, (34a-b, 36) which form a plurality of substrate planes;
producing a cavity (16) in the layer stack and an electromechanical transducer (18; 18a-f) within the substrate (14), so that the former is connected to the substrate (14) within the cavity (16) and comprises an element (22; 22a-f; 30; 40; 150; 160) which is deformable within at least one plane of movement of the plurality of substrate planes, deformation of the deformable element (22; 22a-f; 30; 40; 150; 160) within the plane of movement and the volume flow (12) of the fluid being causally correlated; **characterized in that**
an electronic circuit (17; 17a-b) is arranged within at least one layer (32a-b) of the layer stack, so that the electronic circuit (17; 17a-b) is connected to the electromechanical transducer (18; 18a-f) and is configured to provide a conversion between a deformation of the deformable element (22; 22a-f; 30; 40; 150; 160) and an electric signal;

so that the electronic circuit (17; 17a-b) is configured to convert an electric control signal ($In_1$) to a deflection of the deformable element (22; 22a-f; 30; 40; 150; 160) or to convert a deformation of the deformable element (22; 22a-f; 30; 40; 150; 160) to an electric output signal ($Out_2$); and the electronic circuit (17; 17a-b) is configured to convert the electric control signal (In) to a deflection of the deformable element (22; 22a-f; 30; 40; 150; 160), and includes a switching amplifier configured to provide a digital pulse-width modulated control signal for the deformable element (22; 22a-f; 30; 40; 150; 160).

**Revendications**

1. Transducteur MEMS destiné à interagir avec un débit volumique (12) d'un fluide, avec:

   un substrat (14) qui présente une pile de couches avec une pluralité de couches (32a-b, 34a-b, 36) qui forment une pluralité de plans de substrat et qui présente une cavité (16) dans la pile de couches;
   un transducteur électromécanique (18; 18a à f) qui est connecté au substrat (14) dans la cavité (16) et présente un élément pouvant se déformer (22; 22a à f; 30; 40; 30; 40; 150; 160) dans un plan de déplacement de la pluralité de plans de substrat, où une déformation de l'élément pouvant se déformer (22; 22a à f; 30; 40; 30; 40; 150; 160) et le débit volumique (12) du fluide sont de manière causale cohérents entre eux;
   **caractérisé par**
   un circuit électronique (17; 17a-b) qui est disposé dans au moins une couche (32a-b) de la pile de couches, où le circuit électronique (17; 17a-b) est connecté au transducteur électromécanique (18; 18a à f), et qui est conçu pour assurer une conversion entre une déformation de l'élément pouvant se déformer (22; 22a à f; 30; 40; 150; 160) et un signal électrique;
   dans lequel le circuit électronique (17; 17a-b) est conçu pour convertir un signal de commande électrique ($In_1$) en une déviation de l'élément pouvant se déformer (22; 22a à f; 30; 40; 150; 160), dans lequel le circuit. comporte un amplificateur de commutation conçu pour fournir un signal de commande numérique modulé en largeur d'impulsion pour l'élément pouvant se déformer (22; 22a à f; 30; 40; 150; 160).

2. Transducteur MEMS selon la revendication 1, dans lequel le circuit électronique (17; 17a-b) comporte au moins l'un parmi un convertisseur numérique-analogique destiné à convertir une version numérique du signal de commande ($In_1$) en une version analogique du signal de commande ($Out_1$), un convertisseur analogique-numérique destiné à convertir une version analogique du signal de sortie électrique ($In_1$) en une version numérique du signal de sortie électronique ($Out_1$), un décodeur de signal, un processeur, une mémoire à semi-conducteur, une interface de communication sans fil pour une communication à courte distance et une interface radio mobile.

3. Transducteur MEMS selon la revendication 1 ou 2, dans lequel l'élément pouvant se déformer (22; 22a à f; 30; 40; 150; 160) est un premier élément pouvant se déformer (22; 22a à f; 30; 40; 150; 160), dans lequel le transducteur MEMS comporte au moins un deuxième élément pouvant se déformer (22; 22a à f; 30; 40; 150; 160), dans lequel le circuit électronique (17; 17a-b) est conçu pour convertir le signal de commande électrique (In) en une déviation du premier et du deuxième élément pouvant se déformer (22; 22a à f; 30; 40; 150; 160), ou pour convertir la déformation du premier et du deuxième élément pouvant se déformer (22; 22a à f; 30; 40; 150; 160) en signal de sortie électrique ($Out_2$).

4. Transducteur MEMS selon l'une des revendications précédentes, dans lequel l'élément pouvant se déformer (22; 22a à f; 30; 40; 150; 160) est formé activement et est conçu pour interagir avec le débit volumique (12); ou un élément de plaque (62 ; 62a à c) connecté à l'élément pouvant se déformer (22; 22a à f; 30; 40; 150; 160) et formé de manière rigide est conçu pour interagir avec le débit volumique (12).

5. Transducteur MEMS selon l'une des revendications précédentes, dans lequel la couche (32a-b) dans laquelle est disposé le circuit électronique (17; 17a-b) est connectée électriquement à une face extérieure du transducteur MEMS ou est la face extérieure du Transducteur MEMS, et peut être amené en contact avec un aménagement de conduite.

6. Transducteur MEMS selon l'une des revendications précédentes, dans lequel la couche (32a-b) dans laquelle est disposé le circuit électronique (17; 17a-b) est une première couche de revêtement, dans lequel le circuit électronique (17; 17a b) b) est un premier circuit électronique (17a) et dans lequel un deuxième circuit électronique (17b) est disposé dans une deuxième couche de revêtement du substrat (14).

**7.** Transducteur MEMS selon l'une des revendications précédentes, dans lequel la pile de couches présente une couche d'adaptation (27) qui présente, sur une première face principale de couche (29a), une première structure électronique (31a) avec une première grille d'espacement (33a) et qui présente, sur une deuxième face principale de couche opposée (29b), une deuxième structure électronique (31b) avec une deuxième grille d'espacement (33b), dans lequel la première structure électronique et la deuxième structure électronique sont connectées l'une à l'autre dans la couche d'adaptation, dans lequel la couche d'adaptation (27) est une couche de la pile de couches qui permet une entrée en contact avec un autre composant.

**8.** Transducteur MEMS selon la revendication 7, dans lequel la couche d'adaptation (27) est une couche de revêtement de la pile dans laquelle est disposé au moins partiellement le circuit électronique (17; 17a-b), ou est une couche de revêtement de la pile qui est formée électriquement de manière exclusive comme couche d'adaptation.

**9.** Transducteur MEMS selon l'une des revendications précédentes, dans lequel la couche (32a-b) dans laquelle est disposé le circuit électronique (17; 17a-b) comporte un élément fonctionnel (21) qui est connecté au circuit électronique (17; 17a-b), dans lequel le circuit électronique (17; 17a-b) est conçu pour commander ou évaluer l'élément fonctionnel (21); dans lequel l'élément fonctionnel comporte un capteur, un actionneur, une interface de communication sans fil, une source de lumière, un module de mémoire et/ou un récepteur de navigation.

**10.** Transducteur MEMS selon l'une des revendications précédentes, dans lequel la couche (32a-b) dans laquelle est disposé le circuit électronique (17; 17a-b) est une première couche de revêtement, dans lequel le circuit électronique (17; 17a-b) est un premier circuit électronique (17a), et dans lequel un deuxième circuit électronique (17b) et un élément fonctionnel (21) sont disposés dans une deuxième couche de revêtement du substrat (14), dans lequel le deuxième circuit électronique (17b) est connecté à l'élément fonctionnel (21) et est conçu pour commander ou évaluer l'élément fonctionnel (21); dans lequel l'élément fonctionnel comporte un capteur, un actionneur, une interface de communication sans fil, une source de lumière, un module de mémoire, un processeur et/ou un récepteur de navigation.

**11.** Transducteur MEMS selon la revendication 9 ou 10, dans lequel l'élément fonctionnel (21) comporte au moins un capteur inertiel, un magnétomètre, un capteur de température, un capteur d'humidité, un capteur de gaz ou une combinaison de ces derniers.

**12.** Transducteur MEMS selon la revendication 11, dans lequel l'élément fonctionnel (21) comporte au moins l'un parmi un capteur MEMS et un actionneur MEMS.

**13.** Transducteur MEMS selon l'une des revendications précédentes, dans lequel le circuit électronique (17; 17a-b) est disposé dans une couche de revêtement du substrat (14), et dans lequel un élément fonctionnel capteur de gaz est par ailleurs disposé dans la couche de revêtement, dans lequel la couche de revêtement présente une ouverture (26a) qui est conçue pour laisser passer le flux de fluide (12), dans lequel l'élément fonctionnel capteur de gazeux est conçu pour interagir comme capteur avec le flux de fluide (12).

**14.** Transducteur MEMS selon la revendication 13, dans lequel l'ouverture est entourée par l'élément fonctionnel capteur de gaz (21) dans la couche de revêtement; ou dans lequel l'élément fonctionnel capteur de gaz (21) pénètre au moins partiellement dans l'ouverture (26a).

**15.** Transducteur MEMS selon l'une des revendications précédentes, dans lequel le circuit électronique (17; 17a-b) est disposé dans une direction perpendiculaire au plan de déplacement, et un emplacement du circuit électronique (17; 17a-b), lorsque ce dernier est projeté dans le plan de déplacement, correspond à un emplacement auquel se trouve au moins temporairement l'élément pouvant se déformer (22; 22a à f; 30; 40; 150; 160) lors de la déformation.

**16.** Transducteur MEMS selon l'une des revendications précédentes, dans lequel le transducteur électromécanique (18; 18a à f) est conçu pour provoquer de manière causale, en réponse à une commande électrique (Out, $Out_1$) du circuit électronique (17; 17a-b), un déplacement du fluide dans la cavité (16) et/ou pour fournir de manière causale, en réponse au déplacement du fluide dans la cavité (16), un signal électrique ($Out_2$) par le circuit électronique (17; 17a-b).

**17.** Dispositif avec un transducteur MEMS selon l'une des revendications précédentes, dans lequel le transducteur MEMS est conçu comme un haut-parleur MEMS, dans lequel le dispositif est conçu comme un dispositif de reproduction de musique mobile ou comme un casque.

**18.** Système avec un transducteur MEMS selon l'une des revendications 1 à 17, dans lequel le transducteur MEMS est réalisé comme un haut-parleur et est conçu pour reproduire un signal acoustique à partir d'un signal de sortie.

**19.** Système d'assistance sanitaire (220), avec:

un dispositif capteur (35) destiné à détecter une fonction vitale d'un corps (37) et à sortir un signal de capteur (39) sur base de la fonction vitale détectée;
un dispositif de traitement (41) pour traiter le signal de capteur (39) et fournir un signal de sortie (43) sur base du traitement; et
un casque (200) comportant un transducteur MEMS selon l'une des revendications 1 à 16, dans lequel le transducteur MEMS est réalisé comme un haut-parleur et qui comporte une interface de communication sans fil destinée à recevoir le signal de sortie (43) et qui est conçu pour reproduire sur cette base un signal acoustique.

**20.** Procédé pour mettre à disposition un transducteur MEMS destiné à interagir avec un débit volumique (12) d'un fluide, aux étapes suivantes consistant à:

prévoir un substrat (14) qui comporte une pile de couches avec une pluralité de couches (32a-b, 34a-b, 36) qui forment une pluralité de niveaux de substrat;
générer une cavité (16) dans la pile de couches et un transducteur électromécanique (18; 18a à f) dans le substrat (14), de sorte que ce dernier soit connecté au substrat (14) dans la cavité (16) et présente un élément pouvant se déformer (22; 22a) dans au moins un plan de déplacement de la pluralité de plans de substrat (22; 22a à f; 30; 40; 150; 160), où une déformation de l'élément pouvant se déformer (22; 22a à f; 30; 40; 150; 160) dans le plan de déplacement et le débit volumique (12) du fluide sont de manière causale cohérents entre eux;
**caractérisé par**
un circuit électronique (17; 17a-b) qui est disposé dans au moins une couche (32a-b) de la pile de couches, de sorte que le circuit électronique (17; 17a-b) soit connecté au transducteur électromécanique (18; 18a à f), et qui est conçu pour assurer une conversion entre une déformation de l'élément pouvant se déformer (22; 22a à f; 30; 40; 150; 160) et un signal électrique;
de sorte que le circuit électronique (17 ; 17a-b) soit conçu pour convertir un signal électrique de commande (Im) en une déviation de l'élément pouvant se déformer (22; 22a à f; 30; 40; 150; 160), où le circuit comporte un amplificateur de commutation qui est conçu pour fournir un signal de commande numérique modulé en largeur d'impulsion pour l'élément pouvant se déformer (22; 22a à f; 30; 40; 150; 160).

EP 3 612 493 B1

Fig. 1

Fig. 2a

Fig. 2b

EP 3 612 493 B1

Fig. 2c

EP 3 612 493 B1

Fig. 3

EP 3 612 493 B1

30a

59a

59b

30b

59c

30c

z

y

x

Fig. 4a

58a

58b

58c

y

z

x

Fig. 4b

Fig. 4c

EP 3 612 493 B1

Fig. 5

Fig. 6a

Fig. 6b

Fig. 6c

Fig. 6d

Fig. 6e

Fig. 7a

Fig. 7b

Fig. 7c

Fig. 7d

Fig. 8a

EP 3 612 493 B1

26  44  72a  48  42  72b  48  72c  48  80

30b

22b

22a

46  26  44  84  38  85

y
z  x

Fig. 8b

Fig. 8c

EP 3 612 493 B1

EP 3 612 493 B1

24

80

22a    22b

44  26  72a  48          42

46        44  26    38

Fig. 8d

Fig. 9

EP 3 612 493 B1

Fig. 10

EP 3 612 493 B1

110

Fig. 11a

Fig. 11b

Fig. 12a

Fig. 12b

EP 3 612 493 B1

Fig. 13

Fig. 14

Fig. 15

Fig. 16

Fig. 17a

Fig. 17b

Fig. 17c

EP 3 612 493 B1

Fig. 18a

Fig. 18b

EP 3 612 493 B1

Fig. 19a

Fig. 19b

Fig. 20

Fig. 21a

Fig. 21b

220

200

43

35

39

41

43

37

Fig. 22

Fig. 23

Fig. 24

EP 3 612 493 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102015210919 A1
- US 7803281 B2
- EP 2264058 A1


**In der Beschreibung aufgeführte Nicht-Patentliteratur**

- Thorsten Sven: Magnetostriktive Mikroaktoren und deren Anwendung als Mikrolautsprecher. **ALBACH.** Dissertation. Universität Erlangen-Nürnberg, 2013
- Electrostatically Driven Touch-Mode Poly-SiC Microspeaker. **ROBERTS, ROBERT C. et al.** Sensors. IEEE, 2007, 284-287
- Bi-directional Electrostatic Microspeaker with Two Large-Deflection Flexible Membranes Actuated by Single/Dual Electrodes. **KIM, H. et al.** Sensors. IEEE, 2005, 89-92
- **REHDER, J. ; ROMBACH, P ; HANSEN, O.** Magnetic flux generator for balanced membrane loudspeaker. *Sensors and Actuators A: Physical,* 2002, vol. 97 (8), 61-67
- A novel micromachined loudspeaker topology. **NERI, F. ; DI FAZIO, F. ; CRESCENZI, R ; BALUCANI, M.** 61 st Conf. on Electronic Components and Technology, ECTC. IEEE, 2011, 1221-1227
- CMOS-MEMS Acoustic Devices. **NEUMANN, J. J. ; GABRIEL, K. J. et al.** Advanced Micro and Nanosystems. Wiley-VCH Verlag, 2005, vol. 2
- **LERCH R. ; SESSLER, G. ; WOLF, D.** Technische Akustik. Springer Verlag, 2009
- **SCHENK, H. et al.** A resonantly excited 2D-micro-scanning-mirror with large deflection. *Sensors and Actuators A,* 2001, vol. 89, 104-111
- **ROSA, M. A. et al.** A novel external electrode configuration for the electrostatic actuation of MEMS based devices. *J. Micromech. Microeng,* 2004, 446-451
- **KUMAR, V. ; SHARMA, N. N.** Design and Validation of Silicon-on-Insulator Based U Shaped thermal Microactuator. *Int. J. Materials, Mechanics and Manufacturing,* 2014, vol. 2 (1), 86-91
- **CHENG, MING-CHENG et al.** A lilicon microspeaker for hearing instruments. *J. Micromech. Microeng.,* 2004, vol. 14, 859-866